# EUROPÄISCHE PATENTSCHRIFT

(11) **EP 2 124 712 B1**
(45) Veröffentlichungstag und Bekanntmachung des Hinweises auf die Patenterteilung: **05.03.2014**
(21) Anmeldenummer: 08707296.3
(22) Anmeldetag: 25.01.2008
(51) Int. Cl.: A61B 3/00, G02C 13/00, G02C 7/02, G02C 7/06

(54) **VERFAHREN ZUR BESTIMMUNG DER BEZUGSPUNKTE FERN UND NAH**
METHOD FOR DETERMINING REMOTE AND NEAR REFERENCE POINTS
PROCÉDÉ POUR DÉTERMINER LES POINTS DE RÉFÉRENCE DE LOIN ET DE PRÈS

(30) Priorität: 25.01.2007 DE 102007003845
(43) Veröffentlichungstag der Anmeldung: 02.12.2009
(73) Patentinhaber: Rodenstock GmbH, 80687 München (DE)
(72) Erfinder: ESSER, Gregor, 81735 München (DE); JUNG, Nadine, 80689 München (DE); NICKE, Katrin, 82061 Neuried (DE); SCHWARZ, Ilka, 82538 Geretsried (DE); WELK, Andrea, 81547 München (DE); ZIMMERMANN, Martin, 85253 Erdweg-Kleinberghofen (DE)
(74) Vertreter: Müller-Boré & Partner Patentanwälte PartG mbB
(86) Internationale Anmeldenummer: PCT/EP2008/000587
(87) Internationale Veröffentlichungsnummer: WO 2008/089998

(56) Entgegenhaltungen:
- WO-A-2005/029160
- WO-A2-01/57584
- DE-A1-102005 003 699
- US-A- 5 617 155
- GUTH O: "GRADAL INDIVIDUAL FRAMEFITTM- DIE NEUE GESTALTUNGSFREIHEI BEI GLEITSICHTGLAESERN" 1. Juni 2006 (2006-06-01), DEUTSCHE OPTIKERZEITUNG, OPT. FACHVEROEFF., HEIDELBERG, DE, PAGE(S) 76 - 80 , XP000962819 ISSN: 0344-7103 das ganze Dokument
- ESSER G ET AL: "DIE PERFORMANCE INDIVIDUELLER GLEITSICHTGLAESER" DEUTSCHE OPTIKER ZEITUNG, XX, DE, 1. Dezember 2005 (2005-12-01), Seiten 38-44, XP000962762

## Beschreibung

Die Erfindung betrifft ein Verfahren zum Festlegen bzw. Bestimmen der räumlichen Position eines Fern- und/oder eines Nahbezugspunkts eines progressiven Brillenglases sowie eine entsprechende Vorrichtung, ein Computerprogrammprodukt, ein Speichermedium und eine graphische Benutzerschnittstelle. Ferner bezieht sich die Erfindung auf ein Verfahren zum Bestimmen bzw. Berechnen eines individuellen Brillenglasdesigns sowie auf eine entsprechende Vorrichtung, ein Computerprogrammprodukt, ein Speichermedium und eine graphische Benutzerschnittstelle.

Individuelle Brillengläser, insbesondere progressive individuelle Brillengläser, sind in zahlreichen Patentveröffentlichungen beschrieben worden (beispielsweise DE 197 01 312, DE 103 13 275, WO 01/81979). Sie weisen deutlich bessere Abbildungseigenschaften als konventionelle Brillengläser auf, da die individuelle Gebrauchssituation des Brillenträgers bei der Berechnung und Optimierung berücksichtigt wird.

Die Berechnung und Optimierung von Brillengläsern und insbesondere von progressiven Brillengläsern (Gleitsichtgläsern) in der jeweiligen Gebrauchsstellung bzw. Gebrauchssituation hat mittlerweile einen sehr hohen technischen und optischen Standard erreicht. So können nach dem Stand der Technik Gleitsichtgläser unter Berücksichtigung der individuellen Verordnung (Sph, Zyl, Achse, Add, Prisma, Basis) und der individuellen Lage der Gläser vor dem Auge (Hornhautscheitelabstand HSA, Fassungsscheibenwinkel FSW, Vorneigung VN, Pupillendistanz PD) online nach Bestelleingang optimiert und berechnet werden.

Aufgabe dieser Erfindung ist es, die Optimierung von individuellen progressiven Brillengläsern zu verbessern.

WO 01/57584 A2 offenbart ein Verfahren zur Herstellung von individuellen progressiven Brillengläsern, wobei zunächst Brillenglasrohlinge (Blanks) mit fertiger erster Fläche in einer bestimmten, vergleichsweise engen Abstufung des Flächenbrechwerts hergestellt werden. Ausgehend von den individuellen Daten eines Brillenträgers, nämlich zumindest der jeweils benötigten ersten Wirkung D_{f}, der Addition Add und gegebenenfalls dem Wert und der Achslage des Augenastigmatismus des Brillenträgers, wird aufgrund weiterer Designdaten eine erste Fläche mit einem bestimmten Flächenbrechwert D₁ ausgewählt. Die zweite Fläche wird derart berechnet, dass der im ersten Bezugspunkt benötigte Flächenbrechwert D_{2f} der zweiten Fläche entsprechend dem jeweils gewählten Flächenbrechwert D₁ eingestellt ist, wobei sich in Abhängigkeit von den jeweiligen Designdaten für ein- und dieselbe erste Wirkung D_{f} und ein- und dieselbe Addition Add sowie gegebenenfalls ein- und denselben Wert und Achslage des Augenastigmatismus unterschiedliche Paarungen von ersten Flächen und von zugeordneten, jeweils individuell berechneten zweiten Flächen ergeben. In die Berechnung der zweiten Fläche können individuelle Daten des Brillenträgers berücksichtigt werden.

Aufgabe dieser Erfindung ist es, die Optimierung von individuellen progressiven Brillengläsern zu verbessern.

Diese Aufgabe wird durch ein Verfahren zum Festlegen bzw. Bestimmen der räumlichen Position eines Fern- und/oder eines Nahbezugspunkts eines progressiven Brillenglases mit den Merkmalen gemäß dem Anspruch 1, ein Computerprogrammerzeugnis mit den Merkmalen gemäß dem Anspruch 14, ein Speichermedium mit den Merkmalen gemäß dem Anspruch 15, eine Vorrichtung zum Bestimmen der räumlichen Position eines Fern- und/oder eines Nahbezugspunkts mit den Merkmalen gemäß Anspruch 16 sowie eine graphische Benutzerschnittstelle mit den Merkmalen gemäß Anspruch 17 gelöst. Ferner wird die Aufgabe durch ein Verfahren zum Bestimmen bzw. Berechnen eines individuellen Brillenglasdesigns mit den Merkmalen gemäß dem Anspruch 20, einem Computerprogrammerzeugnis mit den Merkmalen gemäß dem Anspruch 23, einem Speichermedium mit den Merkmalen gemäß dem Anspruch 24, einer Vorrichtung mit den Merkmalen gemäß Anspruch 25 sowie einer graphischen Benutzerschnittstelle mit den Merkmalen gemäß Anspruch 26 gelöst. Bevorzugte Ausführungsformen sind Gegenstand der abhängigen Ansprüche.

Erfindungsgemäß wird ein computerimplementiertes Verfahren zum Festlegen bzw. Bestimmen der räumlichen Position eines Fern- und/oder eines Nahbezugspunkts eines progressiven Brillenglases zur Korrektur einer Fehlsichtigkeit eines Brillenträgers bereitgestellt, wobei das Verfahren folgende Schritte umfaßt:
- Erfassen von individuellen Daten des Brillenträgers;
- Ermitteln bzw. Berechnen der individuellen vertikalen und/oder horizontalen Position des Fern- und des Nahbezugspunkts in Abhängigkeit von der erfaßten individuellen Daten des Brillenträgers,
wobei der vertikale Abstand des Fern- und des Nahbezugspunkts von einem Zentrier- bzw. Anpaßpunkt des Brillenglases in Abhängigkeit von den individuellen Daten des Brillenträgers festgelegt wird. Die individuellen Daten des Brillenträgers umfassen Daten bezüglich der Präferenzen bzw. der Gewichtungen des Fern- und des Nahbereichs und optional des Progressionsbereichs, wobei
- für den vertikalen Abstand *y_{BF}* des Fernbezugspunkts von dem Zentrier- bzw. Anpaßpunkt des Brillenglases gilt ${y}_{BF} = 4 - 4 * {G}_{F} / 33 , 33 \left[mm\right] f ⁢ {u}^{¨} ⁢ r 0 \leq {G}_{F} < 33$
   und ${y}_{BF} = 0 - 4 * \left({G}_{F}-33,33\right) / 66 , 66 \left[mm\right] f ⁢ {u}^{¨} ⁢ r 33 \leq {G}_{F} \leq 100 ;$
   und
- für den vertikalen Abstand *y_{BN}* des Nahbezugspunkts von dem Zentrier- bzw. Anpaßpunkt gilt ${y}_{BN} = - 20 + 2 * \left|2\right| ⁢ {G}_{N} / 33 \left[mm\right] ⁢ f ⁢ {u}^{¨} ⁢ r 0 \leq {G}_{N} < 33$
   und ${y}_{BN} = - 18 + 5 * \left({G}_{N}-33,33\right) / 66 , 66 \left[mm\right] f ⁢ {u}^{¨} ⁢ r 33 \leq {G}_{N} \leq 100 ;$
wobei *G_{F}* die Gewichtung des Fernbereichs und *G_{N}* die Gewichtung des Nahbereichs bezeichnen.

Bei progressiven Brillengläsern (Gleitsichtgläser) gibt es vier ausgezeichnete Punkte auf dem Brillenglas, die durch zumindest eine Permanentmarkierung auf bzw. in dem Brillenglas und eine vorbestimmte Rekonstruktionsvorschrift dieser vier Punkte relativ zu der Permanentmarkierung jederzeit rekonstruierbar sind (vgl. z.B. EN ISO 8989-2). Diese Punkte sind in der Norm (EN ISO 13666) und z.B. im Handbuch für Augenoptik, Zeiss, 4. Auflage 2000, S. 117, beschrieben. Die räumlichen Positionen der Bezugspunkte werden in der Regel ebenfalls mittels einer nicht permanenten Markierung (z.B. Stempelung) des Brillenglases aufgezeichnet (vgl. EN ISO 13 666 und DIN 58 208 Teil 2).

Der Fernbezugspunkt wird als derjenige Punkt auf der Vorderfläche, in dem die vorgeschriebene Fernwirkung herrschen soll, definiert, und zwar vorzugsweise in demjenigen Strahlengang, der beim Gebrauch des Brillenglases vor dem Auge vorliegt.

Der Nahbezugspunkt wird als derjenige Punkt auf der Vorderfläche, in dem die vorgeschriebene Nahwirkung herrschen soll, definiert, und zwar in demjenigen Strahlengang, der beim Gebrauch des Brillenglases vor dem Auge vorliegt.

Der Prismenbezugspunkt wird als der vom Hersteller angegebene Punkt auf der Vorderfläche, in dem die prismatischen Wirkungen des fertigen Glases bestimmt werden müssen, definiert.

Der Zentrierpunkt wird als der Punkt definiert, mit dem der Anpaßpunkt zusammen fallen soll, wobei der Anpaßpunkt ein Punkt auf der Vorderfläche eines progressiven Brillenglases ist, der nach Angabe des Herstellers als Bezugspunkt für die Positionierung dieses Brillenglases vor dem Auge dienen soll.

Gemäß den herkömmlichen Verfahren zur Herstellung von Brillengläsern und insbesondere von Gleitsichtbrillengläsern werden die Positionen der Bezugspunkte, insbesondere des Fern- und Nahbezugspunkts von den Herstellern fest für das jeweilige Brillenglas vorgegeben. Diese Positionen sind weitgehend unabhängig von den individuellen Daten des Brillenträgers, d.h. die Positionen der Bezugspunkte werden nicht abhängig von individuellen Daten des jeweiligen Brillenträgers gewählt und festgelegt.

Die Zentrierung der fertigen Brillengläser in der Brillenfassung kann nach verschiedenen Brillenzentrierforderungen erfolgen:
1. Blickfeldforderung
2. Drehpunktforderung
3. Bezugspunktforderung

Die Zentrierung von Brillengläsern ist z.B. in dem Buch "Optik und Technik der Brille", H. Diepes und R. Blendowske, 2002, Optische Fachveröffentlichung GmbH, Heidelberg, S. 308, auf welches ausdrücklich Bezug zur Erläuterung aller hier nicht näher definierten Begriffe genommen wird, näher erläutert.

Für die Zentrierung eines progressiven Brillenglases sind insbesondere die Blickfeldforderung und die Bezugspunktforderung von Bedeutung.

Die Blickfeldforderung besagt, daß die Blickfelder beider Augen sich bei habitueller Kopf- und Körperhaltung in der gewünschten Objektentfernung decken. Das Gleitsichtglas wird so zentriert, daß bei normaler habitueller Kopf- und Körperhaltung und Blick geradeaus (Nullblickrichtung) der Zentrierpunkt vor der Pupillenmitte liegt. Dadurch wird erreicht, daß in der Regel die Blickfeldforderung erfüllt wird.

Die Bezugspunktforderung besagt, daß der Bezugspunkt mit dem Hauptdurchblickpunkt bei beiden Augen zusammenfallen soll. Die Hauptblickrichtungen des Brillenträgers sind inhärente und individuelle Eigenschaften des Brillenträgers, während die Bezugspunkte auf dem Brillenglas festgelegt sind.

Erfindungsgemäß ist insbesondere erkannt worden, daß mit den herkömmlichen Herstellungsverfahren für Gleitsichtgläser mit (nicht-individuell) festgelegten Positionen von Fern- und Nahbezugspunkten die Bezugspunktforderung in der Regel nicht erfüllt wird, was zu einer Verschlechterung der Abbildungseigenschaften des Brillenglases bzw. der Brille und Ermüdung und Unverträglichkeiten führt. Insbesondere ist erfindungsgemäß erkannt worden, daß für die gemäß herkömmlicher Verfahren mit festgelegten Bezugspunkten hergestellten Gleitsichtgläser die Bezugspunktforderung nur dann erfüllt wird, wenn die Hauptblickrichtungen für Ferne und Nähe des Brillenträgers rein zufällig mit den vom Hersteller festgelegten Bezugspunkten auf dem Brillenglas zusammen fallen. In einer Vielzahl von Fällen fallen jedoch die Hauptblickrichtungen und die Lage der Fern- und Nahbezugspunkte nicht zusammen. Liegt beispielsweise der Fernbezugspunkt 4 mm oberhalb des Zentrierpunktes und der Nahbezugspunkt 18 mm unterhalb des Zentrierpunktes (Standardwerte bei konventionellen Brillengläsern), so müßte die Hauptblickrichtung "Ferne" ca. 8 Grad über der Nullblickrichtung und die Hauptblickrichtung "Nähe" ca. 36 Grad unter der Nullblickrichtung liegen. Weichen jedoch die individuellen Hauptblickrichtungen von diesen ab, so kann die Bezugspunktforderung nicht mehr erfüllt werden und der Brillenträger muß seine Hauptblickrichtungen nach dem Brillenglas richten.

Die Erfindung bricht mit der herkömmlichen Vorgehensweise, bei der die räumliche Lage, insbesondere die vertikale Lage der Bezugspunkte, festgelegt ist. Statt dessen wird vorgeschlagen, die räumliche Lage, insbesondere die vertikale Position der Fern- und Nahbezugspunkte, frei (in gewissen Grenzen) in Abhängigkeit von den individuellen Daten des Brillenträgers zu variieren und speziell für den jeweiligen, individuellen Brillenträger festzulegen.

Nachdem die optimale, individuelle Position des Fern- und/oder Nahbezugspunkts ermittelt wurde, wird das Brillenglas derart optimiert, daß die vorgeschriebenen bzw. für den Brillenträger erforderlichen Fern- und Nahteilwirkungen in den so festgelegten individuellen Bezugspunkten für die Ferne und Nähe erreicht werden. Dabei bezieht sich die Wirkung vorzugsweise auf die Gebrauchswirkung, d.h. die Wirkung des Brillenglases in Gebrauchsstellung. Die Positionen der individuell ermittelten Fern- und/oder Nahbezugspunkte gehen dabei als Designparameter bei der Berechnung und Optimierung des individuellen Brillenglasdesigns bzw. des individuellen Brillenglases ein. Die individuell festlegbaren Fern- und Nahbezugspunkte werden daher auch nachfolgend jeweils als Designpunkt "Ferne" bzw. Designpunkt "Nähe" bezeichnet.

Insbesondere entspricht der individuell ermittelte Fernbezugspunkt bzw. Designpunkt "Ferne" dem Punkt, durch den der Brillenglasträger beim Blick in die Ferne optimal korrigiert ist und welcher seinen persönlichen Sehgewohnheiten entspricht. Der individuell ermittelte Nahbezugpunkt bzw. Designpunkt "Nähe" entspricht dem Punkt, durch den der Brillenglasträger bei Nahsehaufgaben optimal korrigiert ist und die für ihn angenehme Blicksenkung einnehmen kann.

Mit dem erfindungsgemäßen Verfahren kann sichergestellt werden, daß die Fern- und Nahbezugspunkte bzw. Designpunkte "Ferne" und "Nähe" eines erfindungsgemäß optimierten und hergestellten Brillenglases und die Hauptblickrichtungen eines Brillenträgers zusammenfallen.

Die Möglichkeit die Position des Fern- und/oder Nahbezugspunkts in bestimmten Grenzen frei wählen zu können, stellt einen wesentlichen zusätzlichen Freiheitsgrad bei der Festlegung des individuellen Designs des Brillenglases dar. Somit kann die Lage, insbesondere die vertikale Position, und gegebenenfalls die Größe der Sehbereiche (Fern-, Nah- und Zwischen- bzw. Progressionsbereich) in bestimmten Grenzen frei bzw. individuell gewählt und an die jeweilige individuelle Benutzersituation optimal angepaßt werden. Da der Fern- und der Nahbezugspunkt des Brillenglases optimal an die Hauptblickrichtungen des Brillenträgers angepaßt werden können, ist es möglich optimale, bedürfnisorientierte Sehbereiche und somit einen optimalen Sehkomfort mit dem Brillenglas zu erzielen. Ferner werden die Augen und die Halswirbelsäule entlastet und die Augenermüdung verringert.

Wie oben erläutert, werden die räumlichen Positionen des individuell ermittelten Fern- und/oder Nahbezugspunkts sowie des Anpaßpunkts beispielsweise mittels einer nicht permanenten Markierung (z.B. Stempelung) des fertigen individuellen Brillenglases aufgezeichnet. Die Positionen der individuell ermittelten Fern- und Nahbezugspunkte und des Zentrier- bzw. Anpaßpunkts können ebenfalls mittels geeigneten, dem individuellen Brillenglas zugeordneten Rekonstruktionsvorschriften (individuelle Schablonen, Zentrierkarten, etc.) anhand der permanenten Markierungen des Brillenglases (vgl. EN ISO 8989-2, Punkt 7.1) eindeutig rekonstruiert werden. Die Positionen der Fern- und Nahbezugspunkte und des Zentrier- bzw. Anpaßpunkts lassen sich somit anhand von permanenten Markierungen des Brillengases eindeutig rekonstruieren. Gemäß einem Aspekt der Erfindung wird somit das System umfassend ein bevorzugtes individuelles Brillenglas, insbesondere hergestellt durch ein bevorzugtes erfindungsgemäßes Verfahren und eine diesem Brillenglas zugeordnete individuelle Rekonstruktionsvorschrift geschützt.

Die individuellen Daten des Brillenträgers, welche bei der Bestimmung der räumlichen Position, insbesondere der vertikalen Position des Fern- und/oder Nahbezugspunkts, berücksichtigt werden, umfassen ein oder mehrere der folgenden Daten bzw. Datensätze:
- Refraktionsdaten bzw. Wirkungsparameter, insbesondere Sphäre, Zylinder, Achslage, Addition; und/oder
- Individualparameter des Brillenträgers und der individuellen Gebrauchsstellung des Brillenglases bzw. der Brille vor den Augen des Brillenträgers. Die individuellen Parameter umfassen insbesondere die Pupillendistanz, den Hornhautscheitelabstand (HSA), Vorneigung (VN), Fassungsscheibenwinkel (FSW), etc.; und/oder
- Daten bezüglich der Präferenzen bzw. der Gewichtung des Fern-, Nah- und des Zwischen- bzw. Progressionsbereichs des progressiven Brillenglases; und/oder
- Daten bezüglich des bisher getragenen Brillenglases, insbesondere ob das bisherige Brillenglas ein Einstärken-, Bifokal- oder Gleitsichtbrillenglas ist, Daten bezüglich des Designs (Hart/Weich), der Progressionslänge, des Typs (Individuell, Konventionell), des Materials (Kunststoff/Silikat), des Brechungsindexes, der Lage der Bezugspunkte, der Addition des Vorgängerglases, und/oder der Veränderung der Refraktionsdaten im Vergleich zu den Refraktionsdaten des Vorgängerglases, und/oder
- Daten bezüglich der Verbesserungswünsche gegenüber der bisher getragenen Brille, insbesondere größerer Fernbereich, größerer Zwischenbereich, größerer Nahbereich, geringere Blicksenkung beim Lesen oder geringere Schaukelbewegung; und/oder
- Daten bezüglich der Hauptnutzung des Brillenglases (Autofahren, Computerarbeitsplatz, Lesen, Handwerken, etc); und/oder
- Daten bezüglich der Umwelteinflüsse (Umgebungshelligkeit etc.); und/oder
- Daten bezüglich der Hauptblickrichtung in der Ferne und der Nähe;
- Daten bezüglich eventuell vorhandener individueller, außergewöhnlicher Kopf- und Körperhaltungen; und/oder
- Fassungs- und Zentrierdaten, insbesondere Scheibenhöhe und Zentrierhöhe, Fassungsform; Abstand zwischen den Gläsern, Korrigierte PD zum Einschleifen (CorPD); und/oder
- Physiologische Parameter, insbesondere des Auges bzw. der Augen des Brillenträges, insbesondere Sehschärfe mit Korrektion, Stereogrenzwinkel; und/oder
- Daten bezüglich der individuellen Objektabstände, insbesondere Arbeitsabstand beim Lesen (Naharbeit), Arbeitsabstand in der Ferne; und/oder
- Daten bezüglich der Objektabstände bei der Refraktionsbestimmung: "Ferne" und "Nähe".

Die individuellen Daten des Brillenträgers können ferner weitere individuelle Parameter umfassen.

Die individuellen Daten des Brillenträgers werden erfaßt, ausgewertet und steuern bzw. bestimmen die räumliche Lage des individuellen Fern- und/oder Nahbezugspunkts.

Vorzugsweise wird der vertikale Abstand des Fern- und des Nahbezugspunkts von einem vorgegebenen bzw. vorgebbaren Zentrier- bzw. Anpaßpunkt des Brillenglases in Abhängigkeit von den individuellen Daten des Brillenträgers festgelegt. Das Verfahren zum Festlegen bzw. Bestimmen der räumlichen Position eines Fern- und/oder eines Nahbezugspunkts umfaßt vorzugsweise einen Schritt des Festlegens der Position des Zentrier- bzw. Anpaßpunkts.

Vorzugsweise kann:
- die vertikale Höhe des Fernbezugspunkts gemessen von dem Zentrier- bzw. Anpaßpunkt des Brillenglases auf einen Wert von -4 mm unterhalb bis 4 mm oberhalb des Zentrier- bzw. Anpaßpunkts, vorzugsweise in 0,1 mm Stufen, in Abhängigkeit von den individuellen Daten des Brillenträgers festgelegt werden; und/oder
- die vertikale Höhe des Nahbezugspunkts gemessen von dem Zentrierpunkt des Brillenglases auf einen Wert von -13 mm bis -20 mm unterhalb des Zentrier- bzw. Anpaßpunkts, vorzugsweise in 0,1 mm Stufen, in Abhängigkeit von den individuellen Daten des Brillenträgers festgelegt werden.

Die vertikale Richtung bezieht sich vorzugsweise auf die vertikale Richtung in Gebrauchsstellung des Brillenglases, wobei das Brillenglas beispielsweise in einer durchschnittlichen (wie z.B. in DIN 58 208 Teil 2 definiert) oder in einer individuellen Gebrauchsstellung angeordnet ist. Vorzugsweise ist das Brillenglas in einer individuellen Gebrauchsstellung angeordnet. Das Koordinatensystem ist vorzugsweise ein kartesisches Koordinatensystem in der objektseitigen Fläche des Brillenglases, wobei der Ursprung des Koordinatensystems mit dem Zentrier- bzw. Anpaßpunkt des Brillenglases zusammenfällt. Die vertikale und horizontale Achse liegen in der tangentialen Ebene zu der objektseitigen Fläche in dem geometrischen Mittelpunkt bzw. in dem Zentrier- bzw. Anpaßpunkt.

Ferner bevorzugt ist der Abstand zwischen der vertikalen Höhen des Fern- und des Nahbezugspunkts (d.h. die Progressionszonenlänge) größer oder gleich 13 mm. Weiter bevorzugt befindet sich der Nahbezugspunkt mindestens 2 mm über dem unteren Fassungsrand und/oder der Fernbezugspunkt mindestens 8 mm unter dem oberen Fassungsrand.

Die Position des individuellen Fern- und Nahbezugspunkts kann beispielsweise von dem Optiker/Optometristen mit der Bestellung direkt angegeben werden. Vorzugsweise wird jedoch die optimale Position des Fern- und/oder Nahbezugspunkts aus den individuellen Daten des Brillenträgers mit Hilfe eines Computers automatisch berechnet.

Die individuellen Daten des Brillenträgers umfassen Daten bezüglich der Präferenzen bzw. der Gewichtung des Fern-, Nah- und/oder Progressionsbereichs. Dann gilt:
- für den vertikalen Abstand *y_{BF}* des Fernbezugspunkts von dem Zentrier- bzw. Anpaßpunkt des Brillenglases ${y}_{BF} = 4 - 4 * {G}_{F} / 33 , 33 \left[mm\right] f ⁢ {u}^{¨} ⁢ r 0 \leq {G}_{F} < 33$
   und ${y}_{BF} = 0 - 4 * \left({G}_{F}-33,33\right) / 66 , 66 \left[mm\right] f ⁢ {u}^{¨} ⁢ r 33 \leq {G}_{F} \leq 100 ;$
   und/oder
- für den vertikalen Abstand *y_{BN}* des Nahbezugspunkts von dem Zentrier- bzw. Anpaßpunkt ${y}_{BN} = - 20 + 2 * {G}_{N} / 33 \left[mm\right] ⁢ f ⁢ {u}^{¨} ⁢ r 0 \leq {G}_{N} < 33$
   und ${y}_{BN} = - 18 + 5 * \left({G}_{N}-33,33\right) / 66 , 66 \left[mm\right] f ⁢ {u}^{¨} ⁢ r 33 \leq {G}_{N} \leq 100 ;$
wobei *G_{F}* die Gewichtung des Fernbereichs und *G_{N}* die Gewichtung des Nahbereichs bezeichnen.

Gemäß einer weiteren Ausführungsform kann der vertikale Abstand *y_{BF}* des Fernbezugspunkts von dem für dieses Brillenglas vorgegebenen Zentrierpunkt bzw. Anpaßpunkt und der vertikale Abstand *y_{BN}* des Nahbezugspunkts von dem Zentrierpunkt bzw. Anpaßpunkt gemäß den folgenden Formeln berechnet werden: ${y}_{BF} = \left(GW⁢1*{y}_{BF ⁢ 1}+{y}_{BF ⁢ 2}\right) / \left({g}_{1}\right)$ ${y}_{BN} = \left(GW⁢2*{y}_{BN ⁢ 1}+{y}_{BN ⁢ 2}\right) / \left({g}_{2}\right)$
wobei ${y}_{BF ⁢ 1} = 4 - 4 * {G}_{F} / 33 , 33 \left[mm\right] f ⁢ {u}^{¨} ⁢ r 0 \leq {G}_{F} < 33$
und ${y}_{BF ⁢ 1} = 0 - 4 * \left({G}_{F}-33,33\right) / 66 , 66 \left[mm\right] f ⁢ {u}^{¨} ⁢ r 33 \leq {G}_{F} \leq 100 ;$ ${y}_{BN ⁢ 1} = - 20 + 2 * {G}_{N} / 33 \left[mm\right] f ⁢ {u}^{¨} ⁢ r 0 \leq {G}_{N} < 33$
und ${y}_{BN ⁢ 1} = - 18 + 5 * \left({G}_{N}-33,33\right) / 66 , 66 \left[mm\right] f ⁢ {u}^{¨} ⁢ r 33 \leq {G}_{N} \leq 100 ;$ ${y}_{BF ⁢ 2} = {y}_{BN ⁢ 1} + {y}_{P}$ ${y}_{BN ⁢ 2} = {y}_{BF ⁢ 1} - {y}_{P}$ ${y}_{P} = 13 + 5 * {G}_{P} / 33 , 33 \left[mm\right] f ⁢ {u}^{¨} ⁢ r 0 \leq {G}_{P} < 33$
und ${y}_{P} = 18 + 2 * \left({G}_{P}-33,33\right) / 66 , 66 \left[mm\right] f ⁢ {u}^{¨} ⁢ r 33 \leq {G}_{P} \leq 100 ;$
wobei:
die Koeffizienten *GW1* und GW2 Werte zwischen 1 und 2 annehmen und wobei
- *G_{F}*: die Gewichtung des Fernbereichs;
- *G_{N}*: die Gewichtung des Nahbereichs; und
- *G_{P}*: die Gewichtung des Zwischen- bzw. Progressionsbereichs
${g}_{1} = 1 + GW ⁢ 1$ ${g}_{2} = 1 + GW ⁢ 2$
bezeichnen.

Vorzugsweise umfassen die individuellen Daten des Brillenträgers individuelle Parameter der Augen des Brillenträgers und/oder der Anordnung der Brille vor den Augen des Brillenträgers. Insbesondere umfassen die individuellen Parameter der Augen des Brillenträgers und/oder die Anordnung der Brille vor den Augen des Brillenträgers die Pupillendistanz, den Hornhautscheitelabstand, die Vorneigung, den Fassungsscheibenwinkel, etc..
Die räumliche Lage, insbesondere der vertikale Abstand des Fern- und/oder des Nahbezugspunkts von dem Zentrierpunkt, hängt insbesondere von der Vorneigung und dem Hornhautscheitelabstand ab. Der Standardwert bzw. der Wert, welcher einem Standard- bzw. einem Grunddesign zugrunde liegt, beträgt beispielsweise für Vorneigung 9 Grad und für den Hornhautscheitelabstand 13 mm. Weichen die tatsächlichen Werte (HSA und Vorneigung) von diesen ab, wird erfindungsgemäß die vertikale Position der Bezugspunkte entsprechend angepaßt, um z.B. extrem hohe Blickauslenkungen beim Nahsehen bei kleinen HSA-Werten zu vermeiden.

Dies kann z.B. mit folgender Formel erfolgen: ${y}_{Bneu} = {y}_{Balt} * \left(HSA+13,5 mm\right) / 26 , 5 mm * Cosinus \left(Vorneigung\right) / Cosinus \left(9 Grad\right) ,$
wobei:
- y_{Bneu}: die neue vertikale Position des Fern- bzw. des Nahbezugspunkts
- y_{balt}: die vertikale Position des Fern- bzw. des Nahbezugspunkts gemäß dem Standarddesign;
bezeichnen.

Ferner bevorzugt umfassen die individuellen Daten des Brillenträgers Daten bezüglich
- des individuellen Objektabstands für die Ferne und/oder des individuellen Objektabstands für die Nähe; und/oder
- des individuellen Objektabstands für die Ferne bei der Refraktionsbestimmung und/oder des individuellen Objektabstands für die Nähe bei der Refraktionsbestimmung.

Vorzugsweise umfassen die individuellen Daten des Brillenträgers:
- Daten bezüglich einer bisher getragenen Brille; und/oder
- Daten bezüglich Verbesserungswünsche zu der bisherigen getragenen Brille.

Weiter bevorzugt umfassen die individuellen Daten des Brillenträgers:
- Daten bezüglich der individuellen Hauptblickrichtung für die Ferne und die Nähe; und/oder
- Daten bezüglich der individuellen Kopf- und Körperhaltung; und/oder
- physiologische Parameter, insbesondere des Auges des Brillenträgers; und/oder
- Präferenzen bzw. eine Gewichtung der Wichtigkeit der Abbildungseigenschaften gegenüber den ästhetischen Eigenschaften des Brillenglases.

Gemäß einer weiteren bevorzugten Ausführungsform des erfindungsgemäßen Verfahrens umfassen die individuellen Daten des Brillenträgers Daten in zumindest zwei unterschiedlichen Kategorien der individuellen Daten und das Ermitteln der Position des Fern- und/oder des Nahbezugspunkts folgende Schritte
- Ermitteln einer idealen Position des Fern- und/oder einer idealen Position des Nahbezugspunkts für jede der Kategorien anhand von individuellen Daten des Brillenträgers in der jeweiligen Kategorie;
- Berechnen der Position des Fern- und/oder der Position des Nahbezugspunkts anhand der ermittelten idealen Positionen des Fern- und/oder Nahbezugspunkts in den jeweiligen Kategorien.

Vorzugsweise wird die Position des Fern- und/oder des Nahbezugspunkts nach der Formel: ${y}_{DF , DN} = {\sum }_{i = 1}^{N} {g}_{i} ⁢ {y}_{DF , DN}^{i}$ ${\sum }_{i = 1}^{N} {g}_{i} = 1$
berechnet, wobei:
- *gᵢ*: die Gewichtung der *i* -ten Kategorie;
- *yⁱ_{DF,DN}*: die ideale Position des Fernbezugspunkts DF bzw. des Nahbezugspunkts DN für die *i* -te Kategorie ; und
- N: die Anzahl der unterschiedlichen Kategorien
bezeichnen.

Vorzugsweise umfaßt das erfindungsgemäße Verfahren ferner die Schritte
- Berechnen eines individuellen Brillenglasdesigns, welches den individuell festgelegten Fern- und/oder Nahbezugspunkt aufweist;
- Visualisierung des berechneten individuellen Brillenglasdesigns und der räumlichen Position des individuellen Fern- und/oder Nahbezugspunkts.

Gemäß der Erfindung wird ferner ein Computerprogrammerzeugnis sowie ein Speichermedium mit darauf gespeichertem Computerprogramm bereitgestellt, wobei das Computerprogramm ausgelegt ist, wenn geladen und ausgeführt auf einem Computer, das erfindungsgemäße Verfahren zum Festlegen bzw. Bestimmen der individuellen räumlichen Position eines Fern- und/oder eines Nahbezugspunkts eines progressiven Brillenglases durchzuführen.

Gemäß der Erfindung wird ferner eine Vorrichtung zum Bestimmen der individuellen räumlichen Position eines Fern- und/oder eines Nahbezugspunkts eines progressiven Brillenglases bereitgestellt, umfassend:
- Erfassungsmittel zum Erfassen von individuellen Daten des Brillenträgers;
- Berechnungs- bzw. Optimierungsmittel, welche derart ausgelegt sind, ein bevorzugtes erfindungsgemäßes Verfahren zum Bestimmen der räumlichen Position des Fern- und/oder des Nahbezugspunkts in Abhängigkeit von den erfaßten individuellen Daten des Brillenträgers durchzuführen.

Gemäß der Erfindung wird ferner eine grafische Benutzerschnittstelle zum Festlegen bzw. Bestimmen und Darstellen der räumlichen Position eines individuellen Fern- und/oder eines Nahbezugspunkts eines progressiven Brillenglases bereitgestellt, umfassend
- zumindest einen Individualdaten-Eingabeabschnitt, welcher ausgelegt ist, individuelle Daten des Brillenträgers einzugeben; und
- zumindest einen Anzeigeabschnitt, welcher ausgelegt ist, die räumliche Position des Fern- und/oder Nahbezugspunkts darzustellen, wobei die räumliche Position des Fern- und/oder Nahbezugspunkts nach einem bevorzugten erfindungsgemäßen Verfahren in Abhängigkeit von den individuellen Daten des Brillenträgers ermittelt wird.

Vorzugsweise ist der Anzeigeabschnitt ferner ausgelegt ein individuelles Brillenglasdesign darzustellen, wobei das individuelle Brillenglasdesign den individuell festgelegten Fern- und/oder Nahbezugspunkt aufweist.

Vorzugsweise umfaßt die grafische Benutzerschnittstelle einen Tuning- bzw. Anpassungsabschnitt, welcher ausgelegt ist eine Anpassung der vertikalen und/oder der horizontalen Position des Fern- und/oder Nahbezugspunkts und/oder eine Anpassung zumindest eines Teils der individuellen Parameter des Brillenträgers durchzuführen.

Gemäß der Erfindung wird ferner ein computerimplementiertes Verfahren zum Bestimmen bzw. Berechnen eines individuellen Brillenglasdesigns für ein progressives Brillenglas zur Korrektur einer Fehlsichtigkeit eines bestimmten Brillenträgers vorgeschlagen, umfassend folgende Schritte:
- Erfassen von individuellen Daten des Brillenträgers;
- Ermitteln bzw. Berechnen der räumlichen Position eines Fern- und/oder eines Nahbezugspunkts in Abhängigkeit von den erfaßten individuellen Daten des Brillenträgers nach einem bevorzugten erfindungsgemäßen Verfahren;
- Berechnen der räumlichen Lage und/oder der Größe eines Fern-, Nah- und Progressionsbereichs des Brillenglasdesigns in Abhängigkeit von der ermittelten individuellen räumlichen Position des Fern- und/oder des Nahbezugspunkts.

Ein Design eines Brillenglases umfaßt insbesondere die räumliche Verteilung der Soll-Werte über das Brillenglas für eine oder mehrere Abbildungsfehler, welche in die Optimierung des Brillenglases als Zielwerte eingehen. Insbesondere wird ein Brillenglasdesign durch die Verteilung des Refraktionsfehlers (d.h. die Differenz des Brechwerts des Brillenglases von dem Brechwert, welcher mittels Refraktionsbestimmung ermittelt wird) und/oder die Verteilung des Astigmatismusfehlers bzw. die astigmatische Abweichung (d.h. die Differenz des Astigmatismus des Brillenglases von dem Astigmatismus, welcher mittels Refraktionsbestimmung ermittelt wird) charakterisiert. Ferner kann ein Brillenglasdesign ebenfalls die Verteilung der Soll-Werte für Vergrößerungs-, Verzerrungs- oder anderen Abbildungsfehler umfassen, dabei kann es sich um Flächenwerte oder vorzugsweise um Gebrauchswerte, d.h. Werte in Gebrauchsstellung des Brillenglases handeln. Darüber hinaus kann das Brillenglasdesign ein geeignetes Objektmodell umfassen. Das Objektmodell kann beispielsweise eine Objektabstandsfunktion, welche als die reziproke Objektentfernung entlang der Hauptlinie definiert ist, umfassen. Ein Beispiel eines geeigneten Objektmodells ist in DIN 58 208 Teil 2 (vgl. Bild 6) definiert. Ebenfalls ist in DIN 58 208 Teil 2 eine normierte Gebrauchsstellung definiert.

Ein Design weist eine vorgegebene räumliche Position des Fern- und/oder Nahbezugspunkts auf, wenn in dem jeweiligen Bezugspunkt die vorgeschriebenen bzw. für den Brillenträger erforderlichen Werte für die Fern- und/oder Nahteilwirkungen (welche z.B. mittels Refraktionsbestimmung ermittelt werden) erreicht werden. Anders ausgedrückt, sollen im Fern- und/oder Nahbezugspunkt die dem Design zugeordneten Abbildungsfehler (insbesondere astigmatische Abweichung und Refraktionsfehler) möglichst klein (vorzugsweise im wesentlichen Null) sein.

Um Gleitsichtgläser mit unterschiedlichen Designs, d.h. mit unterschiedlichen Sollvorgaben für die Abbildungsfehler (insbesondere Sollvorgaben für die räumliche Verteilung der astigmatischen Abweichung und/oder des Refraktionsfehlers), welche z.B. durch unterschiedlichen Positionen des Fern- und/oder Nahbezugspunkt bedingt sind, zu erzeugen, müssen die entsprechenden unterschiedlichen Design- bzw. Sollvorgaben erzeugt und damit die Optimierungen durchgeführt werden. Berechnen eines individuellen Brillenglasdesigns im Sinne dieser Anmeldung umfaßt folglich die Berechnung der Sollvorgaben bzw. Sollwerten für die einzelnen Abbildungsfehler, welche dem individuellen Brillenglasdesign zuzuordnen sind, insbesondere der Sollvorgaben für die astigmatische Abweichung und/oder den Refraktionsfehler.

Ein progressives Brillenglasdesign umfaßt in der Regel einen Fern-, einen Nah- und einen Zwischen- bzw. Progressionsbereich. Die 0,5-Dpt. Isoastigmatismuslinie wird in der Regel zur Abgrenzung der einzelnen Sehbereiche gegenüber der Peripherie herangezogen. Es ist jedoch möglich andere Isoastigmatismuslinien, wie z.B. die 0,75 oder 1,0 dpt Isoastigmatismusline zur Abgrenzung der Sehbereiche herangezogen werden. Die Sehbereiche umfassen folglich die Fläche zwischen der temporalen und der nasalen Isoastigmatismuslinie, vorzugsweise zwischen der nasalen und der temporalen 0,5 dpt Isoastigmatismusline. In horizontaler Richtung werden die Sehbereiche folglich jeweils durch die nasale und temporale Isoastigmatismusline (z. B. 0,5 dpt Isoastigmatismuslinie) von der Peripherie abgegrenzt.

Die horizontale Linie (x, y = y_{FP}), welche durch den Punkt (x_{FP},y_{FP}) auf der Hauptlinie verläuft, in welchem einen Brechwertanstieg von 15% der Addition erreicht wird, kann z.B. zur Abgrenzung des Progressionsbereichs vom Fernbereich in vertikaler Richtung dienen. Alle Punkte auf der und oberhalb der horizontalen Linie (x, y = y_{FP}), welche einen Sollastigmatismus ≤ 0,5 dpt aufweisen, können somit dem Fernbereich zugeordnet werden.

Die horizontale Linie (x, y = y_{NP}), welche durch den Punkt (x_{NP},y_{NP}) auf der Hauptlinie verläuft, in welchem einen Brechwertanstieg von 85% der Addition erreicht wird, kann zur Abgrenzung des Progressionsbereichs vom Nahbereich in vertikaler Richtung dienen. Alle Punkte auf der und unterhalb der horizontalen Linie (x, y = y_{NP}), welche einen Sollastigmatismus ≤ 0,5 dpt aufweisen, können somit dem Nahbereich zugeordnet werden.

Dem Progressionsbereich werden alle Punkte (x,y) zwischen den beiden horizontalen Linien (x, y = y_{FP}) und (x, y = y_{NP}), welche einen Sollastigmatismus ≤ 0,5 dpt aufweisen, zugeordnet. Selbstverständlich können die Sehbereiche in anderer geeigneter Weise horizontal voneinander abgegrenzt werden.

Der Brechwertanstieg in einem Punkt auf der Hauptlinie ist die Differenz des Sollbrechwerts in diesem Punkt und dem Brechwert im Fernbezugspunkt. Die Addition ist als der Unterschied zwischen den Sollbrechwerten im Fern- und Nahbezugspunkt definiert.

Das Koordinatensystem bezieht sich vorzugsweise auf das oben definierte Koordinatensystem.

Das Berechnen eines Brillenglasdesigns umfaßt folglich insbesondere die Bestimmung der räumlichen Lage (insbesondere der vertikalen und/oder der horizontalen Lage) sowie gegebenenfalls der Größe der Sehbereiche des Brillenglases (d.h. des Nah-, Fern- und Zwischen- oder Progressionsbereichs) in Abhängigkeit von der ermittelten individuellen räumlichen Position des Fern- und/oder Nahbezugspunkts. Die räumliche Lage der Sehbereiche wird insbesondere von der räumlichen Position der Fern- und Nahbezugspunkte vorgegeben. Die Größe der Sehbereiche wird vorzugsweise automatisch aus den Vorgaben für die räumliche Position des Fern- und Nahbezugspunkts berechnet.

Hier wird unter der Größe des jeweiligen Sehbereichs insbesondere die Fläche des jeweiligen Sehbereichs verstanden. Unter räumlicher Lage des jeweiligen Sehbereichs wird insbesondere der Flächenschwerpunkt des jeweiligen Sehbereichs verstanden.

Das von der variablen Position des (individuellen) Fern- und/oder Nahbezugspunkts variabel abhängige Brillenglasdesign kann jedes Mal neu für jede Position des Fern- und/oder Nahbezugspunkts berechnet werden. Alternativ können für jede Kombination der Positionen der Fern- und/oder Nahbezugspunkte, vorzugsweise unter Berücksichtigung von individuellen Parametern der Augen der Brillenträger und/oder der individuellen Gebrauchssituationen, individuelle Brillenglasdesigns vorberechnet und beispielsweise in einer Datenbank gespeichert werden. Vorzugsweise erfolgt jedoch die Berechnung des individuellen Brillenglasdesigns mit variabler Position des Fern- und/oder Nahbezugspunkts mittels einer Transformation eines vorgegebenen Grund- bzw. Startdesigns.

Ein Verfahren zum Berechnen eines individuellen Designs mittels einer Transformation eines vorgegebenen Grund- bzw. Startdesigns ist zum Beispiel in der deutschen Patentanmeldung DE 10 2007 003 849 beschrieben.

Vorzugsweise wird das Brillenglasdesign mittels einer Transformation (z.B. einer geeigneten Streckung oder Stauchung) eines vorgegebenen Grund- bzw. Startdesigns berechnet, wobei die Transformation eine Funktion der vertikalen und/oder der horizontalen räumlichen Position des festgelegten Fern- und/oder Nahbezugspunkts ist.

Beispielsweise können die Sollvorgaben S(y) für die räumliche Verteilung mindestens eines Abbildungsfehlers (beispielsweise des astigmatischen Fehlers) des individuellen Brillenglases mittels einer Zuordnung *S*(*y*') = *S*'(*y*') und einer Transformation Y: y ↦ y',y ↦ y'(y) = y-Δy(y) einer entsprechenden Sollvorgabe S'(y') des Grund- bzw. Startdesigns berechnet werden, wobei *y*' die vertikale Koordinate der Sollvorgabe des Startdesigns und y die vertikale Koordinate der transformierten Sollvorgabe des individuellen Brillenglasdesigns bezeichnen.

Die vertikale Richtung bezieht sich vorzugsweise auf die vertikale Richtung in Gebrauchsstellung des Brillenglases, wobei das Brillenglas beispielsweise in einer durchschnittlichen (wie z.B. in DIN 58 208 Teil 2 definiert) oder in einer individuellen Gebrauchsstellung angeordnet ist. Vorzugsweise ist das Brillenglas in einer individuellen Gebrauchsstellung angeordnet. Das Koordinatensystem ist vorzugsweise das oben definierte Koordinatensystem. Es ist selbstverständlich möglich, die Transformation in anderen geeigneten Koordinatensystemen zu definieren.

Im einfachsten Fall kann die Transformation Y der Form Δ*y*(*y*) = y₀ sein, wobei y₀ eine Konstante bezeichnet.

Wird das Strecken oder Stauchen und das Verschieben z.B. durch eine affine Funktion der Form Δ*y*(*y*) = *αy* + y₀ gesteuert, dann sind alle Bereiche im Fern- und Nahteil sowie in der Progressionszone gleichmäßig von der Änderung betroffen. Oft ist es jedoch bevorzugt, bestimmte Eigenschaften des Fern- oder des Nahteils zu erhalten und nur die Progressionszone in ihrer Länge zu variieren. Vorzugsweise wird dann eine Funktion Δ*y*(*y*) gewählt, welche bestimmte Eigenschaften aufweist, wie z.B. in der Progressionszone steiler verläuft als im Nah- oder Fernbereich, und allgemein eine Funktion der Form Δy(y) = *f*(*y*) + y₀ ist. Ein positiver Wert der Ableitung *f'*(y) steht für eine lokale Streckung, ein negativer Wert für eine lokale Stauchung.

Vorzugsweise ist f (y) monoton und ebenso ist vorzugsweise |*f*'(y)| in der Progressionszone höher als im Nah- oder Fernbereich, so daß diese stärker vom Strecken oder Stauchen betroffen ist. Vorzugsweise hängt die Transformation Y von der Differenz der vertikalen Position des Fern- und/oder des Nahbezugspunkts des individuellen Brillenglasdesigns und der Differenz der vertikalen Position des Fern- und/oder des Nahbezugspunkts des Startdesigns ab.

Für die Funktion *f*(y) gibt es verschiedene Möglichkeiten der Parametrisierung.

Beispiele geeigneter Funktionen sind:
a) *f*(y)=*αy,* wobei *α* eine Konstante bezeichnet;
b) Doppelasymptote mit Transformationskoeffizienten *a, b, c, m, d :* $f \left(y\right) = b + \frac{a}{{\left(1+{e}^{c ⁢ \left(y+d\right)}\right)}^{m}}$
c) Sigmoid $f \left(y\right) = \frac{a}{1 + {e}^{c ⁢ \left(y+d\right)}}$ (Spezialfall der Doppelasymptote)
d) Gauß'sche Kumulative mit Transformationskoeffizienten *a, b, c* : $f \left(y\right) = \frac{a}{2} ⁢ \left(1+erf⁢\left(\frac{y - b}{\sqrt{2} ⁢ c}\right)\right)$
d) Lorentz'sche Kumulative mit Transformationskoeffizienten *a, b, c* : $f \left(y\right) = \frac{a}{π} ⁢ \left(arctan\frac{y - b}{c}\right) + \frac{π}{2}$
f) Kumulative SDS Funktion mit Transformationskoeffizienten *a, b, c, d* : $f \left(y\right) = \frac{a}{2 ⁢ c} ⁢ \left(2⁢d⁢ln⁢\left(exp\left(\frac{2 ⁢ y + c}{2 ⁢ d}\right)+exp\left(\frac{b}{d}\right)\right)-2⁢d⁢ln⁢\left(exp\left(\frac{y}{d}\right)+exp\left(\frac{2 ⁢ b + c}{2 ⁢ d}\right)\right)+c\right)$
g) Logistische Dosis-Response Funktion mit Transformationskoeffizienten *a, b, c* $f \left(y\right) = a / \left(1+{\left(\frac{y}{b}\right)}^{c}\right)$
h) Log-Normal-Kumulative Funktion mit Transformationskoeffizienten *a, b, c* : $f \left(y\right) = \frac{a}{2} ⁢ erfc ⁢ \left(-ln\left(\frac{y}{b}\right)/\sqrt{2}⁢c\right) .$

Ferner bevorzugt erfolgt das Ermitteln bzw. Berechnen eines individuellen Brillenglasdesigns und insbesondere das Berechnen der Lage und/oder der Größe eines Fern-, Nah- und Progressionsbereichs des Brillenglasdesigns unter Berücksichtigung ein oder mehrerer der erfaßten individuellen Daten des Brillenträgers. Die individuellen Daten des Brillenträgers, welche bei der Berechnung des individuellen Brillenglasdesigns berücksichtigt werden, umfassen insbesondere ein oder mehrere der vorstehend aufgeführten Daten bzw. Datensätze (z.B. Refraktionsdaten bzw. Wirkungsparameter; Pupillendistanz, den Hornhautscheitelabstand (HSA), Vorneigung (VN), Fassungsscheibenwinkel (FSW), Fassungsdaten, Zentrierdaten (wie z.B. Verkippung des Brillenglases vor dem Auge und korrigierte PD zum Einschleifen, d.h. CorPD), etc.).

Erfindungsgemäß wird ferner ein Computerprogrammerzeugnis sowie ein Speichermedium mit darauf gespeichertem Computerprogramm bereitgestellt, wobei das Computerprogramm ausgelegt ist, wenn geladen und ausgeführt auf einem Computer, das erfindungsgemäße Verfahren zum Bestimmen bzw. Berechnen eines individuellen Brillenglasdesigns für ein progressives Brillenglases durchzuführen.

Gemäß der Erfindung wird ferner eine Vorrichtung zum Bestimmen bzw. Berechnen eines individuellen Brillenglasdesigns für ein progressives Brillenglas zur Korrektur einer Fehlsichtigkeit eines bestimmten Brillenträgers vorgeschlagen, umfassend:
- Erfassungsmittel zum Erfassen von individuellen Daten des Brillenträgers;
- Berechnungs- bzw. Optimierungsmittel zum Berechnen der individuellen räumlichen Position eines Fern- und/oder eines Nahbezugspunkts in Abhängigkeit von den individuellen Daten des Brillenträgers nach dem erfindungsgemäßen Verfahren zum Berechnen der individuellen räumlichen Position eines Fern- und/oder eines Nahbezugspunkts;
- Berechnungs- bzw. Optimierungsmittel zum Berechnen der räumlichen Lage und/oder der Größe eines Fern-, Nah- und Progressionsbereichs des Brillenglasdesigns in Abhängigkeit von der berechneten individuellen räumlichen Position des Fern- und/oder des Nahbezugspunkts.

Ferner wird gemäß der Erfindung eine grafische Benutzerschnittstelle zum Festlegen bzw. Bestimmen und Darstellen eines individuellen Brillenglasdesigns für ein progressives Brillenglas vorgeschlagen, umfassend:
- zumindest einen Individualdaten-Eingabeabschnitt, welcher ausgelegt ist, individuelle Daten des Brillenträgers einzugeben; und
- zumindest einen Anzeigeabschnitt, welcher ausgelegt ist, das individuelle Brillenglasdesign darzustellen, wobei das individuelle Brillenglasdesign nach einem bevorzugten Verfahren zum Berechnen eines individuellen Brillenglasdesigns berechnet bzw. bestimmt wird.

Ebenfalls wird ein Verfahren bereitgestellt, gemäß welchem anhand von Daten bezüglich der Objektabstände Ferne und Nähe bei der Refraktionsbestimmung und Daten bezüglich individueller Objektabstände eine Anpassung der Bestellwerte bzw. der Gebrauchswerte vorgenommen wird. Dies kann z.B. mit folgender Berechnung erfolgen: $Ferne : {sphF}_{k} = {sphF}_{R} + {OF}_{R} - {OF}_{G}$ $N ⁢ {a}^{¨} ⁢ he : {sphN}_{k} = {sphN}_{R} + {ON}_{R} - {ON}_{G}$ ${Add}_{K} = {sphN}_{K} - {sphF}_{K} ,$
wobei:
- sphF_{R}, sphN_{R}:: Sphärische Werte in der Ferne bzw. Nähe der Refraktionsbestimmung;
- sphF_{K}, sphN_{K}:: Korrigierte sphärische Werte in der Ferne bzw. Nähe;
- Add_{K}: korrigierte Addition;
- OF_{R}, ON_{R}:: Kehrwerte der Objektentfernungen (Vorzeichenbehaftet) in der Ferne bzw. Nähe der Refraktionsbestimmung
- OF_{G}, ON_{G}:: Kehrwerte der Objektentfernungen (Vorzeichenbehaftet) in der Ferne bzw. Nähe in der tatsächlichen Gebrauchssituation
bezeichnen.

Die individuellen Objektabstände umfassen insbesondere Arbeitsabstand beim Lesen (Naharbeit), Arbeitsabstand in der Ferne; und/oder Daten bezüglich der Objektabstände bei der Refraktionsbestimmung: Ferne und Nähe.

Die Angaben zu den Objektentfernungen Ferne und Nähe werden bei der Berechnung und Optimierung berücksichtigt. Es kann somit der Strahlengang besser simuliert werden, welcher der tatsächlichen Gebrauchssituation entspricht und damit die Abbildungsqualität verbessert werden.

Ein Verfahren zum Herstellen eines individuellen progressiven Brillenglases mit einer variabel einstellbaren, vertikalen Position des Fern- und/oder Nahbezugspunkts umfaßt folgende Schritte:
- Erfassen von individuellen Daten des Brillenträgers;
- Ermitteln bzw. Berechnen der individuellen vertikalen und/oder horizontalen Position des Fern- und/oder des Nahbezugspunkts in Abhängigkeit von der erfaßten individuellen Daten des Brillenträgers;
- Berechnen eines individuellen Brillenglasdesigns, wobei das Berechnen eines individuellen Brillenglasdesigns Berechnen der räumlichen Lage und/oder Größe eines Fern-, Nah- und/oder Progressionsbereichs des Brillenglasdesigns in Abhängigkeit von der ermittelten individuellen räumlichen Position des Fern- und/oder des Nahbezugspunkts;
- Berechnen bzw. Optimieren des Brillenglases nach dem individuellen Brillenglasdesign.

Vorzugsweise erfolgt das Berechnen und Optimieren des Brillenglases unter Berücksichtigung zumindest eines Teils, vorzugsweise jedoch unter Berücksichtigung aller erfaßten individuellen Daten des Brillenträgers.

Wie oben dargelegt wurde, umfaßt das Berechnen eines individuellen Brillenglasdesigns, das Erzeugen bzw. Berechnen der Sollvorgaben für die räumliche Verteilung zumindest eines Abbildungsfehlers des Brillenglases (insbesondere Sollvorgaben für die räumliche Verteilung der astigmatischen Abweichung und/oder des Refraktionsfehlers), wobei im individuell ermittelten Fern- und/oder Nahbezugspunkt die dem Design zugeordneten Abbildungsfehler möglichst klein, vorzugsweise im wesentlichen Null, sind.

Das Berechnen bzw. Optimieren des Brillenglases nach dem individuellen Brillenglasdesign erfolgt vorzugsweise mittels Minimierung einer Zielfunktion, in welcher die dem Design zugeordneten individuell ermittelten Sollvorgaben für zumindest einen Abbildungsfehler eingehen.

Vorzugsweise erfolgt das Berechnen bzw. Optimieren des Brillenglases mittels Minimierung einer Zielfunktion der Form: $F \left(\vec{x}\right) = {\sum }_{i = 1}^{m} \left[{g}_{i , S}⁢{\left({S}_{i}-{S}_{i , Soll}\right)}^{2}+…\right] ,$
wobei:
- *S_{i,Soll}*: den Sollwert des zumindest einen Abbildungsfehlers an der *i* -ten Bewertungsstelle;
- *Sᵢ*: den tatsächlichen Wert des Abbildungsfehlers an der *i* -ten Bewertungsstelle;
- *g_{i,s}*: die lokale Gewichtung des Abbildungsfehlers an der *i* -ten Bewertungsstelle;
bezeichnen.

Vorzugsweise erfolgt die Optimierung des progressiven Brillenglases durch Minimierung einer Zielfunktion der Form: $F \left(\vec{x}\right) = {\sum }_{i = 1}^{m} \left[{g}_{i , Δ ⁢ R}⁢{\left({Δ ⁢ R}_{i}-{Δ ⁢ R}_{i , Soll}\right)}^{2}+{g}_{i , Ast}⁢{\left({Ast}_{i}-{Ast}_{i , Soll}\right)}^{2}+…\right] ,$
wobei:
- Δ*R_{i,Soll}*: den Sollwert des lokalen Refraktionsfehlers an der *i*-ten Bewertungsstelle;
- Δ*Rᵢ*: den tatsächlichen Wert des lokalen Refraktionsfehlers an der *i* -ten Bewertungsstelle;
- *Ast_{i,soll}*: den Sollwert der lokalen astigmatischen Abweichung bzw. des lokalen astigmatischen Fehlers an der *i*-ten Bewertungsstelle;
- *Astᵢ*: den tatsächlichen Wert der lokalen astigmatischen Abweichung in der *i*-ten Bewertungsstelle;
- *gᵢ*,_{Δ*R*}: die lokale Gewichtung des Refraktionsfehlers an der *i* -ten Bewertungsstelle;
- *g_{i,Ast}*: die lokale Gewichtung der astigmatischen Abweichung an der *i* -ten Bewertungsstelle
bezeichnen.

Vorzugsweise erfolgt die Berechnung bzw. Optimierung des Brillenglases ferner unter Berücksichtigung von individuellen Daten des Brillenträgers. Besonders bevorzugt erfolgt die Berechnung bzw. Optimierung des Brillenglases online.

Ferner umfaßt das Herstellungsverfahren:
- Bereitstellen von Flächendaten des berechneten bzw. optimierten Brillenglases; und
- Fertigen des Brillenglases gemäß den bereitgestellten Flächendaten des Brillenglases.

Die Fertigung bzw. Bearbeitung kann mittels numerisch gesteuerten CNC Maschinen, mittels eines Gießverfahrens, einer Kombination der beiden Verfahren oder nach einem anderen geeigneten Verfahren erfolgen.

Ferner wird gemäß der Erfindung eine Vorrichtung zum Herstellen eines individuellen progressiven Brillenglases mit einer variabel einstellbaren vertikalen Position des Fern- und/oder Nahbezugspunkts vorgeschlagen, umfassend:
- Designberechnungsmittel, welche ausgelegt sind ein bevorzugtes erfindungsgemäßes Verfahren zum Berechnen eines individuellen Brillenglasdesigns durchzuführen;
- Optimierungs- bzw. Berechnungsmittel, welche ausgelegt sind ein Berechnen bzw. Optimieren des Brillenglases nach dem individuellen Brillenglasdesign durchzuführen.

Insbesondere umfassen die Designberechnungsmittel:
- Erfassungsmittel zum Erfassen von individuellen Daten des Brillenträgers;
- Berechnungs- bzw. Optimierungsmittel zum Berechnen der individuellen räumlichen Position eines Fern- und/oder eines Nahbezugspunkts in Abhängigkeit von den individuellen Daten des Brillenträgers nach dem erfindungsgemäßen Verfahren zum Berechnen der individuellen räumlichen Position eines Fern- und/oder eines Nahbezugspunkts;
- Berechnungs- bzw. Optimierungsmittel zum Berechnen der räumlichen Lage und/oder der Größe eines Fern-, Nah- und/oder Progressionsbereichs des Brillenglasdesigns in Abhängigkeit von der berechneten individuellen räumlichen Position des Fern- und/oder des Nahbezugspunkts.

Ferner umfaßt die Vorrichtung zum Herstellen eines individuellen Brillenglases mit einer variabel einstellbaren vertikalen Position des Fern- und/oder Nahbezugspunkts vorzugsweise Bearbeitungsmittel zum Fertigbearbeiten des Brillenglases. Die Bearbeitungsmittel können z.B. CNC gesteuerte Maschinen zur Direktbearbeitung eines Blanks nach den individuellen Optimierungsvorgaben umfassen. Vorzugsweise weist das fertigbearbeitete Brillenglas eine einfache sphärische oder rotationssymmetrische asphärische Fläche und eine individuell nach den erfindungsgemäß berechneten individuellen Designvorgaben sowie individuellen Parameter des Brillenträgers optimierte asphärische/atorische progressive Freiformfläche auf. Vorzugsweise ist die sphärische oder rotationssymmetrische asphärische Fläche die Vorderfläche (d.h. die objektseitige Fläche) des Brillenglases. Selbstverständlich ist es jedoch möglich die individuell optimierte Fläche auf die Vorderfläche des Brillenglases anzuordnen. Ebenfalls ist es möglich, daß beide Flächen des Brillenglases individuell optimierte progressive Flächen sind.

Vorzugsweise umfaßt die Vorrichtung zum Herstellen eines individuellen progressiven Brillenglases ferner Erfassungsmittel zum Erfassen von individuellen Daten des Brillenträgers, welche insbesondere Daten bezüglich der individuell für den Brillenträger erforderlichen dioptrischen Wirkung des Brillenglases umfassen.

Die erfindungsgemäßen Verfahren und Vorrichtungen zum Bestimmen der individuellen Position der Fern- und/oder Nahbezugspunkte und zum Ermitteln bzw. Berechnen eines individuellen Brillenglasdesigns mit variabel einstellbaren Positionen der Fern- und/oder Nahbezugspunkte zeichnen sich insbesondere dadurch aus, daß neben einer automatischen Anpassung an den individuellen Wirkungsparameter eine individuelle Anpassung der Position bzw. Lage der Fern- und/oder Nahbezugspunkte und eine damit verbundene Anpassung der Lage und Größe der Sehbereichen möglich ist. Darüber hinaus ist es ebenfalls möglich, eine automatische Anpassung des Designs auf die individuelle Brillenglasfassung unter Berücksichtigung der Fassungsform sowie der individuellen Parameter (Pupillendistanz, Hornhautscheitelabstand, Vorneigung, Fassungsscheibenwinkel, etc.) durchzuführen. Damit wird immer eine optimale physiologische Blicksenkung sichergestellt. Ferner ist es möglich, den individuellen Nahabstand zu berücksichtigen sowie die Basiskurve frei auszuwählen, was insbesondere von Vorteil bei modernen, stärker durchgebogenen Fassungen ist. Ebenfalls kann eine individuelle Vordezentration berücksichtigt werden. Durch das optimale Abstimmen des Brillenglasdesign bzw. des Brillenglases auf die Bedürfnisse bzw. auf die individuellen Daten des Brillenträgers werden die Kompromisse eines Standard-Gleitsichtglases deutlich verringert.

Die erfindungsgemäßen Verfahren und Vorrichtungen zum Bestimmen der individuellen Position der Fern- und/oder Nahbezugspunkte und zum Ermitteln bzw. Berechnen eines individuellen Brillenglasdesigns mit variabel einstellbaren Positionen der Fern- und/oder Nahbezugspunkte ermöglichen somit die Erzeugung von individuellen Brillenglasdesigns bzw. die Herstellung von individuellen Brillengläsern mit möglichst großen, bedürfnisorientierten Sehbereichen. Dadurch ergeben sich für den Brillenträger erhebliche Vorteile wie eine Entlastung von Augen und Halswirbelsäule und eine Verringerung des Sehstress und der Augenermüdung sowie ein optimaler Seh- und Tragekomfort.

Bevorzugte Ausführungsformen der Erfindung werden nachfolgend unter Bezugnahme auf die Figuren beispielhaft beschrieben.

Es zeigen:
- Fig. 1: ein schematisches Flussdiagram eines Verfahrens zum Ermitteln der individuellen Position des Fern- und Nahbezugspunkts und zum Ermitteln eines individuellen Brillenglasdesigns;
- Fig. 2: Beispiel einer Maske bzw. Graphische Benutzerschnittstelle zur Eingabe von individuellen KundenparameternFig. 3 ein Beispiel einer graphischen Benutzerschnittstelle zur Ergebnisdarstellung;
- Fig. 4: ein Beispiel einer graphischen Benutzerschnittstelle zur Ergebnisdarstellung.

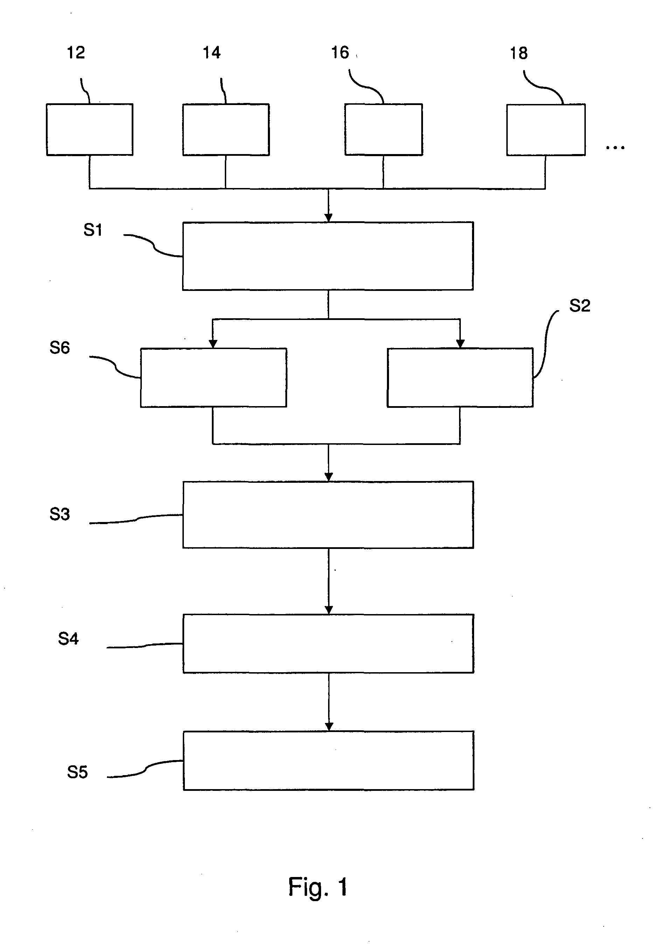

Fig. 1 zeigt ein schematisches Flussdiagram eines beispielhaften Verfahrens zum Ermitteln der individuellen Position des Fern- und Nahbezugspunkts und zum Ermitteln eines individuellen Brillenglasdesign mit der individuellen Position des Fern- und Nahbezugspunkts.

In einem ersten Schritt (Schritt **S1)** werden individuelle Daten des Brillenträgers erfaßt. Die Erfassung der individuellen Daten des Brillenträgers erfolgt mittels geeigneten grafischen Benutzerschnittstellen (GUI), welche die Eingabe und gegebenenfalls die Änderung der eingegebenen individuellen Daten des Brillenträgers ermöglichen.

Die für einen bestimmten Brillenträger in einer bestimmten Gebrauchssituation optimale räumliche Lage des Fern- und/oder Nahbezugspunkts wird anhand der erfaßten individuellen Daten berechnet (Schritt **S2).**

In einem weiteren Schritt (Schritt **S3)** wird ein Gleitsichtglasdesign bzw. einen Vorschlag für ein Gleitsichtsglasdesign mit den im zweiten Schritt **S2** ermittelten optimalen Positionen des Fern- und Nahbezugspunkts berechnet. Dieser Designvorschlag wird mittels geeigneter graphischen Benutzerschnittstellen visualisiert, wobei dem Benutzer die Möglichkeit gegeben wird, durch eine Veränderung der individuellen Position des Fern- und/oder des Nahbezugspunkts und/oder eine Veränderung der individuellen Kundendaten (insbesondere der Präferenzen bezüglich der Sehbereiche, der Fassungsdaten, etc.) das Design aktiv zu verändern.

Vorzugsweise wird das Brillenglasdesign mit den anhand der individuellen Kundendaten ermittelten, frei bestimmbaren, individuellen Positionen des Fern- und/oder Nahbezugspunkts aus einem bestehenden Brillenglasdesign mit einer feststehenden räumlichen Lage der Fern- und Nahbezugspunkte unter Beibehaltung der Designcharakteristik abgeleitet. Die vertikale Position und Länge der Progressionszone der Gleitsichtfläche wird dann automatisch für die individuelle Benutzersituation angepaßt.

Die erfaßten individuellen Daten des Brillenträgers umfassen vorzugsweise individuelle Refraktionsdaten **12** (Sphäre, Zylinder, Achslage, Addition, Prisma und Basis); individuelle Fassungs- und Zentrierdaten **14** (Scheibenhöhe und Zentrierhöhe, Fassungsform, Abstand zwischen den Gläsern AZG); Individuallisierungsparameter bzw. individuelle Parameter **16** der Augen des Brillenträgers und/oder der individuellen Gebrauchssituation bzw. Gebrauchsstellung der Brille vor den Augen des Brillenträgers (insbesondere Pupillendistanz PD, Hornhautscheitelabstand HSA, Vorneigung VN, Fassungsscheibenwinkel FSW, etc.); Daten **18** bezüglich der individuellen Präferenzen bzw. Gewichtungen der Sehbereiche sowie weitere individuelle Daten.

Die individuellen Parameter der Augen des Brillenträgers und/oder der individuellen Gebrauchssituation bzw. Gebrauchsstellung (Pupillendistanz, Hornhautscheitelabstand, Vorneigung, Fassungsscheibenwinkel, etc.) können beispielsweise automatisch mittels einer geeigneten 3-D Meßvorrichtung, wie z.B. 3-D Videozentriervorrichtung ImpressionIST der Firma Rodenstock GmbH ermittelt werden. Alternativ können die individuellen Parameter mittels herkömmlichen Meßtools ermittelt werden.

Die individuellen Parameter können beispielsweise in folgenden Grenzen variieren:
Pupillendistanz (PD) von 30 bis 80 mm
Hornhautscheitelabstand (HSA) von 3 bis 50 mm
Vorneigung (VN) von -10 bis +20 Grad
Fassungsscheibenwinkel (FSW) von -10 bis +35 Grad.

Die Fassungs- und Zentrierdaten können von dem Anwender (z.B. einem Augenoptiker oder einem Optometristen) angegeben werden, mittels eines Tracers automatisch erfaßt oder z.B. aus einer Datenbank abgerufen werden. Die Zentrierdaten können ebenfalls direkt von einem 3-D Videozentriersystem (z.B. 3-D Videozentriersystem ImpressionIST der Firma Rodenstock GmbH) übernommen werden.

Die erfaßten individuellen Daten des Brillenträgers werden bei der Berechnung bzw. Optimierung der räumlichen Lagen, insbesondere der vertikalen und/oder horizontalen Position des Fern- und/oder Nahbezugspunkts, wie nachfolgend im Detail erläutert wird, berücksichtigt. Darüber hinaus werden die erfaßten individuellen Daten des Brillenträgers bei der Berechnung und Optimierung des Brillenglases berücksichtigt.

In einem vierten Schritt (Schritt **S4)** wird ein individuelles Brillenglas optimiert bzw. berechnet, wobei die Optimierung unter Berücksichtigung zumindest eines Teils der ermittelten individuellen Daten, insbesondere Daten bezüglich der individuelle Parameter des Brillenträgers und der individuellen Gebrauchsstellung (Fassungsscheibenwinkel, Vorneigung, Pupillendistanz, Hornhautscheitelabstand, etc) erfolgt.

Die Fertigung des individuell berechneten Brillenglases erfolgt z.B. mittels Präzisionsmaschinen, vorzugsweise CNC-Schleif- und Poliermaschinen (Schritt **S5).**

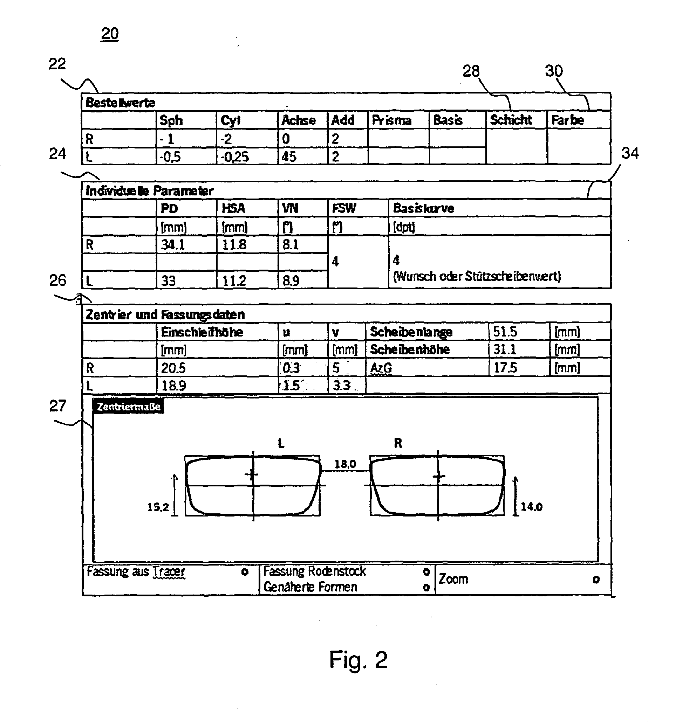

**Fig. 2** zeigt eine graphische Benutzerschnittstellen zur Eingabe von individuellen Kundenparametern.

In Fig. 2 ist ein Beispiel einer graphischen Benutzerschnittstelle **20** zur Eingabe von individuellen Daten (Bestellwerten) des Brillenträgers gezeigt. Die individuellen Daten können direkt in den entsprechenden Eingabefeldern bzw. -abschnitten der Maske bzw. der graphischen Benutzerschnittstelle eingegeben oder z.B. aus einer Datenbank abgerufen werden. Die grau hinterlegten Felder werden automatisch vom dem Programm berechnet bzw. mit Daten belegt.

Die in **Fig. 2** gezeigte graphische Benutzerschnittstelle **20** enthält:
- einen Abschnitt "Bestellwerte" (Abschnitt **22)** umfassend Eingabefelder zur Eingabe von individuellen Refraktionsdaten wie Sphäre "Sph", Zylinder "Cyl", Achse, Prisma, Basis);
- einen Abschnitt "Individuelle Parameter" (Abschnitt **24)** umfassend Eingabefelder zur Eingabe von individuellen Parametern der Augen des Brillenträgers und/oder der individuellen Gebrauchsstellung (Pupillendistanz "PD", Hornhautscheitelabstand "HSA", Vorneigung "VN", Fassungsscheibenwinkel "FSW");
- einen Abschnitt "Fassungs- und Zentrierdaten" (Abschnitt 26) umfassend Eingabefelder zur Eingabe von Fassungs- und Zentrierdaten (Einschleifhöhe, Scheibenlänge, Scheibenhöhe, Abstand zwischen den Gläsern "AzG" bzw. "AZG") sowie gegebenenfalls einen Anzeigeabschnitt (Abschnitt 27) zur Anzeige der Zentrierung der Brille angepasst an dem Kastenmaß.

Die Fassungsdaten können in entsprechenden Eingabefeldern eingegeben werden. Diese Werte können automatisch eingetragen werden, wenn die Fassung z.B. mittels eines Tracers oder aus einer Liste von Fassungen ausgewählt wird. Mit einer Funktion "Anpassen an Kastenmaß" kann die Fassung den gegebenenfalls geänderten Fassungsdaten anpasst werden.

Im Abschnitt "Fassungs- und Zentrierdaten" kann ferner eine Fassung aus einer Datenbank ausgewählt werden, z.B. falls vorher mittels eines Tracers keine Fassung aufgenommen bzw. vermessen worden ist. Insbesondere kann die Fassung aus einer Liste in einem sich öffnenden Pop-up-Fenster ausgewählt werden. Form- und Fassungsdaten werden vorzugsweise ebenfalls angezeigt. Die Auswahl kann durch Bestätigen übernommen werden. Ebenfalls kann eine genäherte Fassung aus einer Anzahl verschiedener Fassungsformen ("genäherte Formen") ausgewählt werden. Hierbei kann sich z.B. ein anderes Pop-up-Fenster öffnen, in dem die Fassung aus einer Auswahl gängiger Formen ausgewählt werden kann.

Die in **Fig.** 2 gezeigte graphische Benutzerschnittstelle 20 enthält ferner:
- Abschnitte bzw. Eingabefelder zur Eingabe von weiteren Informationen, wie z.B. Beschichtung (vgl. Abschnitt 28 in Fig. 2), Farbe (vgl. Abschnitt 30 in Fig. 2); sowie
- einen Abschnitt bzw. ein Eingabefeld "Basiskurve" (Abschnitt 34) auf, in welches die für die ausgewählte Fassung am besten passende Basiskurve eingetragen werden kann.

Wie oben dargelegt wurde, wird die für einen bestimmten Brillenträger in einer bestimmten Gebrauchssituation optimale räumliche Lage des Fern- und/oder Nahbezugspunkts anhand der erfaßten individuellen Daten berechnet (Schritt S2 in Fig. 1). Die Berechnung der optimalen räumlichen Lage der Bezugs- bzw. Designpunkte nach dem erfindungsgemäßen Verfahren bzw. mittels des erfindungsgemäßen Computerprogramms wird nachfolgend anhand von Beispielen im Detail erläutert.

Die vertikale Höhe des Fernbezugspunkts kann vorzugsweise in einem Bereich von +4 bis -4 mm gegenüber dem für dieses Brillenglas vom Hersteller festgelegten Zentrier- bzw. Anpaßpunkt in Abhängigkeit von den individuellen Daten des Brillenträgers flexibel bestimmt werden. Der Nahbezugspunkt kann vorzugsweise flexibel zwischen 13 und 20 mm vertikal unterhalb des Zentrier- bzw. Anpaßpunkts festgelegt werden. Daraus ergibt sich eine flexibel wählbare Progressionslänge, welche vorzugsweise minimal 13 mm und maximal 24 mm betragen kann. Vorzugsweise können der Fern- und Nahbezugspunkt in Stufen von 0,1 mm innerhalb eines zulässigen Bereichs frei festgelegt werden.

Vorzugsweise wird anhand der Fassungs- und Zentrierdaten eine Grenzberechnung für die Lage des Fern- und Nahbezugspunkte durchgeführt. Dadurch wird sichergestellt, daß der Fern- und Nahbezugspunkte innerhalb der Fassung liegen. Vorzugsweise beträgt der minimale vertikale Abstand vom unteren Fassungsrand zum Nahbezugspunkt 2 mm. Der Fernbezugspunkt weist vorzugsweise minimal einen vertikalen Abstand von 6 mm, vorzugsweise 8 mm von dem oberen Fassungsrand auf. Mit den minimal zulässigen Abständen des Fern- und Nahbezugspunkts von dem oberen bzw. dem unteren Fassungsrand läßt sich die maximal zulässige Progressionslänge berechnen. Die Progressionslänge wird hierbei als der vertikale Abstand zwischen dem Fern- und dem Nahbezugspunkt definiert.

Ferner wird anhand der Fassungs- und Zentrierdaten eine ideale Lage der Position des Fern- und des Nahbezugspunkts in Bezug auf die Fassungs- und Zentrierdaten berechnet. Vorzugsweise beträgt der ideale vertikale Abstand vom unteren Fassungsrand zum Nahbezugspunkt mindestens 5 mm. Der ideale vertikale Abstand des Fernbezugspunkts vom oberen Fassungsrand beträgt vorzugsweise mindestens 12 mm. Falls sich aufgrund unterschiedlicher Zentrierhöhen in der Fassung rechts/links abweichende Bezugspunktlagen ergeben, werden diese vorzugsweise abgeglichen. So wird sichergestellt, daß sich die Bezugspunkte immer innerhalb der Fassung und auf der gleichen Höhe befinden.

Aus den idealen Abständen des Fern- und Nahbezugspunkts von dem jeweiligen Fassungsrand kann die minimale Progressionslänge berechnet werden. Der Benutzer (z.B. Optiker/Optometrist) kann diese Werte direkt bei der Bestellung angeben oder mit dem erfindungsgemäßen Computerprogramm berechnen.

Die individuellen Parameter (Pupillendistanz PD, Hornhautscheitelabstand HSA, Vorneigung VN, Fassungsscheibenwinkel FSW, etc.), welche beispielsweise automatisch mittels einer geeigneten 3-D Meßvorrichtung, wie z.B. mittels der 3-D Videozentriervorrichtung ImpressionIST der Firma Rodenstock GmbH oder alternativ mittels herkömmlichen Meßtools ermittelt werden, werden bei der Berechnung bzw. Optimierung der räumlichen Lagen, insbesondere der vertikalen und/oder horizontalen Position des Fern- und/oder Nahbezugspunkts, wie nachfolgend im Detail erläutert wird, berücksichtigt.

Die individuellen Parameter können beispielsweise in folgenden Grenzen variieren:
Pupillendistanz (PD) von 30 bis 80 mm
Hornhautscheitelabstand (HSA) von 3 bis 50 mm
Vorneigung (VN) von -10 bis +20 Grad
Fassungsscheibenwinkel (FSW) von -10 bis +35 Grad.

Die räumliche Lage, insbesondere der vertikale Abstand des Fern- und Nahbezugspunkts von dem Zentrierpunkt, hängt insbesondere von der Vorneigung und dem Hornhautscheitelabstand ab. Der Standardwert bzw. der Wert, welcher einem Standard- bzw. einem Grunddesign zugrunde liegt, beträgt beispielsweise für Vorneigung 9 Grad und für den Hornhautscheitelabstand 13 mm. Weichen die tatsächlichen Werte (HSA und Vorneigung) von diesen ab, wird erfindungsgemäß die vertikale Position der Bezugspunkte entsprechend angepaßt, um z.B. extrem hohe Blickauslenkungen beim Nahsehen bei kleinen HSA-Werten zu vermeiden.

Dies kann z.B. mit folgender Formel erfolgen: ${y}_{Bneu} = {y}_{Balt} * \left(HSA+13,5 mm\right) / 26 , 5 mm * Cosinus \left(Vorneigung\right) / Cosinus \left(9 Grad\right) ,$
wobei:
- y_{Bneu}: die neue vertikale Position des Fern- bzw. des Nahbezugspunkts
- y_{Balt}: die vertikale Position des Fern- bzw. des Nahbezugspunkts gemäß dem Standarddesign;
bezeichnen.

Liegen die so berechneten Werte außerhalb des zulässigen Intervalls, welches aus den Fassungs- und Zentrierdaten berechnet wird, so wird die Position der Bezugspunkte so lange verändert bis die Bezugspunkte innerhalb des zulässigen Intervalls liegen. Vorzugsweise werden die vertikalen Höhen des Fern- und/oder des Nahbezugspunkts links/rechts (d.h. für das linke und das rechte Auge) - wie oben beschrieben - aneinander angeglichen.

Die individuellen Daten des Brillenträgers umfassen ferner gegebenenfalls Daten bezüglich des Vorgängerglases, insbesondere
- Daten bezüglich des Typs des Brillenglases (Einstärken, Bifokal, Progressiv), und/oder der Progressionslänge;
- Daten bezüglich der dioptrischen Wirkung, insbesondere der Addition des Vorgängerglases.

Ferner können ebenfalls Daten bezüglich des Materials (Kunststoff/Silikat) und/oder des Brechungsindexes des Vorgängerglases erfaßt und ausgewertet werden. Falls bereits ein Gleitsichtglas getragen worden ist, können ferner die Daten bezüglich des Vorgängerglases, Daten bezüglich des Designs des Brillenglases (Hart/Weich) und/oder des Typs des Gleitsichtglases (Individuell, Konventionell) umfassen.

Die Daten bezüglich des Typs des Brillenglases (Einstärken, Bifokal, Progressiv) und der Progressionslänge des Vorgängerglases können bei der Optimierung der räumlichen Position des Fern- und Nahbezugspunkte, der optischen Wirkung, etc. berücksichtigt werden.

Ferner können die Daten bezüglich des Vorgängerglases Daten bezüglich Verbesserungswünschen gegenüber der bisherigen Brille, beispielsweise größerer Fernbereich, größerer Zwischenbereich, größerer Nahbereich, geringere Blicksenkung beim Lesen oder geringere Schaukelbewegung, umfassen.

Aus der Lage der Bezugspunkte des bisherigen Gleitsichtglases und evtl. vorhandenen Verbesserungswünschen kann beispielsweise eine ideale, individuelle Lage der Bezugspunkte in bezug auf die Historie berechnen werden. War bisher z.B. der Fernbereich zu klein und/oder wird eine bessere Fernsicht gewünscht, so wird der Fernbezugspunkt gegenüber der bisherigen Lage nach unten verschoben. War der Nahbereich zu klein und/oder die Blicksenkung beim Lesen zu groß, wird der Nahbezugspunkt nach oben verschoben. War der Zwischenbereich für das Arbeiten z.B. am Computer zu klein und/oder die Schaukelbewegung zu groß, wird der Fernbezugspunkt nach oben und der Nahbezugspunkt nach unten verschoben.

Die individuellen Daten des Brillenträgers können ferner Daten bezüglich der Präferenzen hinsichtlich der Sehbereiche (Fern-, Nah- und Zwischen- bzw. Progressionsbereich) umfassen. Die Abfrage dieser Daten kann beispielsweise mittels geeigneter graphischer Benutzerschnittstellen erfolgen. Diese Daten gehen vorzugsweise ebenfalls in die Berechnung der optimalen Lage des Fern- und/oder Nahbezugspunkts ein.

Insbesondere kann eine ideale Lage der Bezugspunkte im Bezug auf die Präferenzen berechnet werden. Wird das Brillenglas beispielsweise hauptsächlich beim Autofahren genutzt, wird der Fernbezugspunkt gegenüber dem physiologisch idealen Wert nach unten verschoben. Wird das Brillenglas beispielsweise hauptsächlich zum Lesen genutzt, wird der Nahbezugspunkt gegenüber dem physiologisch idealen Wert nach oben verschoben. Wird das Brillenglas beispielsweise hauptsächlich zur Computerarbeit genutzt, wird der Fernbezugspunkt nach oben und der Nahbezugspunkt nach unten gegenüber dem physiologisch idealen Werten verschoben.

In gleicher Weise kann aus dem bisher getragenen Brillenglas und den Verbesserungswünschen eine ideale Lage der Bezugspunkte (Fern- und Nahbezugspunkt) im Bezug auf die Verbesserungswünsche bestimmt werden.

War bisher beispielsweise der Fernbereich zu klein und/oder wird eine bessere Fernsicht gewünscht, so wird der Fernbezugspunkt gegenüber der bisherigen Lage nach unten verschoben. War der Nahbereich zu klein und/oder die Blicksenkung beim Lesen zu groß, wird der Nahbezugspunkt nach oben verschoben. War der Zwischenbereich für das Arbeiten z.B. am Computer zu klein und/oder die Schaukelbewegung zu groß, wird der Fernbezugspunkt nach oben und der Nahbezugspunkt nach unten verschoben.

Der Augenoptiker/Optometrist kann eine ideale, individuelle Position des Fern- und/oder Nahbezugspunkts anhand der erfaßten Präferenzen hinsichtlich der Sehbereiche und gegebenenfalls der Verbesserungswünsche selbst für die Glasbestellung festlegen. Vorzugsweise wird jedoch die ideale Position aus den erfaßten Präferenzen und gegebenenfalls Verbesserungswünschen automatisch mit Hilfe eines Computers berechnet. Dies kann beispielsweise folgendermaßen erfolgen:
Anhand der erfaßten individuellen Präferenzen können den einzelnen Sehbereichen entsprechende Gewichtungen (PräferenzFerne, PräferenzNähe, PräferenzZwischenbereich) zugeordnet werden. Die einzelnen Gewichtungen können beispielsweise Werte von 0 bis 100 annehmen, wobei die Summe der einzelnen Gewichtungen zwischen 0 und 100 liegt.

Die ideale vertikale Lage des Fern- und Nahbezugspunkts bezüglich der Sehbereichspräferenzen kann beispielsweise folgendermaßen berechnet werden:
Wenn die Gewichtung des Fernbereichs (PräferenzFerne) kleiner als 33 ist, dann gilt: ${Y}_{BF} = 4 - 4 * Pr ⁢ {a}^{¨} ⁢ ferenzFerne / 33 , 33$
sonst ${Y}_{BF} = 0 - 4 * \left(Pr⁢{a}^{¨}⁢ferenzFerne-33,33\right) / 66 , 66.$
Wenn die Gewichtung des Nahbereichs (PräferenzNähe) kleiner 33 ist, dann gilt: ${Y}_{BN} = - 20 + 2 * Pr ⁢ {a}^{¨} ⁢ ferenzN ⁢ {a}^{¨} ⁢ he / 33 , 33$
sonst ${Y}_{BN} = - 18 + 5 * \left(Pr⁢{a}^{¨}⁢ferenzN⁢{a}^{¨}⁢he-33,33\right) / 66.66.$
Wenn die Gewichtung des Zwischenbereichs (PräferenzZwischenbereich) kleiner als 33 ist, dann gilt $Progressionsl ⁢ {a}^{¨} ⁢ nge = 13 + 5 * Pr ⁢ {a}^{¨} ⁢ ferenzZwischenbereich / 33 , 33$
sonst $Progressionsl ⁢ {a}^{¨} ⁢ nge = 18 + 2 * \left(Pr⁢{a}^{¨}⁢ferenzZwischenbereich-33,33\right) / 66 , 66.$

Da die Bezugspunkte und die Progressionslänge nicht unabhängig von einander sind, sondern die Progressionslänge die Differenz der vertikalen Lage der Bezugspunkte darstellt, können vorzugsweise auch aus der Progressionslänge die vertikalen Positionen der Bezugspunkte berechnet werden: ${Y}_{BF ⁢ 2} = {Y}_{BN} + Progressionsl ⁢ {a}^{¨} ⁢ nge$ ${Y}_{Bn ⁢ 2} = {Y}_{BF} - Progressionsl ⁢ {a}^{¨} ⁢ nge$

Die endgültigen Bezugspunkte ergeben sich dann zu: ${Y}_{BF} = \left(GW⁢1*{Y}_{BF}+{Y}_{BF ⁢ 2}\right) / \left({g}_{1}\right)$ ${Y}_{BN} = \left(GW⁢2*{Y}_{BN}+{Y}_{BF ⁢ 2}\right) / \left({g}_{2}\right)$ ${g}_{1} = GW ⁢ 1 + 1$ ${g}_{2} = GW ⁢ 2 + 1$

Die Gewichtungen GW1 und GW2 nehmen vorzugsweise Werte zwischen 1 und 2 an.

In gleicher Weise kann aus den Daten bezüglich des bisher getragenen Brillenglases und Daten bezüglich der Verbesserungswünsche durch geeignete Vergabe von Gewichtungen eine ideale Lage der Bezugspunkte (Fern- und Nahbezugspunkt) im Bezug auf die Verbesserungswünsche bestimmt werden.

Ferner können die individuellen Daten des Brillenträgers Daten bezüglich der Hauptnutzung (Autofahren, Computerarbeitsplatz, Lesen, Handwerken, etc.) des Gleitsichtglases umfassen.

Aus den Daten bezüglich der Hauptnutzung läßt sich in ähnlicher Weise eine ideale Lage der Bezugspunkte berechnen. Wird das Brillenglas beispielsweise hauptsächlich beim Autofahren genutzt, wird der Fernbezugspunkt gegenüber dem physiologisch idealen Wert nach unten verschoben. Wird das Brillenglas beispielsweise hauptsächlich zum Lesen genutzt, wird der Nahbezugspunkt gegenüber dem physiologisch idealen Wert nach oben verschoben. Wird das Brillenglas beispielsweise hauptsächlich zur Computerarbeit genutzt, wird der Fernbezugspunkt nach oben und der Nahbezugspunkt nach unten gegenüber den physiologisch idealen Werten verschoben.

Ferner können die individuellen Daten des Brillenträgers Daten bezüglich der:
- Umwelteinflüsse (Umgebungshelligkeit etc.); und/oder
- individuellen Hauptblickrichtung Ferne und Nähe; und/oder
- eventuell vorhandene außergewöhnliche Kopf- und Körperhaltung;
- Physiologische Parameter der Augen des Brillenträgers, insbesondere Sehschärfe mit Korrektion, Stereogrenzwinkel.

Entsprechend können geeignete graphische Benutzerschnittstellen zur Eingabe und gegebenenfalls Korrektion dieser Daten vorgesehen werden.

Zusätzlich können Daten bezüglich der Präferenz Ästhetik/Optik erfaßt werden und anhand dieser Daten das Material des Brillenglases bestimmt werden. Bei einer höheren Gewichtung der Präferenz Ästhetik wird ein hochbrechendes Material gewählt, welches sehr dünn und leicht ist. Bei einer höheren Gewichtung der Optik wird ein Material mit hoher Abbe-Zahl gewählt, welches sehr gute optische Eigenschaften aufweist. Diese Wahl kann auch wirkungsabhängig sein.

Die individuellen Daten des Brillenträgers werden erfaßt und ausgewertet und steuern die Lage der Bezugspunkte "Ferne" und "Nähe".

So kann beispielsweise eine ideale Lage der Bezugspunkte jeweils im Bezug auf die Fassungs- und Zentrierdaten, der Physiologie und Ergonomie, der Historie, den Präferenzen und gegebenenfalls anderen relevanten individuellen Daten des Brillenträgers erhalten bzw. berechnet werden. In anderen Worten kann eine ideale Lage der Bezugspunkte (Fern- und Nahbezugspunkte) jeweils im Bezug auf unterschiedliche Kategorien (Fassungs- und Zentrierdaten, Physiologie und Ergonomie, Historie, Präferenzen, etc.) ermittelt werden.

Aus diesen Daten kann dann ein optimaler Kompromiß, z.B. durch Mittelwertbildung, bestimmt werden. Die unterschiedlichen Kategorien können unterschiedliche gewichtet werden. Die Berechnung der optimalen Lage des Fern- und/oder Nahbezugspunkts *y_{DF,DN}* kann z.B. anhand der folgenden Formel berechnet werden: ${y}_{DF , DN} = {\sum }_{i = 1}^{N} {g}_{i} ⁢ {y}_{DF , DN}^{i}$ ${\sum }_{i = 1}^{N} {g}_{i} = 1$
wobei:
- *gᵢ*: die Gewichtung der *i* -ten Kategorie;
- *yⁱ_{DF,DN}*: die ideale Position des Fernbezugspunkts (Designpunkt Ferne) DF bzw. des Nahbezugspunkts (Designpunkt Nähe) DN für die *i*-te Kategorie; und
- N: die Anzahl der unterschiedlichen Kategorien
bezeichnen.

Ferner können die individuellen Daten des Brillenträgers Daten bezüglich der optischen Größen umfassen. Anhand der erfaßten optischen Größen kann vorzugsweise eine Umrechnung der Addition, des Nahabstandes und des Zusatzparameters erfolgen (Schritt S6 in Fig. 1). Die Daten bezüglich der optischen Größen umfassen insbesondere Daten bezüglich der individuellen Objektabstände, wie Arbeitsabstand beim Lesen (Naharbeit), Arbeitsabstand in der Ferne und/oder Daten bezüglich der Objektabstände Ferne und Nähe bei der Refraktionsbestimmung.

Die Angaben zu den Objektentfernungen "Ferne" und "Nähe" bei der Refraktionsbestimmung werden bei der Berechnung und Optimierung berücksichtigt. Somit kann der Strahlengang, welcher der tatsächlichen Gebrauchssituation entspricht, genauer simuliert werden und folglich die Abbildungsqualität verbessert werden.

Sind die Objektabstände "Ferne" und "Nähe" der Refraktionsbestimmung bekannt, kann vorzugsweise eine Anpassung der Bestellwerte bzw. der Gebrauchswerte vorgenommen werden. Dies kann z.B. mit folgender Berechnung erfolgen: $Ferne : {sphF}_{k} = {sphF}_{R} + {OF}_{R} - {OF}_{G}$ $N ⁢ {a}^{¨} ⁢ he : {sphN}_{k} = {sphN}_{R} + {ON}_{R} - {ON}_{G}$ ${Add}_{K} = {sphN}_{K} - {sphF}_{K} ,$
wobei:
- sphF_{R}, sphN_{R}: Sphärische Werte in der Ferne bzw. Nähe der Refraktionsbestimmung;
- sphF_{K}, sphN_{K},: Korrigierte sphärische Werte in der Ferne bzw. Nähe;
- Add_{K}: korrigierte Addition;
- OF_{R}, ON_{R}: Kehrwerte der Objektentfernungen (vorzeichenbehaftet) in der Ferne bzw. Nähe der Refraktionsbestimmung (in dpt)
- OF_{G}, ON_{G}: Kehrwerte Objektentfernungen (vorzeichenbehaftet) in der Ferne bzw. Nähe in der tatsächlichen Gebrauchssituation (in dpt)
bezeichnen.

Insbesondere ist es so möglich, die Hauptsehentfernung beim Blick in die Nähe in der tatsächlichen Gebrauchsstellung des Brillenglases bei der Glasberechnung zu berücksichtigen.

Wenn die optimale individuelle Position des Fern- und/oder Nahbezugspunkts anhand von individuellen Daten des Brillenträgers ermittelt wird, wird ein entsprechendes Brillenglasdesign mit den so ermittelten Positionen des Fern- und Nahbezugspunkts und gegebenenfalls unter Berücksichtigung der individuellen Parameter des Brillenträgers automatisch berechnet (Schritt S3 in Fig. 1).

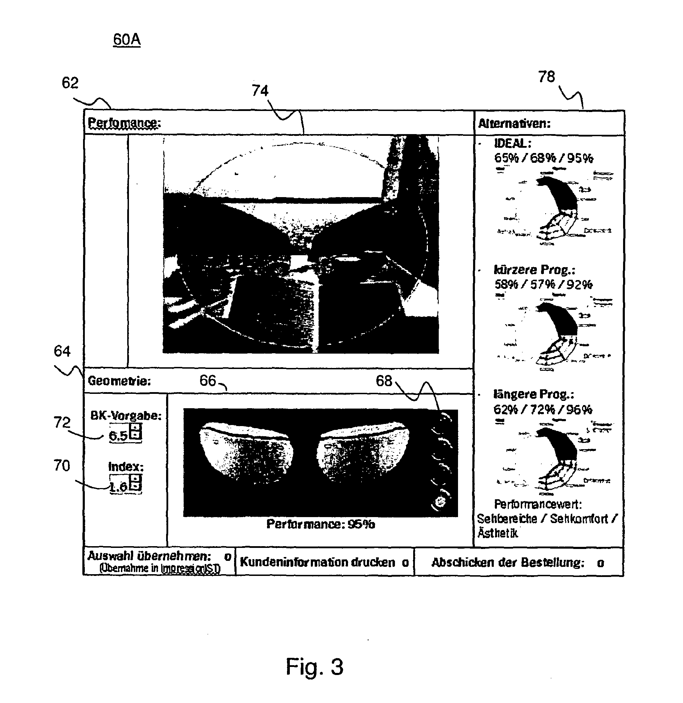

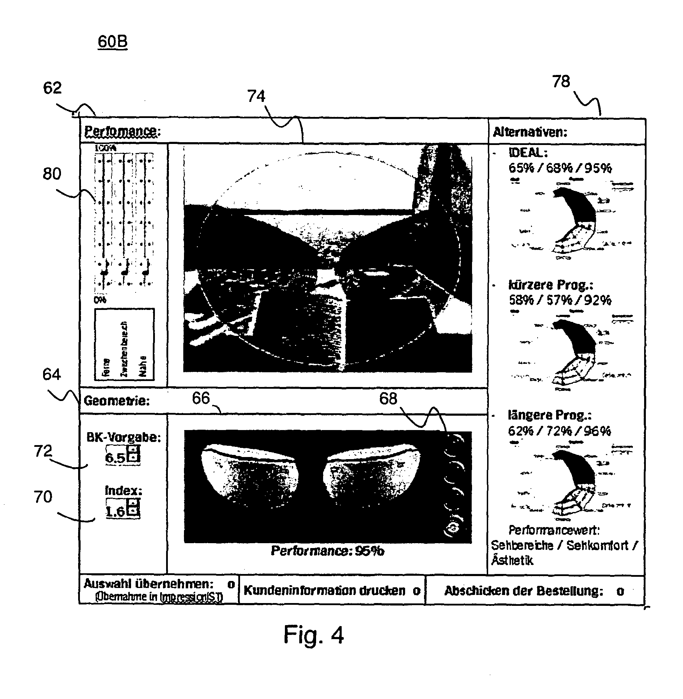

Der Designvorschlag kann mittels einer geeigneten graphischen Benutzerschnittstellen **60A** (vgl. **Fig. 3****)** zur Ergebnisdarstellung visualisiert werden. Je nach Auswahl in den Einstellungen, kann zusätzlich eine graphische Benutzerschnittstelle **60B** (Designtuner) gezeigt werden (vgl. **Fig.** 4) mit deren Hilfe, neben einer Ergebnisdarstellung, dem Benutzer die Möglichkeit gegeben wird, durch eine Veränderung der individuellen Position des Fern- und/oder des Nahbezugspunkts und/oder eine Veränderung der individuellen Daten des Brillenträgers (insbesondere der Präferenzen, der Fassungsdaten, etc.) das Design aktiv zu verändern. Zusätzlich können die entsprechenden geometrischen Daten des Brillenglases (Mittendicke, Randdicke, Basiskurve, Gewicht) berechnet und ebenfalls mittels einer geeigneten graphischen Benutzerschnittstelle (vorzugsweise in Form eines dreidimensionalen Models) visualisiert werden. Die graphischen Benutzerschnittstellen **60A und 60B** sind in zwei Bereiche aufgeteilt: im oberen Bereich **62** werden Informationen zum Thema "Sehen" und/oder "Performance" mit dem vorgeschlagenen Design für ein individuelles Brillenglas; im unteren Bereich **64** Informationen zum Thema "Aussehen" bzw. "Geometrie" des individuellen Brillenglases bzw. Brille dargestellt.

Im Bereich "Aussehen" **64** können insbesondere kosmetische Eigenschaften bzw. Daten bezüglich der Ästhetik des Brillenglases (z.B. Gewicht, geometrische Daten wie Bauhöhe, maximale Randdicke, Mittendicke, Basiskurve, etc.) des gerandeten Brillenglases bzw. der gerandeten Brillengläser visualisiert bzw. graphisch dargestellt werden. Die Visualisierung der kosmetischen Eigenschaften des Brillenglases kann beispielsweise mittels einer dreidimensionalen graphischen Darstellung eines Models des Brillenglases **66** mit den ermittelten geometrischen Daten erfolgen, wie z.B. in **Fig. 3** **und** **Fig. 4** gezeigt. Die Darstellung der kosmetischen Eigenschaften des Brillenglases kann z.B. durch eine Auswahl der Basiskurve und des Brechungsindexes beeinflußt werden. Die Auswahl kann wirkungsabhängig sein. Der Bereich "Aussehen" **64** umfaßt ferner einen Abschnitt **70** zur Anzeige von numerischen Werten bezüglich des Brechungsindexes und einen Abschnitt zur Anzeige der Basiskurve (Abschnitt **72).** Die angezeigten Werte für die Basiskurve und den Brechungsindex setzen sich aus dem Wirkungsbereich, dem notwendigen Durchmesser, den Basiskurvenwunsch und den Refraktionsdaten zusammen. Daher sind Abweichungen zu dem der Maske "Bestellwerte" eingegebenen Basiskurvenwunsch möglich. Über entsprechende Auswahlfelder können die technisch realisierbaren Werte für Basiskurve und Brechungsindex des Glases verändert werden. Falls Änderungen der Basiskurven-Vorgabe, der Brechungsindex, etc. vorgenommen werden, können z.B. durch Betätigen eines Buttons "Aktualisieren" die Grafik und die Geometriedaten entsprechend den geänderten Werten neu berechnet werden. Mittels geeigneter Buttons **68** können Ansichten der gerandeten Brillengläser aus verschiedenen statischen Perspektiven gezeigt werden (Fassung von oben, Fassung von vorn, von der Seite, von schräg oben). Zur besseren Detailansicht kann das Bild z.B. über einen entsprechenden Button vergrößert darstellen werden.

Neben der Visualisierung der kosmetischen Eigenschaften des Brillenglases erfolgt ferner eine Visualisierung der optischen Eigenschaften des Brillenglases (Sehbereiche, insbesondere räumliche Lage und Größe der einzelnen Sehbereiche). Die Darstellung der Sehbereichsgrößen kann lediglich relativ zu den Verordnungsdaten erfolgen, ohne daß eine eventuelle Materialabhängigkeit berücksichtigt wird. Selbstverständlich kann jedoch eine Berücksichtigung einer Materialabhängigkeit vorgesehen werden. Fig. **3** **und** **4** zeigen Beispiele der Darstellung der optischen Eigenschaften des Brillenglases.

Ferner kann eine Visualisierung des Sehkomforts (z.B. Blicksenkung, Schaukeln, peripheres Sehen, Verzerrungen, etc.) vorgesehen werden.

Zusätzlich kann eine geeignete Darstellung von Performancewerten bezüglich der Sehbereiche, des Sehkomforts und/oder der kosmetischen Eigenschaften bzw. der Ästhetik des individuellen Brillenglases vorgesehen werden. Ebenfalls können Performancewerte alternativer Designvorschläge dargestellt werden.

Der Bereich "Sehen" **62** der graphischen Benutzerschnittstellen 60A bis 60D ist daher grundsätzlich in mehrere Unter- bzw. Subbereiche gegliedert. In dem Subbereich **74** "Binokulare Sehbereichsdarstellung" des Bereichs **62** wird z.B. das für den Kunden und die angegebene Fassung ideale Design anhand einer Ellipse schematisch gezeigt. Graue Bereiche sind dabei mit Abbildungsfehlern (z.B. Astigmatismus in Gebrauchsstellung größer als 0,5 dpt) behaftete Bereiche. Im Subbereich **78** "Designprofil" des Bereichs **62** wird ein qualitativer Vergleich der Sehbereichsgrößen zueinander, z.B. in Form von Diagrammen **(vgl.** **Fig. 3** **und** **4****)** dargestellt.

Zusätzlich kann dem Augenoptiker/Optometristen und/oder dem Brillenträger die Möglichkeit gegeben werden das so berechnete individuelle Brillenglasdesign aktiv zu verändern. Die Veränderung erfolgt beispielsweise in dem die räumliche Position, insbesondere die vertikale Höhe des Fern- und/oder Nahbezugspunkts, aktiv verändert wird. Alternativ können die Gewichtungen der Sehbereiche verändert werden.

Die Änderung bzw. die Anpassung der Position des Fern- und/oder Nahbezugspunkts und/oder der Präferenzen bezüglich der Sehbereiche kann beispielsweise mittels einer geeigneten graphischen Benutzerschnittstelle erfolgen. Ein Beispiel einer geeigneten graphischen Benutzerschnittstelle in Form von Schieberegler **80,** welche eine Anpassung der Präferenzen bezüglich der Sehbereiche ermöglicht, ist in Fig. 4 gezeigt.

Das neue Brillenglasdesign mit der geänderten Position des Fern- und/oder Nahbezugspunkts wird vorzugsweise in Echtzeit berechnet und visualisiert. Vorzugsweise wird ebenfalls die Differenz bzw. die Veränderung der optischen Eigenschaften des neuen gegenüber dem alten Brillenglasdesign visualisiert.

Weitere Ausführungsbeispiele, Vorteile und Eigenschaften der Erfindung ergeben sich aus den beigefügten exemplarischen und nicht beschränkenden Beschreibungsseiten.

Nachfolgend werden die individuell festlegbaren Fern- und Nahbezugspunkte jeweils als Designpunkt "Ferne" bzw. Designpunkt "Nähe" bezeichnet. Insbesondere entspricht der individuell ermittelte Fernbezugspunkt bzw. Designpunkt "Ferne" dem Punkt, durch den der Brillenglasträger beim Blick in die Ferne optimal korrigiert ist und welcher seinen persönlichen Sehgewohnheiten entspricht. Der individuell ermittelte Nahbezugpunkt bzw. Designpunkt "Nähe" entspricht dem Punkt, durch den der Brillenglasträger bei Nahsehaufgaben optimal korrigiert ist und die für ihn angenehme Blicksenkung einnehmen kann.

Es zeigen:

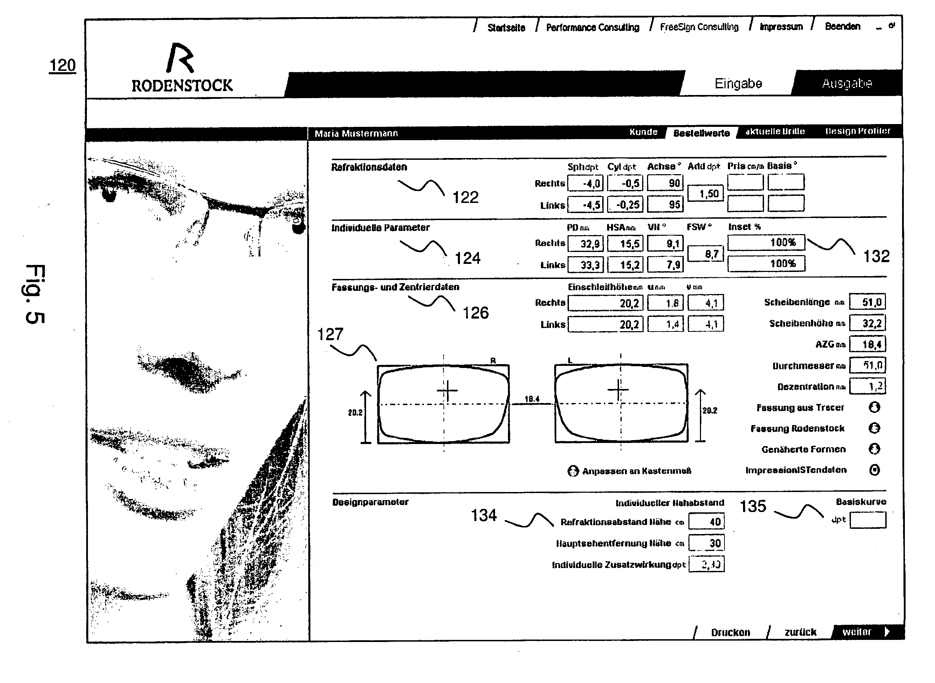

- Fig. 5: Beispiel einer Maske bzw. graphischen Benutzerschnittstelle zur Eingabe von individuellen Kundenparametern;

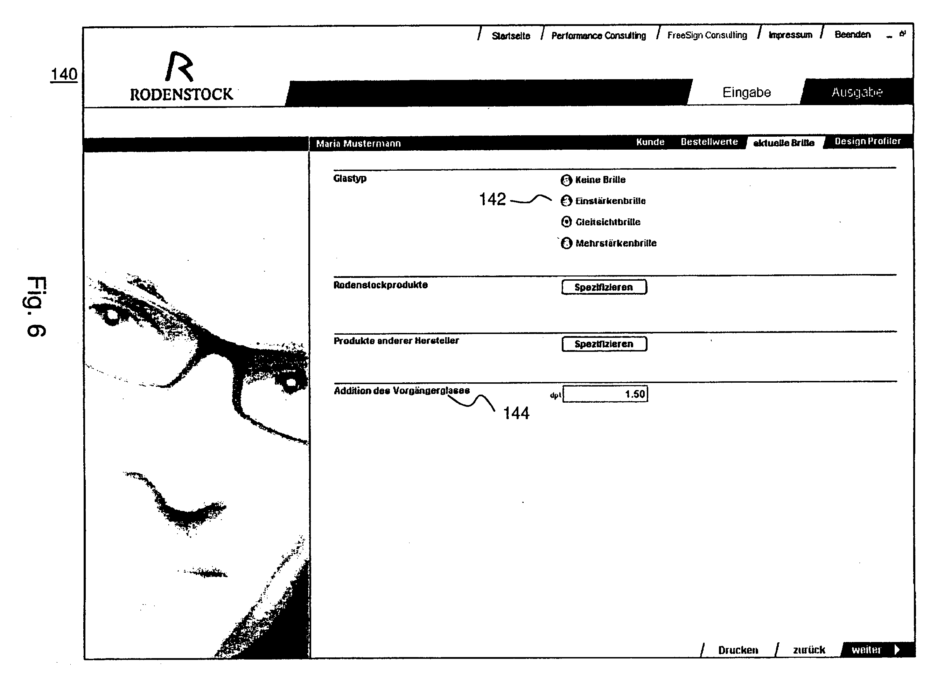

- Fig. 6: Beispiel einer Maske bzw. graphischen Benutzerschnittstelle zur Eingabe von Daten bezüglich der aktuellen Brille;

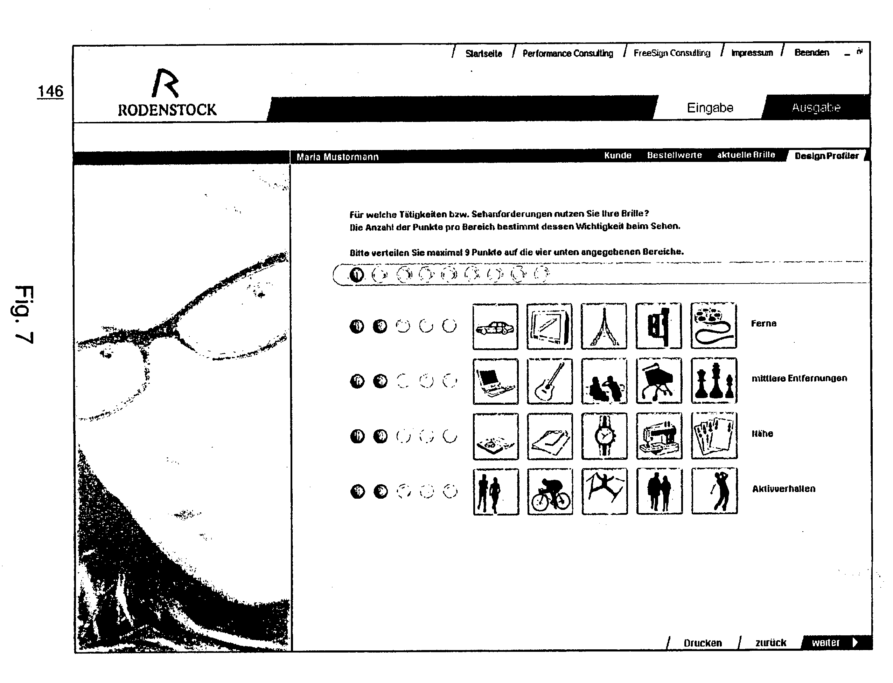

- Fig. 7: Beispiel einer Maske bzw. graphischen Benutzerschnittstelle zur Eingabe von Daten bezüglich der individuellen Präferenzen bzw. Gewichtungen der Sehbereiche;

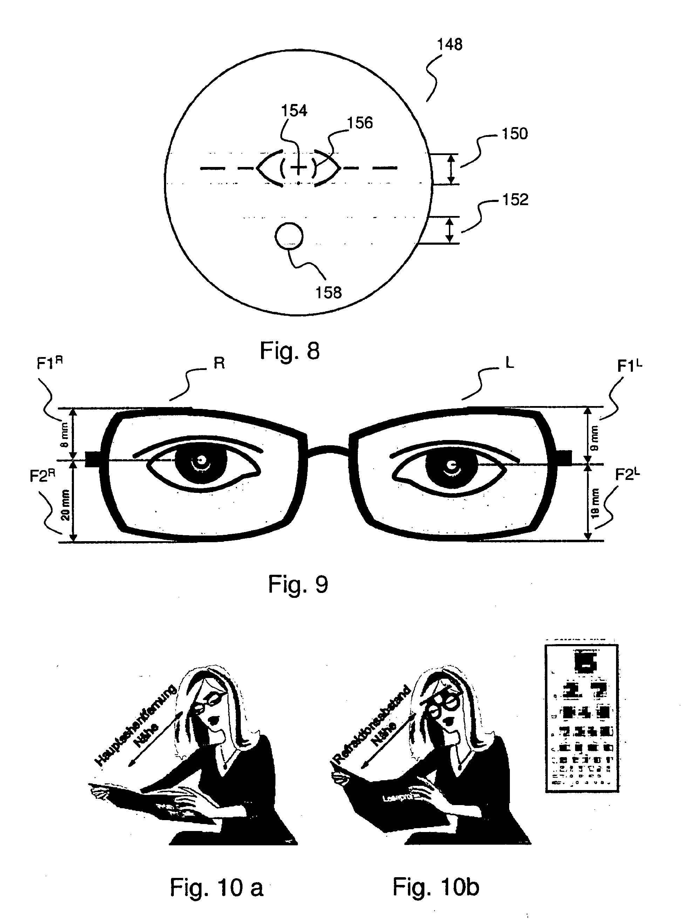

- Fig. 8: Eine schematische Darstellung der Lage der Fern- und Nahbezugspunkte eines individuellen Brillenglasdesigns;
- Fig. 9: Ein Beispiel unterschiedlicher Durchblickshöhen der beiden Augen;
- Fig. 10: Eine schematische Darstellung der Hauptsehentfernung Nähe (Fig. 10a) und Refraktionsabstand Nähe (Fig. 10b);

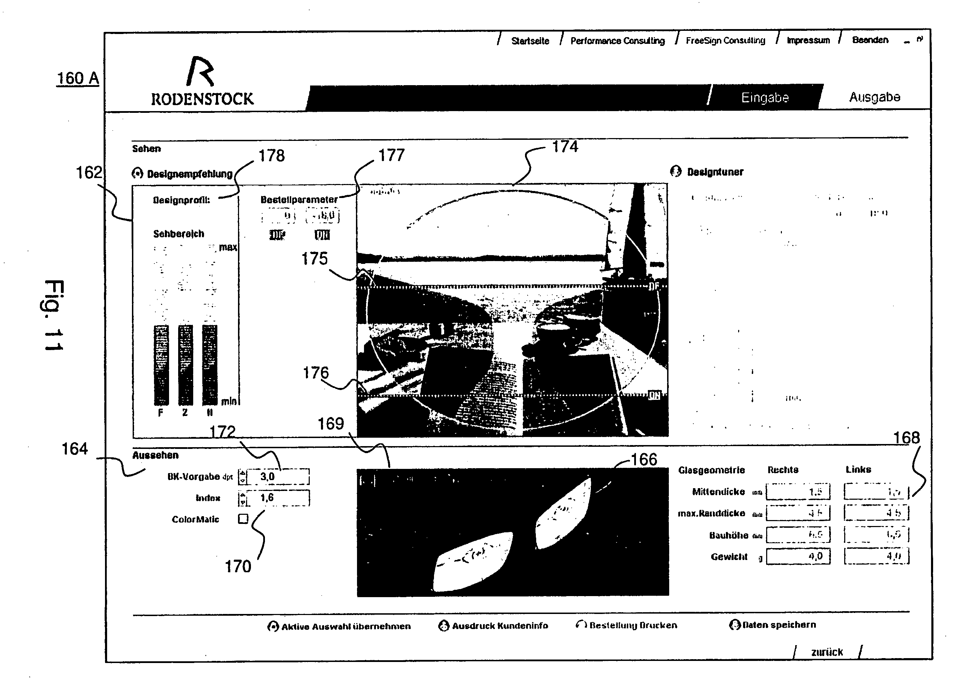

- Fig. 11: Ein Beispiel einer graphischen Benutzerschnittstelle zur Ergebnisdarstellung;

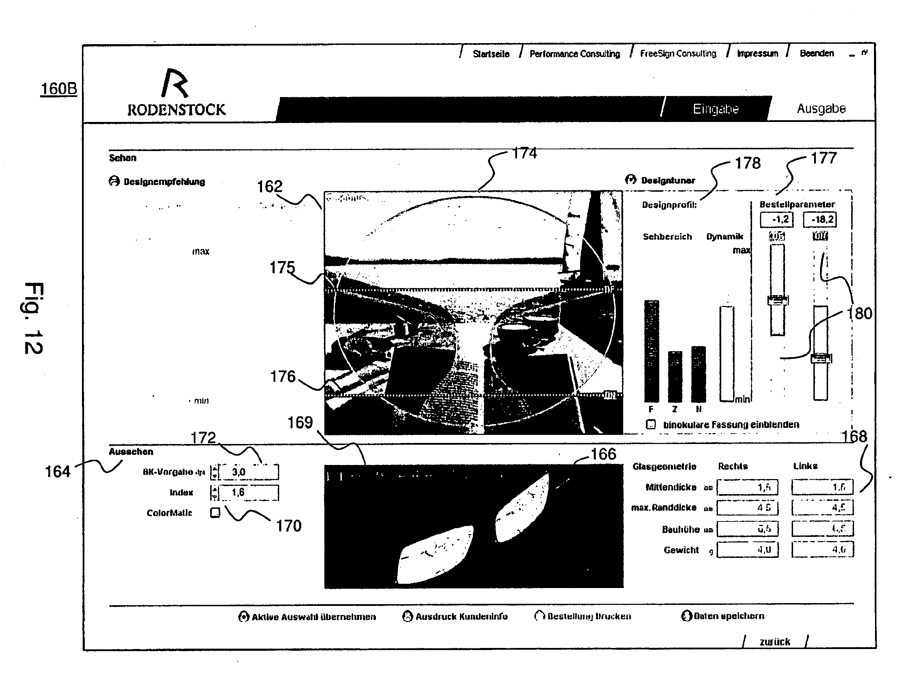

- Fig. 12: Ein Beispiel einer graphischen Benutzerschnittstelle zur Ergebnisdarstellung und Designänderung bzw. -tuning;

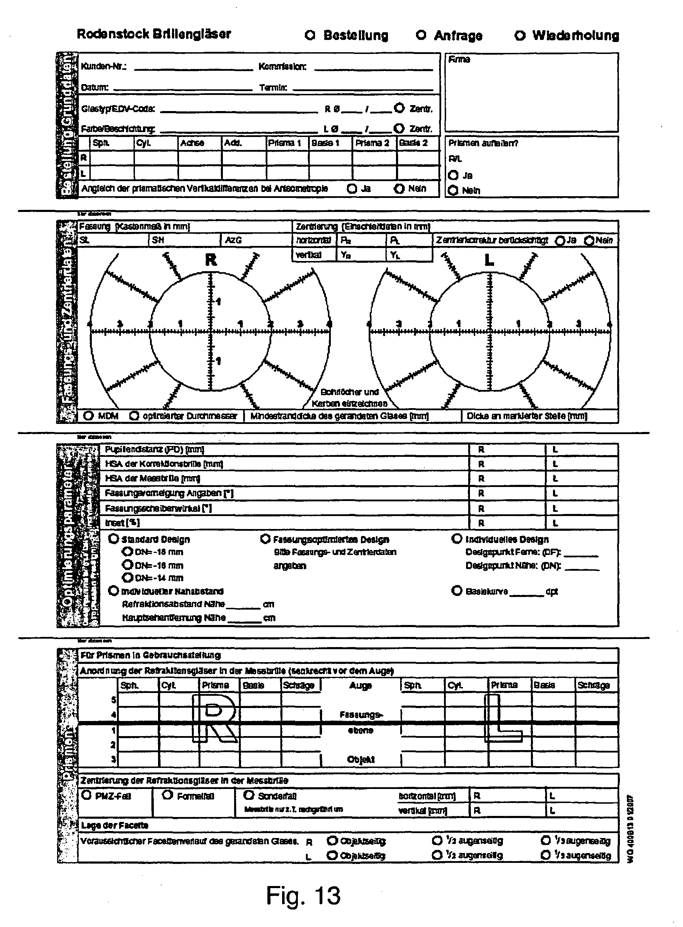

- Fig. 13: Ein Beispiel eines Bestellformulars;

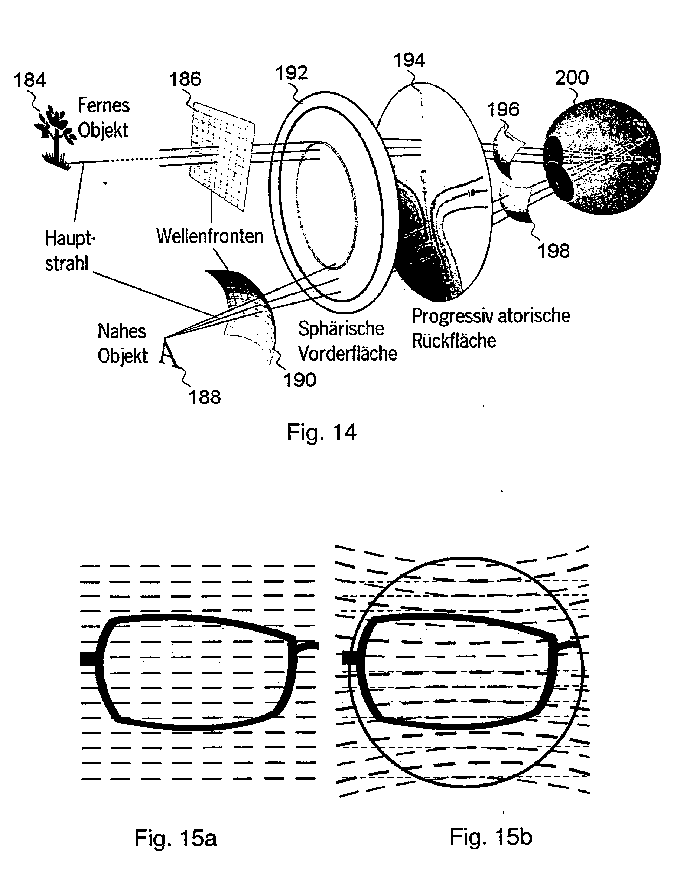

- Fig. 14: Eine schematische Darstellung des physiologischen und physikalischen Modells eines Brillenglases in einer vorgegebenen Gebrauchsstellung;
- Fig. 15 a, b: Schematische Darstellungen der Achslagen in einem Brillenglas ohne Berücksichtigung der Listing'schen Regel (Fig. 15a) und mit Berücksichtigung der Listing'schen Regel (Fig. 15b) ;

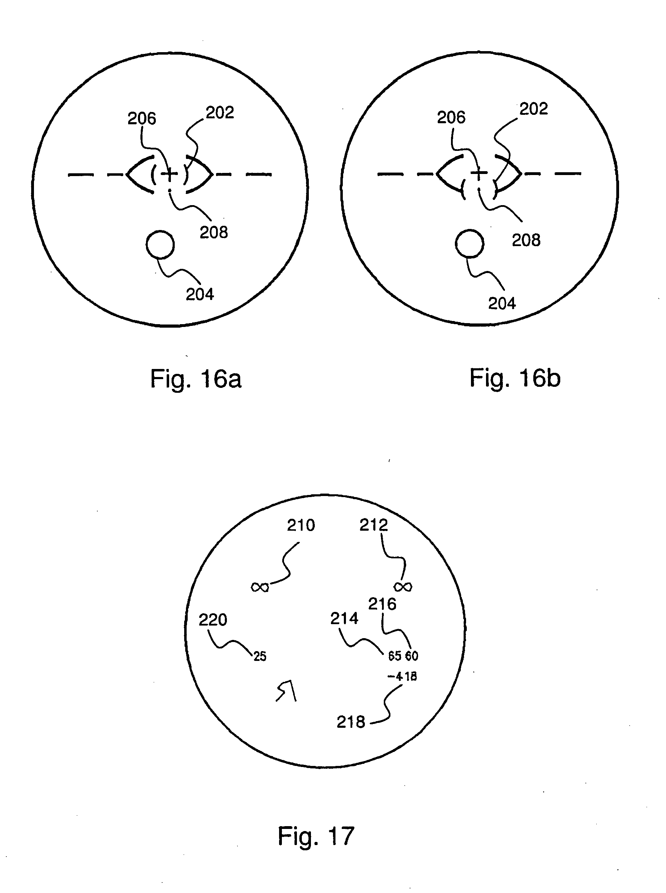

- Fig. 16 a,b: Zwei Beispiele nicht permanenter Stempelungen individuell berechneten progressiven Brillengläsern;
- Fig. 17: Ein Beispiel der permanenten Gravur eines linken individuell optimierten progressiven Brillenglases;

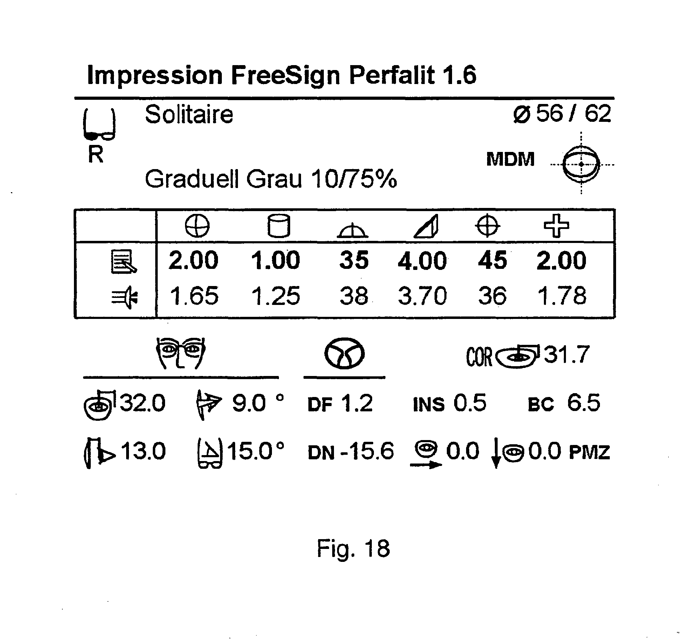

- Fig. 18: Ein Beispiel einer Glastüte für ein individuell optimiertes Brillenglases;

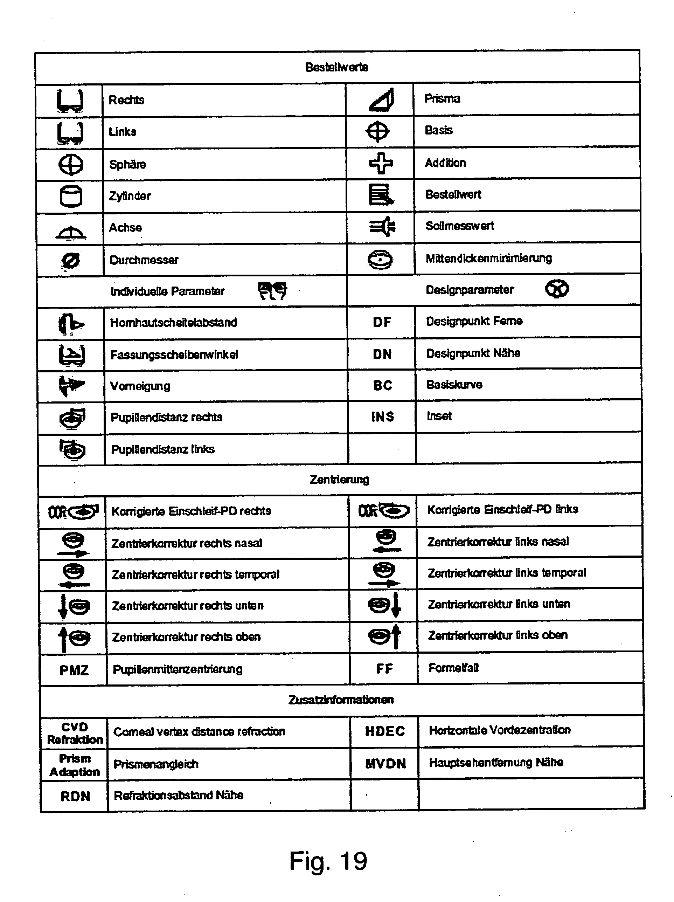

- Fig. 19: Legende der auf der Glastüte verwendeten Piktogramme;

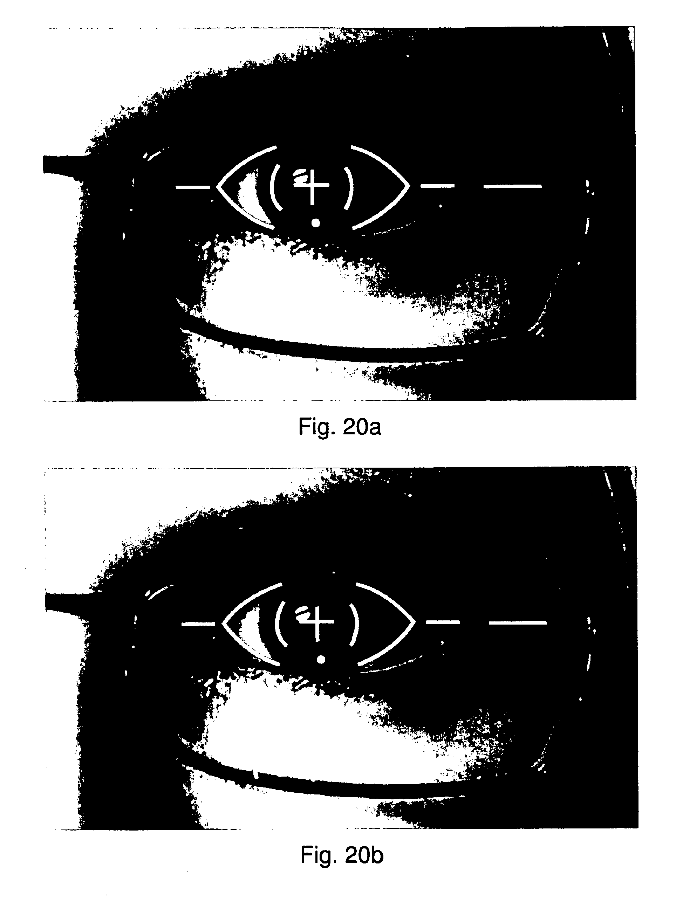

- Fig. 20 a,b: Beispiele der Zentrierung eines individuellen Brillenglases (Fig. 20a) oder eines Standardbrillenglases (Fig. 20b) vor den Augen des Brillenträgers;

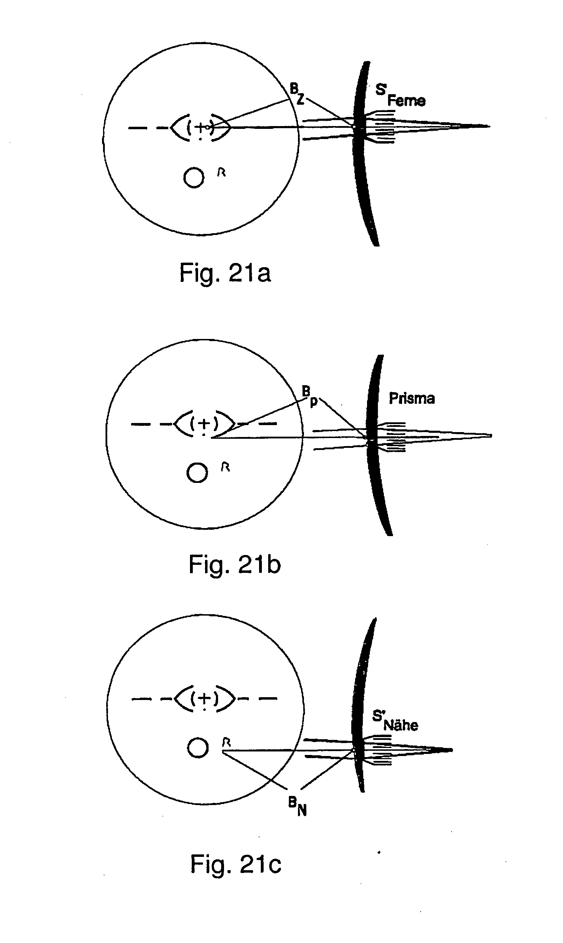

- Fig. 21 a-c: Eine schematische Darstellung der Messung der Wirkungen eines individuellen Brillenglases;

Konventionelle progressive Brillengläser (Gleitsichtgläser) besitzen in der Regel eine progressive Vorderfläche, während die Rezeptfläche augenseitig nach Bestelleingang gefertigt wird. Bei der Fertigung nach dem Basiskurvensystem wird auf eine begrenzte Anzahl (z.B. 72) auf Fehlsichtigkeit angepasste, vorgefertigte und damit standardisierte Gleitsichtflächen zurückgegriffen. Diese gelten allerdings nicht für jede Wirkung separat, sondern für ein bestimmtes Spektrum des Wirkungsbereichs. Die Optimierung der Gleitsichtflächen erfolgt jeweils nur für die mittlere Wirkung pro Basiskurve bzw. Wirkungsbereich. Weichen die Refraktionsdaten von den optimierten Wirkungen ab, hat dies Einschränkungen der nutzbaren Blickfelder zur Folge.

Bereits bei kleinen Abweichungen der bestellten Wirkung in Sphäre, Zylinder, Achse oder auch Prisma und Basis von der dem Blank zugrunde liegenden Berechnung treten bei konventionellen Gleitsichtgläsern Einschränkungen in der Designtreue auf, die zu Unverträglichkeiten beim Brillenträger führen können. Hinzu kommt, dass der Optimierung konventioneller Gleitsichtgläser nur Standardwerte zugrunde liegen, die oft der Individualität der Glas-, Fassungs- und Kundendaten des Brillenträgers nicht gerecht werden.

Bei den wirkungsoptimierten Gleitsichtgläsern werden die Nachteile der konventionellen Gleitsichtgläser durch eine für jede Wirkungskombination einzeln online optimierte asphärische oder atorische Rezeptfläche beseitigt. Mit der Freiformtechnologie ist es möglich, wirkungsoptimierte Gleitsichtgläser zu fertigen. Je nach Berechnungs- und Fertigungs-Know-how können mit der Freiformtechnologie auch individuelle Gleitsichtgläser gefertigt werden.

Es sind ebenfalls individuelle Gleitsichtgläser bekannt, welche unter Berücksichtigung der individuellen Verordnung (Sph, Zyl, Achse, Add, Prisma, Basis) und der individuellen Lage der Gläser vor dem Auge (HSA, FSW, Vorneigung, Pupillendistanz) optimiert und berechnet werden.

Eine zweite Gruppe individueller Gleitsichtgläser sind Gleitsichtgläser, welche auf eine andere Art und Weise personalisiert sind, z.B. durch persönliche Verhaltensweisen des Brillenträgers oder seiner Präferenzen. Diese Gleitsichtgläser berücksichtigen jedoch nicht oder nur teilweise die individuellen Parameter. Diesen Gleitsichtgläsern liegt ein physionomisches Standardmodell zugrunde, welches mit den tatsächlichen Gegebenheiten meist nicht übereinstimmt und so optische Abweichungen und/oder Leistungsverluste nach sich zieht.

In allen Fällen war jedoch das Design eines progressiven Brillenglases bisher fest definiert. Mit einem bevorzugten erfindungsgemäßen Verfahren ist es möglich, das Design des Brillenglases individuell auf die Kundenbedürfnisse anzupassen, wobei individuelle Kundenparameter (z.B. Pupillendistanz (PD), Hornhautscheitelabstand (HSA), Fassungsform, Vorneigung (VN), Fassungsscheibenwinkel (FSW), individuelle Position des Fern- und/oder Nahbezugspunkte, individueller Nahabstand, etc.) berücksichtigt werden.

Vorzugsweise fließen die Seherfahrung und die Bedürfnisse bzw. Sehanforderungen des Kunden in einem bevorzugten erfindungsgemäßen Verfahren zum Berechnen eines individuellen Designs und zum Herstellen eines Brillenglases ein. Somit ist es möglich, unter Benutzung des fachlichen Know-how z.B. eines Augenoptikers und unter Mitwirkung des Kunden (Brillenträgers), ein individuelles progressives Brillenglas zu gestalten. Vorzugsweise werden dabei Vor- und Nachteile des Vorgängerglases berücksichtigt.

Die individuellen Parameter (z.B. Pupillendistanz PD, Hornhautscheitelabstand HSA, Vorneigung VN, Fassungsscheibenwinkel FSW, etc.), welche beispielsweise automatisch mittels einer geeigneten 3-D Meßvorrichtung, wie z.B. mittels der 3-D Videozentriervorrichtung ImpressionIST der Firma Rodenstock GmbH oder alternativ mittels herkömmlichen Meßtools ermittelt werden, werden bei der Berechnung bzw. Optimierung der räumlichen Lagen, insbesondere der vertikalen und/oder horizontalen Position des Fern- und/oder Nahbezugspunkts berücksichtigt.

Die individuellen Parameter können beispielsweise in folgenden Grenzen variieren:
Pupillendistanz (PD) von 30 bis 80 mm
Hornhautscheitelabstand (HSA) von 3 bis 50 mm
Vorneigung (VN) von -10 bis +20 Grad
Fassungsscheibenwinkel (FSW) von -10 bis +35 Grad.

Ferner können neben den individuellen Parametern auch die speziellen Sehgewohnheiten des Brillenträgers berücksichtigt werden.

Figuren 5 bis 7 zeigen graphische Benutzerschnittstellen zur Eingabe von individuellen Kundenparametern.

So können z.B. in einer ersten Maske bzw. graphischen Benutzerschnittstelle (nicht gezeigt) Informationen zu dem Kunden (z.B. Name, Kontaktadresse, Fassungswahl, etc.) eingegeben oder z.B. aus einer Datenbank übernommen werden. Die ausgewählte Fassung, welche z.B. mit Hilfe eines geeigneten Tracers (wie z.B. ImpressionIST der Firma Rodenstock) direkt vermessen oder aus einer Datenbank abgerufen werden kann, kann ebenfalls angezeigt werden.

In Fig. 5 ist ein Beispiel einer Maske bzw. einer graphischen Benutzerschnittstelle 120 zur Eingabe von individuellen Daten (Bestellwerten) des Brillenträgers gezeigt. Die individuellen Daten können direkt in den entsprechenden Eingabefeldern bzw. -abschnitten der Maske bzw. der graphischen Benutzerschnittstelle eingegeben oder z.B. aus einer Datenbank abgerufen werden. Die grau hinterlegten Felder werden automatisch vom dem Programm berechnet bzw. mit Daten belegt.

Die in **Fig. 5** gezeigte graphische Benutzerschnittstellen **120** enthält:
- einen Abschnitt "Refraktionsdaten" (Abschnitt **122)** umfassend Eingabefelder zur Eingabe von individuellen Refraktionsdaten wie Sphäre "Sph", Zylinder "Cyl", Achse, Prisma, Basis);
- einen Abschnitt "Individuelle Parameter" (Abschnitt **124)** umfassend Eingabefelder zur Eingabe von individuellen Parametern der Augen des Brillenträgers und/oder der individuellen Gebrauchsstellung (Pupillendistanz "PD", Hornhautscheitelabstand "HSA", Vorneigung "VN", Fassungsscheibenwinkel "FSW");
- einen Abschnitt "Fassungs- und Zentrierdaten" (Abschnitt **126)** umfassend Eingabefelder zur Eingabe von Fassungs- und Zentrierdaten (Einschleifhöhe, Scheibenlänge, Scheibenhöhe, Abstand zwischen den Gläsern "AzG" bzw. "AZG") sowie gegebenenfalls einen Anzeigeabschnitt (Abschnitt **127)** zur Anzeige der Zentrierung der Brille angepasst an dem Kastenmaß.

Die Fassungsdaten können in entsprechenden Eingabefeldern eingegeben werden. Diese Werte können automatisch eingetragen werden, wenn die Fassung z.B. mittels eines Tracers oder aus einer Liste von Fassungen ausgewählt wird. Die Zentrierdaten können gegebenenfalls direkt von einem 3-D Videozentriersystem (z.B. 3-D Videozentriersystem der Firma Rodenstock GmbG) übernommen werden. Mit einer Funktion "Anpassen an Kastenmaß" kann die Fassung den gegebenenfalls geänderten Fassungsdaten anpasst werden.

Im Abschnitt **126** "Fassungs- und Zentrierdaten" kann eine Fassung aus einer Datenbank ausgewählt werden, z.B. falls vorher mittels eines Tracers keine Fassung aufgenommen bzw. vermessen worden ist. Insbesondere kann die Fassung aus einer Liste in einem sich öffnenden Pop-up-Fenster ausgewählt werden. Form- und Fassungsdaten werden vorzugsweise ebenfalls angezeigt. Die Auswahl kann durch Bestätigen übernommen werden. Ebenfalls kann eine genäherte Fassung aus einer Anzahl verschiedener Fassungsformen ("genäherte Formen") ausgewählt werden. Hierbei kann sich z.B. ein anderes Pop-up-Fenster öffnen, in dem die Fassung aus einer Auswahl gängiger Formen ausgewählt werden kann.

Die in **Fig. 5** gezeigte graphische Benutzerschnittstelle **120** enthält ferner einen Abschnitt bzw. Eingabefeld "Inset" (Abschnitt **132).** Sollte der Brillenglasträger ein vom Standardfall abweichendes Konvergenzverhalten beim Blick in die Nähe zeigen, so kann der im Eingabefeld "Inset" vorbelegte Wert geeignet geändert werden. Der Inset-Wert wird vorzugsweise unter Berücksichtigung der individuellen Kundenparameter berechnet.

Ebenfalls weist die graphische Benutzerschnittstelle **120** einen Abschnitt **134 "Designparameter"** mit entsprechenden Eingabefelder zur Eingabe von:
- den individuellen Nahabstand bei der Refraktionsbestimmung (Refraktionsabstand Nähe);
- der Hauptsehentfernung Nähe; und
- der individuellen Zusatzwirkung
auf.

Wenn keine Daten bezüglich des individuellen Nahabstands eingegeben werden, wird angenommen, daß bis zur einen Addition von 2,5 dpt der individuelle Nahabstand bei der Refraktionsbestimmung 40 cm beträgt. In anderen Worten wird angenommen, daß die bestellte Addition in 40 cm bestimmt wurde und auch die Hauptsehentfernung des Brillenglasträgers in dieser Entfernung liegt. Bei höheren Additionen entspricht der Kehrwert der Addition dem maximalen Nahabstand. Wenn nur eines der beiden Eingabefelder "Refraktionsabstand Nähe" oder "Hauptsehentfernung Nähe" ausgefüllt wird, wird angenommen, daß der Wert auch für die jeweils andere Entfernung gilt. Der Inset und der Astigmatismus werden auf die Hauptsehentfernung "Nähe" berechnet.

Wenn für den Refraktionsabstand "Nähe" und für die Hauptsehentfernung "Nähe" jeweils unterschiedliche Werte in dem entsprechenden Eingabefeld eingetragen werden, wird automatisch auch die individuelle Zusatzwirkung für die Hauptsehentfernung berechnet. Die individuelle Zusatzwirkung wird angezeigt, wenn sie außerhalb des Lieferbereichs liegt (0,75 dpt bis 3,50 dpt) oder mehr als 0,5 dpt von der Bestelladdition abweicht.

### Beispiel:

Bestellte Addition (Refraktion) = 2.00 dpt, Hauptsehentfernung Nähe = 30 cm, Refraktionsabstand in der Nähe = 40 cm. Die bestellte Addition von 2.00 dpt wird für 30 cm optimiert und es erfolgt eine Anpassung der Addition. Neben dem Inset wird auch der Astigmatismus schiefer Bündel für die gewünschte Hauptsehentfernung korrigiert.

Wenn nur ein Abstand (Hauptsehentfemung oder Refraktionsabstand) angegeben wird, wird davon ausgegangen, daß sich die bestellte Addition auf den angegebenen Abstand bezieht. Hier erfolgt keine Anpassung der Addition, und das Brillenglasdesign bzw. das Brillenglas wird für die bestellte Addition im angegebenen Nahabstand berechnet bzw. optimiert. Wird kein Nahabstand (Hauptsehentfernung und/oder Refraktionsabstand) angegeben, wird davon ausgegangen, daß in 40 cm refraktioniert worden ist und dieser Refraktionsabstand der Hauptsehentfernung in der Nähe entspricht. Hier erfolgt keine Anpassung der Addition und das individuelle Brillenglasdesign bzw. das individuelle Brillenglas wird für die bestellte Addition für 40 cm berechnet bzw. optimiert. In der Regel liegen die von Herstellern lieferbaren Additionen in einem Bereich zwischen 0,75 dpt bis 3,50 dpt. Anhand der folgenden einfachen Rechnung kann der Optiker bzw. Optometrist überprüfen, ob das Brillenglas lieferbar ist: $IZ \left(dpt\right) = Add \left(dpt\right) - \left(\frac{1}{RDN \left(m\right)}\right) + \left(\frac{1}{MVDN \left(m\right)}\right) ,$
wobei:
- *IZ*: die individuelle Zusatzwirkung in dpt;
- *Add*: die Addition in dpt;
- *RDN*: den Betrag des Refraktionsabstands Nähe in Meter; und
- *MVDN*: den Betrag der Hauptsehentfernung Nähe
bezeichnen.

Beispiele:

### Möglich:

Addition Refraktion = 1,75 dpt;
Refraktionsabstand Nähe = 40 cm;
Hauptsehentfernung Nähe = 30 cm; $IZ = 1 , 75 dpt - 2 , 50 dpt + 3 , 33 dpt = 2 , 58 dpt .$

### Nicht möglich:

Addition Refraktion = 2,00 dpt;
Refraktionsabstand Nähe = 40 cm;
Hauptsehentfernung Nähe = 20 cm; $IZ = 2.00 dpt - 2.50 dpt + 5.00 dpt = 4 , 50 dpt$

In der Rechnung wird davon ausgegangen, daß sich keine Änderung des Akkommodationserfolgs aufgrund der Veränderung des Nahabstands ergibt. Dies stellt jedoch lediglich eine Näherung dar.

Die in **Fig. 5** gezeigte graphische Benutzerschnittstelle **120** weist ferner einen Abschnitt bzw. ein Eingabefeld "Basiskurve" (Abschnitt **135)** auf, in welches die für die ausgewählte Fassung am besten passende Basiskurve eingetragen werden kann. Insbesondere ist es möglich, eine abweichende Basiskurve in Abhängigkeit von der Durchbiegung der Brillenfassung einzugeben und in der Optimierung des Brillenglases zu berücksichtigen. Das Programm berechnet automatisch die am besten geeignete Durchbiegung bzw. Basiskurve für die jeweiligen Refraktionsdaten und den jeweiligen Basiskurvenwunsch. Die von dem Programm berechnete Basiskurve kann sich von der im Eingabefeld "Basiskurve" eingegebenen Basiskurve unterscheiden. Vorzugsweise wird die eingegebene bzw. bestellte Basiskurve dahingehend automatisch überprüft, daß keine Plan- und Konvexflächen auf der Rückfläche oder zu stark gekrümmte Rückflächen entstehen, die insbesondere zu hohe Randdicken verursachen können.

**Fig. 6** zeigt eine Maske bzw. eine graphische Benutzerschnittstelle **140** zur Eingabe von individuellen Daten bezüglich der aktuellen bzw. bisher getragenen Brille.

In diese Maske können Angaben zum Vorgängerglas gemacht werden, falls dieses bekannt ist. So kann aus einer Liste **142** ("Glastyp") ausgewählt werden, ob der Kunde zuvor ein Einstärken-, Mehrstärken- oder Gleitsichtglas getragen hat oder ob es sich um die erste Brille des Kunden handelt (keine Vorgängerbrille). Wurde ein Gleitsichtglas getragen, können zum Beispiel nähere Angaben zum Material, zum Brechungsindex und/oder zur Progressionslänge z.B. in einem Pop-up-Menü gemacht werden. Die Progressionslänge des Vorgängerglases kann ferner automatisch anhand der Wahl des Vorgängerprodukts oder manuell eingegeben werden. Insbesondere kann die Progressionslänge des Vorgängerglases grob z.B. als eine "Standard" bzw. lange Progression oder als eine kurze ("XS") Progressionslänge eingestuft werden.

Wenn die Addition des Vorgängerglases bekannt ist, kann diese in einem dafür vorgesehenen Eingabefeld **144** "Addition des Vorgängerglases" eintragen werden. So kann die Addition des Vorgängerglases mit der neuen Addition verglichen werden. Bei einer Additionserhöhung von mehr als 0,5 dpt kann ein Hinweisfeld (z.B. als Pop-up-Fenster) erscheinen, das auf die Besonderheiten der Additionserhöhung hinweist.

**Fig. 7** zeigt ein Beispiel einer Maske bzw. eine graphische Benutzerschnittstelle **146** ("Designprofiler") zur Eingabe von Daten bezüglich der individuellen Präferenzen bzw. Gewichtungen der Sehbereiche.

Jeweils fünf verschiedene Piktogramme für die Ferne, die mittlere Entfernung und die Nähe sowie für das Aktivverhalten des Brillenträgers symbolisieren die Bereiche, die der Brillenträger bei der Wahl seines Designprofils gegeneinander abwägen soll. Die Piktogramme dienen als Beispiele für den jeweiligen Entfernungsbereich und stellen nur eine kleine Auswahl aller möglichen Tätigkeiten für diese Entfernung dar. Mit den zu vergebenden Punkten können die Bereiche gewichtet werden.

In einem konkreten Beispiel können insgesamt 9 Punkte auf die vier verschiedenen Bereiche (Ferne, mittlere Entfernung, Nähe und Aktivverhalten) verteilt werden. Je wichtiger dem Kunden der jeweilige Entfernungsbereich ist bzw. je mehr seiner Tätigkeiten in einen Bereich fallen, desto mehr Punkte für diesen Bereich werden vergeben. Die Anzahl der Punkte pro Bereich sowie die Gesamtpunktzahl können beschränkt werden. So können z.B. pro Bereich maximal 5 Punkte vergeben werden, insgesamt jedoch nicht mehr als 9 Punkte.

Die vergebenen Punkte bestimmen das individuelle Designprofil des Brillenträgers. Vereinfacht gilt: Je mehr Punkte für die Ferne in Relation zur gegebenen Gesamtpunktzahl vergeben werden, desto tiefer liegt der individuelle Fernbezugspunkt und je mehr Punkte für die Nähe in Relation zur gegebenen Gesamtpunktzahl vergeben werden, desto höher liegt der individuelle Nahbezugspunkt. Die Punkte für das Aktivverhalten und das Sehen in mittleren Entfernungen wirken sich in erster Linie auf die Länge der Progressionszone aus und bestimmen somit auch wie verzeichnungsfrei das Brillenglas ist. Bei der Vergabe der Punkte entspricht eine gleich große Punktzahl in jedem Bereich einem ausgewogenen, universellen Design.

**Fig. 8** zeigt die Lage der Fern- und Nahbezugspunkte eines individuellen Brillenglasdesigns **148.** Grau hinterlegt sind die Bereiche **(150** und **152),** in welchen sich der Fernbezugspunkt (Bereich **150)** und der Nahbezugspunkt (Bereich **152)** vorzugsweise befinden können. Die Position des Zentrier- bzw. Anpaßpunkts wird mittels eines Kreuzes **154** (Zentrierkreuz) gekennzeichnet. Der Fernbezugspunkt befindet sich in der Mitte von zwei runden Klammern **156.** Der Nahbezugspunkt befindet sich in der Mitte des Nahmeßkreises **158.**

Die vertikale Höhe des Fernbezugspunkts kann vorzugsweise in einem Bereich von +4 bis -4 mm gegenüber dem für dieses Brillenglas vom Hersteller festgelegten Zentrier- bzw. Anpaßpunkt in Abhängigkeit von den individuellen Daten des Brillenträgers flexibel bestimmt werden. Der Nahbezugspunkt kann vorzugsweise flexibel zwischen 13 und 20 mm vertikal unterhalb des Zentrier- bzw. Anpaßpunkts festgelegt werden. Daraus ergibt sich eine flexibel wählbare Progressionslänge, welche vorzugsweise minimal 13 mm und maximal 24 mm betragen kann. Vorzugsweise können der Fernund Nahbezugspunkt in Stufen von 0,1 mm innerhalb eines zulässigen Bereichs frei festgelegt werden. Wird z.B. der Fernbezugspunkt bzw. auf -4 mm vertikale Höhe verschoben, so muss der Nahbezugspunkt mindestens auf einer vertikalen Höhe von -17 mm liegen. Wird der Fernbezugspunkt +4 mm verschoben, so ergibt sich eine Mindestprogressionslänge von 17 mm, da der Nahbezugspunkt vorzugsweise nicht weiter als -13 mm verschoben wird.

Dabei beträgt vorzugsweise der minimale vertikale Abstand vom unteren Fassungsrand zum Nahbezugspunkt 2 mm. Der Fernbezugspunkt weist vorzugsweise minimal einen vertikalen Abstand von 6 mm, vorzugsweise 8 mm von dem oberen Fassungsrand auf. Mit den minimal zulässigen Abständen des Fern- und Nahbezugspunkts von dem oberen bzw. dem unteren Fassungsrand läßt sich die maximal zulässige Progressionslänge berechnen. Die Progressionslänge wird hierbei als der vertikale Abstand zwischen dem Fern- und dem Nahbezugspunkt definiert.

Aus der folgenden Tabelle kann entnommen werden, was durch Verschieben der Designpunkte im Gleitsichtglas beispielsweise erreicht werden kann:

**Tabelle:**

| **Individuelle Sehanforderung des Brillenträgers** | **Umsetzung beim Festlegen der Position der Designpunkte** |
|---|---|
| Besonders großer Fernbereich, z. B. Autofahrer | Der Designpunkt "Ferne" sollte unter das Zentrierkreuz verschoben werden. Die Progressionszone beginnt dann erst (deutlich) unterhalb des Zentrierkreuzes |
| Besonders großer Nahbereich, z.B. Lektor | Der Designpunkt "Nähe" sollte im Vergleich zum bisherigen Gleitsichtglas nach oben verschoben werden. Dies ermöglicht entspanntes Sehen in der Nähe bei bequemer Blicksenkung |
| Besonders breite Progressionszone, z.B. Architekt | Verschiebung des Designpunkts "Ferne" nach oben und des Designpunkts "Nähe" nach unten. Je länger die Progressionszone, desto breiter wird der Progressionskanal und umso weniger Schaukelbewegungen nimmt der Kunde wahr. |

Die Positionen der Fern- und Nahbezugspunkte sind vorzugsweise gleich für das rechte und das linke Brillenglas. Bei unterschiedlicher Durchblickshöhe können sich dadurch jedoch Nachteile in den Sehbereichen für eines der beiden Augen ergeben. Damit die Sehbereiche für beide Augen voll genutzt werden können, ist es vorteilhaft den jeweils kleineren vertikalen Abstand des Nahbezugspunktes vom Zentrierpunkt auszuwählen bzw. festzulegen.

**Fig. 9** verdeutlicht diesen Zusammenhang. In **Fig. 9** bezeichnet:
- F1^{L,R}: den vertikalen Abstand "Zentrierpunkt- oberer Fassungsrand" des linken (L) bzw. des rechten (R) Brillenglases; und
- F2^{L,R}: den vertikalen Abstand "unterer Zentrierpunkt - unterer Fassungsrand" des linken (L) bzw. des rechten (R) Brillenglases.

In **Fig. 9** fallen der Zentrierpunkt und der Fernbezugspunkt zusammen. Wenn sich die Auswahl der geeigneten vertikalen Position des Nahbezugspunkts an dem unteren Fassungsrand orientiert, würde sich für das rechte Auge ein vertikaler Abstand des Nahbezugspunkts vom Zentrierpunkt vom -18 mm ergeben und für das linke Auge würde dieser Abstand -17 mm betragen. In diesem Fall ist es bevorzugt, den kleineren Abstand auszuwählen bzw. festzulegen.

Die Angaben zu den Objektentfernungen "Ferne" und "Nähe" bei der Refraktionsbestimmung werden bei der Berechnung und Optimierung berücksichtigt. Somit kann der Strahlengang, welcher der tatsächlichen Gebrauchssituation entspricht, genauer simuliert werden und folglich die Abbildungsqualität verbessert werden.

Insbesondere ist es so möglich, die Hauptsehentfernung beim Blick in die Nähe in der tatsächlichen Gebrauchsstellung des Brillenglases bei der Glasberechnung zu berücksichtigen. **Fig. 10a** illustriert die Hauptsehentfernung beim Blick in die Nähe in der tatsächlichen Gebrauchsstellung des Brillenglases und **Fig. 10b** den Refraktionsabstand Nähe bzw. den Nahabstand bei der Refraktionsbestimmung.

Generell wird davon ausgegangen, daß die Addition in einem Refraktionsabstand in der Nähe von 40 cm bestimmt wurde (gilt bis Addition 2,50 dpt, für höhere Additionen gilt 1/Addition) und diese einer Hauptsehentfernung in der Nähe von 40 cm entspricht. Weicht die Hauptsehentfernung in der Nähe vom Refraktionsabstand in der Nähe ab, kann das individuelle Brillenglasdesign auf diese Hauptsehentfernung optimiert werden.

Wenn die optimale individuelle Position des Fern- und/oder Nahbezugspunkts anhand von individuellen Daten des Brillenträgers ermittelt wird, wird ein entsprechendes Brillenglasdesign mit den so ermittelten Positionen des Fern- und Nahbezugspunkts und gegebenenfalls unter Berücksichtigung weiterer individueller Parameter des Brillenträgers automatisch berechnet.

Der Designvorschlag kann mittels einer geeigneten graphischen Benutzerschnittstellen 160A zur Ergebnisdarstellung (Designempfehlung) - wie in **Fig. 11** gezeigt - visualisiert werden. Je nach Auswahl in den Einstellungen, kann zusätzlich eine graphische Benutzerschnittstelle **160B** (Designtuner) gezeigt werden (vgl. **Fig. 12****)** mit deren Hilfe, neben einer Ergebnisdarstellung, dem Benutzer die Möglichkeit gegeben wird, durch eine Veränderung der individuellen Position des Fern- und/oder des Nahbezugspunkts und/oder eine Veränderung der individuellen Daten des Brillenträgers (insbesondere der Präferenzen, der Fassungsdaten, etc.) das Design aktiv zu verändern. Zusätzlich können die entsprechenden geometrischen Daten des Brillenglases (Mittendicke, Randdicke, Basiskurve, Gewicht) berechnet und ebenfalls mittels einer geeigneten graphischen Benutzerschnittstelle (vorzugsweise in Form eines dreidimensionalen Models) visualisiert werden.

Die graphischen Benutzerschnittstellen **160A** und **160B** sind in zwei Bereiche aufgeteilt: im oberen Bereich **162** werden Informationen zum Thema "Sehen" und/oder "Performance" mit dem vorgeschlagenen Design für ein individuelles Brillenglas; im unteren Bereich **164** Informationen zum Thema "Aussehen" bzw. "Geometrie" des individuellen Brillenglases bzw. Brille dargestellt.

Im Bereich "Aussehen" **164** können insbesondere kosmetische Eigenschaften bzw. Daten bezüglich der Ästhetik des Brillenglases (z.B. Gewicht, geometrische Daten wie Bauhöhe, maximale Randdicke, Mittendicke, Basiskurve, etc.) des gerandeten Brillenglases bzw. der gerandeten Brillengläser visualisiert bzw. graphisch dargestellt werden. Die Visualisierung der kosmetischen Eigenschaften des Brillenglases kann beispielsweise mittels einer dreidimensionalen graphischen Darstellung eines Models des Brillenglases **166** mit den ermittelten geometrischen Daten erfolgen, wie z.B. in **Figuren 11** **und** **12** gezeigt. Die Darstellung der kosmetischen Eigenschaften des Brillenglases kann z.B. durch eine Auswahl der Basiskurve und des Brechungsindexes beeinflußt werden. Die Auswahl kann wirkungsabhängig sein.

Der Bereich "Aussehen" **164** kann ferner einen Subbereich **168** umfassen, in welchem numerische Werte bezüglich der geometrischen Eigenschaften des Brillenglases, wie z.B. Bauhöhe, maximale Randdicke, Mittendicke, Gewicht, Basiskurve des gerandeten Glases, etc. gezeigt werden. Diese Werte können Näherungswerte sein, welche gegebenenfalls von den tatsächlichen Glasgeometriedaten abweichen. Zusätzlich können neben den Gravuren auch die individuell ermittelten Fern- und Nahbezugspunkte als Stempelpunkte gezeigt werden.

Mittels geeigneter Buttons können Ansichten **169** der gerandeten Brillengläser aus verschiedenen statischen Perspektiven gezeigt werden (Fassung von oben, Fassung von vorn, von der Seite, von schräg oben). Ebenfalls können z.B. mittels Betätigung eines Animationsbutton die gerandeten Brillengläser in der gewählten Ansicht dynamisch rotierend dargestellt werden. Zur besseren Detailansicht kann das Bild z.B. über einen entsprechenden Button vergrößert dargestellt werden.

Der Bereich "Aussehen" **164** umfaßt ferner einen Abschnitt **170** zur Anzeige von numerischen Werten bezüglich des Brechungsindexes und einen Abschnitt zur Anzeige der Basiskurve (Abschnitt **172**). Die angezeigten Werte für die Basiskurve und den Brechungsindex setzen sich aus dem Wirkungsbereich, dem notwendigen Durchmesser, den Basiskurvenwunsch und den Refraktionsdaten zusammen. Daher sind Abweichungen zu dem der Maske "Bestellwerte" eingegebenen Basiskurvenwunsch möglich. Über entsprechende Auswahlfelder können die technisch realisierbaren Werte für Basiskurve und Brechungsindex des Glases verändert werden. Falls Änderungen der Basiskurven-Vorgabe, der Brechungsindex, etc. vorgenommen werden, können z.B. durch Betätigen eines Buttons "Aktualisieren" die Grafik und die Geometriedaten entsprechend den geänderten Werten neu berechnet werden.

Neben der Visualisierung der kosmetischen Eigenschaften des Brillenglases erfolgt ferner eine Visualisierung der optischen Eigenschaften des Brillenglases (Sehbereiche, insbesondere räumliche Lage und Größe der einzelnen Sehbereiche). Die Darstellung der Sehbereichsgrößen kann lediglich relativ zu den Verordnungsdaten erfolgen, ohne daß eine eventuelle Materialabhängigkeit berücksichtigt wird. Selbstverständlich kann jedoch eine Berücksichtigung einer Materialabhängigkeit vorgesehen werden. Neben einer Visualisierung des "Aussehens" erfolgt eine Visualisierung des "Sehens" durch das Brillenglas. Insbesondere kann eine Visualisierung des Sehkomforts (z.B. Blicksenkung, Schaukeln, peripheres Sehen, Verzerrungen, etc.) vorgesehen werden.

Zusätzlich kann eine geeignete Darstellung von Performancewerten bezüglich der Sehbereiche, des Sehkomforts und/oder der kosmetischen Eigenschaften bzw. der Ästhetik des individuellen Brillenglases vorgesehen werden. Ebenfalls können Performancewerte alternativer Designvorschläge dargestellt werden.

Der Bereich "Sehen" **162** der graphischen Benutzerschnittstellen **160A** und **160B** ist daher grundsätzlich in mehrere Unter- bzw. Subbereiche gegliedert.

In dem Subbereich **174** "Binokulare Sehbereichsdarstellung" des Bereichs **162** wird z.B. das für den Kunden und die angegebene Fassung ideale Design anhand einer Ellipse schematisch gezeigt. Graue Bereiche sind dabei mit Abbildungsfehlern (z.B. Astigmatismus in Gebrauchsstellung größer als 0,5 dpt) behaftete Bereiche. Zusätzlich kann gegebenenfalls der Verlauf der 0,5 dpt Isoastigmatismuslinie gezeigt werden. Die vertikalen Höhen der Fern- und Nahbezugspunkte können jeweils durch (gegebenenfalls unterschiedlich gefärbte) Linien **175, 176** gekennzeichnet werden. Im Subbereich **177** des Bereichs **162** werden numerische Werte für die räumliche Lage (insbesondere für die vertikale Höhe im Bezug auf den Zentrierpunkt) des Fern- und des Nahbezugspunkts gezeigt.

Im Subbereich **178** "Designprofil" des Bereichs **162** wird ein qualitativer Vergleich der Sehbereichsgrößen zueinander, z.B. in Form von unterschiedlich längen Balken dargestellt, wobei F den Fernbereich, Z den Zwischenbereich und N den Nahbereich bezeichnet. Die Länge des jeweiligen Balkens bzw. Schiebers korreliert mit der jeweiligen Schwerpunktsetzung, die einem entsprechenden Entfernungsbereich zugeordnet ist. Da sich die Länge im Designprofil aus den Angaben aller vorheriger Masken ergibt, können sie gegebenenfalls von den zuvor ermittelten Präferenzen bzw. Gewichtungen des Kunden abweichen. Darüber hinaus kann eine qualitative Bewertung des dynamischen Seheindrucks durch das individuelle Brillenglas dargestellt werden. Je höher der Balken, welcher den dynamischen Seheindruck (Balken "Dynamik") darstellt, desto länger wird die Progressionszonenlänge und desto einstärkenähnlicher wird das Brillenglas bzw. desto weniger Schaukeleffekt weist das Brillenglas auf.

Zusätzlich kann dem Augenoptiker/Optometristen und/oder dem Brillenträger die Möglichkeit gegeben werden das so berechnete individuelle Brillenglasdesign aktiv zu verändern. Die Veränderung erfolgt beispielsweise in dem die räumliche Position, insbesondere die vertikale Höhe des Fern- und/oder Nahbezugspunkts, aktiv verändert wird. Alternativ können die Gewichtungen der Sehbereiche verändert werden.

Die Änderung bzw. die Anpassung der Position des Fern- und/oder Nahbezugspunkts und/oder der Präferenzen bezüglich der Sehbereiche kann beispielsweise mittels einer geeigneten graphischen Benutzerschnittstelle erfolgen. Ein Beispiel einer geeigneten graphischen Benutzerschnittstelle in Form von Schieberegler **180** ist in **Fig. 12** gezeigt. Mit den in **Fig. 12** gezeigten Schieberegler **180** ist eine direkte Anpassung der Position des Fern- und/oder Nahbezugspunkts möglich.

Das neue Brillenglasdesign mit der geänderten Position des Fern- und/oder Nahbezugspunkts wird vorzugsweise in Echtzeit berechnet und visualisiert. Vorzugsweise wird ebenfalls die Differenz bzw. die Veränderung der optischen Eigenschaften des neuen gegenüber dem alten Brillenglasdesign visualisiert.

So kann z.B. in dem Designtuner über die in der Designempfehlung (**Fig. 11**) beschriebenen Möglichkeiten hinaus eine Ellipse eingeblendet werden, welche in den Kastenmaßen und der vorgegebenen Zentrierung der genäherten binokularen Kundenfassung entspricht. Des Weiteren kann hier das vorgeschlagene, individuelle Design verändert werden, indem z.B. Schieberegler für den Fern- und den Nahbezugpunkt nach oben bzw. nach unten verschoben werden. Bei den Bestellparametern in den Anzeigefeldern für den Fern- und den Nahbezugspunkt verändern sich entsprechend die Zahlenwerte für die Lage der Bezugspunkte. Ferner verschieben sich in der binokularen Sehbereichsdarstellung die Linien für den Fern- und den Nahbezugspunkt.

Zusätzlich zu den flächig grau dargestellten Sehbereichen aus der Designempfehlung können vorzugsweise farbliche (z.B. gelbe) Sehbereichslinien (z.B. 0,5 dpt I-soastigmatismuslinie) erscheinen, welche das abgeänderte, individuelle Design zeigen. Ebenfalls ändern sich im Subbereich Designprofil (**178**) das Verhältnis der Größe der Sehbereiche zueinander sowie die Länge des Balkens "Dynamik". Die vergebenen Punkte im Abschnitt "Desigriprofiler" werden vorzugsweise durch die Veränderungen im Abschnitt "Designtuner" nicht beeinflusst.

Die nachfolgend aufgeführten Beispiele zeigen individuelle progressive Designs mit individuell, in Abhängigkeit von den erfaßten individuellen Daten des Brillenträgers ermittelten Fern- und Nahbezugspunkte.

### Beispiel 1: Brillenglasträger Architekt:

Der Brillenglasträger legt großen Wert auf einen breiten Zwischenbereich und wünscht sich ein "eher ruhiges" Brillenglas mit wenigen Schaukelbewegungen, da er aus beruflichen Gründen den überwiegenden Teil des Tages im Zwischenbereich (mittlere Entfernungen) sehen muss. In seiner jetzigen Brille trägt er ein Gleitsichtglas mit einer Progressionszonenlänge von 18 mm.

Für diesen Brillenglasträger empfiehlt das Programm für die ausgewählte Fassung und entsprechende Zentrierung den Fernbezugspunkt auf +2,4 mm über das Zentrier- bzw. Anpaßpunkt zu setzen. Der Nahbezugspunkt würde optimal bei -19 mm unterhalb des Zentrier- bzw. Anpaßpunkt liegen. Mit diesem progressiven Brillenglas hat der Architekt einen guten Kompromiß aus entspannter Kopfhaltung, einem breitem Zwischenbereich und geringen Schaukelbewegungen für seine Sehgewohnheiten.

### Beispiel 2: Brillenglasträgerin Lektorin:

Sie legt großen Wert auf einen großen Nahbereich und wünscht sich eine geringere Blicksenkung als bei ihrem jetzigen Gleitsichtglas, da sie aus beruflichen Gründen den überwiegenden Teil des Tages mit Arbeiten in der Nähe verbringt. In der jetzigen Brille trägt sie ein Gleitsichtglas mit einer Progressionszonenlänge von 18 mm. Für diese Kundin empfiehlt das Programm für die ausgewählte Fassung und entsprechende Zentrierung den Fernbezugspunkt auf 1,5 mm über das Zentrier- bzw. Anpaßpunkt zu verlegen. Der Nahbezugspunkt würde optimal bei -15,5 mm unterhalb des Zentrier- bzw. Anpaßpunkts liegen. Damit hat die Lektorin einen guten Kompromiss aus breiterem Nahbereich und entspannter Kopfhaltung.

Wenn der Brillenglasträger großen Wert auf einen breiten Zwischen- und Nahbereich und geringe Schaukelbewegungen im Brillenglas legt, wird von dem Programm empfohlen in Abhängigkeit von den anderen Eingabeparametern den Fernbezugspunkt nach oben zu verschieben. Der Fernbezugspunkt würde sich dann oberhalb vom Zentrier- bzw. Anpaßpunkt befinden. Je nach Refraktionsdaten und individuellen Parametern kann sich in diesen Fällen eine "Nebelung" im Zentrier- bzw. Anpaßpunkt von bis zu +0,25 dpt ergeben. Neben dieser geringfügigen Nebelung im Zentrierpunkt können sich auch seitliche Einschränkungen im Fernbereich ergeben, da der Brillenglasträger - bei einer Verschiebung des Fernbezugspunkts nach oben - in Nullblickrichtung durch die bereits früher beginnende Progression im Brillenglas sieht. Durch die veränderte Lage der Progressionszone im Brillenglas können die Sehbereiche auf Höhe des Zentrierpunktes dementsprechend kleiner ausfallen, weil die peripheren Abbildungsfehler "nach oben" verschoben werden. Der Brillenglasträger erhält jedoch bei der Wahl dieser Positionierung des Fernbezugspunkts ein nach seinen individuellen Sehgewohnheiten gestaltetes bzw. optimiertes Brillenglasdesign bzw. Brillenglas.

### Beispiel 3: Brillenglasträger arbeitet im Außendienst

Der Brillenglasträger legt besonderen Wert auf einen großen Fernbereich, da er aus beruflichen Gründen den überwiegenden Teil des Tages mit Sehen in die Ferne verbringt. In seiner jetzigen Brille trägt er ein Gleitsichtglas mit Progressionszonenlänge 18 mm. Das Programm berechnet automatisch und empfiehlt bei diesem Kunden für die ausgewählte Fassung und entsprechende Zentrierung den Fernbezugspunkt auf -2,5 mm unterhalb des Zentrier- bzw. Anpaßpunkts zu setzen. Der Nahbezugspunkt würde optimal bei -18,4 mm unterhalb des Zentrier- bzw. Anpaßpunkts liegen. Mit diesem Brillenglas hat der Brillenglasträger einen großen Fernbereich und einen guten Kompromiß aus geringen Schaukelbewegungen sowie gut ausnutzbarem Zwischen- und Nahbereich.

### Beispiel 4:

Auf die Frage, für welche Tätigkeiten bzw. Sehanforderungen die Brillenglasträgerin ihre Brille benutzt, ergibt sich z. B. folgendes Profil:
- Sie fährt regelmäßig Auto und sieht fern;
- Sie spielt ein Instrument und geht 2x die Woche zur Orchesterprobe;
- Abends liest Sie zur Entspannung gerne die abonnierte Tageszeitung;
- Mindestens 1 x die Woche treibt sie Sport wie z. B. Joggen oder spielt Handball in ihrem Verein.

In der aktuellen Brille trägt die Brillenglasträgerin Gleitsichtgläser mit normaler Progressionszonenlänge. Da keinerlei Präferenzen festzustellen sind und die Tätigkeiten dieser Kundin gleich auf die Bereiche Ferne, mittlere Entfernung und Nähe verteilt sind, werden jeweils gleich viele Punkte für alle Entfernungen und auch für das Aktivverhalten bzw. Dynamik vergeben. In anderen Worten werden alle Sehbereiche und das dynamische Verhalten bzw. die dynamischen Eigenschaften gleich gewichtet. In dem konkreten Beispiel werden für alle Bereiche des in **Fig. 7** gezeigten "Designprofilers" jeweils 2 Punkte vergeben. In der graphischen Benutzerschnittstelle "Designempfehlung" wird das Ergebnis der Berechnungen unter Berücksichtigung der individuellen Eingaben in die vorherigen Masken dargestellt. Das Programm berechnet automatisch und empfiehlt für diese Kundin den Fernbezugspunkt auf 0 mm und den Nahbezugspunkt auf -18 mm zu positionieren. Dieses Brillenglas würde einem ausgewogenen, universellen progressiven Brillenglas mit einer Progressionszonenlänge von 18 mm entsprechen (z.B. einem Brillenglas bisherigen "Impression ILT^{®}" der Firma Rodenstock GmbH), da bei der Designauslegung davon ausgegangen wird, daß kein Tätigkeitsschwerpunkt in einem der Entfernungsbereiche erkennbar ist.

### Beispiel 5

Auf die Frage, für welche Tätigkeiten bzw. Sehanforderungen der Brillenglasträger seine Brille benutzt, ergibt sich folgendes Profil:
- Er legt besonderen Wert auf ungestörtes Sehen in der Ferne, da er aus beruflichen Gründen den überwiegenden Teil des Tages im Auto verbringt;
- In die mittlere Entfernung schaut er nur, um das Armaturenbrett klar erkennen zu können;
- Die Nähe nutzt der Brillenglasträger nur für kurzzeitige Schreibtischtätigkeiten, wie z.B. Vertragsabschlüsse und Ähnliches;
- In seiner Freizeit spielt er gerne Tennis und Squash, dabei legt er Wert auf wenig Schaukelbewegungen im Brillenglas.

In der aktuellen Brille trägt der Brillenglasträger Gleitsichtgläser mit normaler Progressionszonenlänge (PZL). Die Präferenz des Brillenglasträgers liegt hier eindeutig in der Ferne, die mittlere Entfernung und die Nähe spielen eine untergeordnete Rolle. Daher wurden für dieses Beispiel für die Ferne 4 Punkte, für mittlere Entfernungen und Nähe jeweils 1 Punkt vergeben (vgl. **Fig. 7**). Aufgrund der Anforderungen der dynamischen Sportarten, wie Verzeichnungsfreiheit und gute Raumwahrnehmung, wurde das Aktivverhalten bzw. Dynamik bei dem in **Fig. 7** gezeigten "Designprofiler" mit 3 Punkten gewichtet. In der graphischen Benutzerschnittstelle "Designempfehlung" wird das Ergebnis der Berechnungen unter Berücksichtigung der individuellen Eingaben in die vorherigen Masken dargestellt. Das Programm berechnet automatisch und empfiehlt für diesen Kunden den Fernbezugspunkt auf -1,1 mm und den Nahbezugspunkt auf -18,5 mm zu positionieren. Somit wird ein für die individuellen Anforderungen des Kunden größtmöglicher Fernbereich realisiert. Aufgrund der Lage des Nahbezugspunktes und der damit verbundenen relativ langen Progressionszonenlänge bietet das Brillenglas einstärkenähnliches Sehen und ist nahezu verzeichnungsfrei. Dies wirkt sich positiv z.B. bei den sportlichen Freizeitaktivitäten des Brillenglasträgers aus.

### Beispiel 6

Auf die Frage, für welche Tätigkeiten bzw. Sehanforderungen der Brillenglasträger seine Brille benutzt, ergibt sich folgendes Profil:
- Die Ferne spielt für Ihn eine untergeordnete Rolle, da er z. B. beim Autofahren die Brille meist abnimmt;
- Das Sehen in den mittleren Entfernungen ist ihm besonders wichtig;
- Der Brillenträger reagiert auf ungewohnte Verzeichnungen sehr sensibel, z. B. bei gekrümmten Linien in seinen zeichnerischen Skizzen;
- Nach getaner Arbeit liest er gern Kriminalromane;
- Aufgrund seines stressigen Jobs kommt er nicht dazu, Sport zu treiben oder sich zu bewegen.

Die wichtigste Entfernung für diesen Brillenglasträger ist also die mittlere Entfernung, auch die Nähe ist für ihn wichtig, die Ferne und das Aktivverhalten spielen eine untergeordnete Rolle. Daher werden in dem in **Fig. 7** gezeigten "Designprofiler" für die Ferne und für das Aktivverhalten jeweils 1 Punkt, 3 Punkte für mittlere Entfernungen sowie 2 Punkte für die Nähe vergeben. In der graphischen Benutzerschnittstelle "Designempfehlung" wird das Ergebnis der Berechnungen unter Berücksichtigung der Eingaben in die vorherigen Masken dargestellt. Das Programm berechnet automatisch und empfiehlt, für diesen Kunden den Fernbezugspunkt +0,7 mm und den Nahbezugspunkt auf -18,1 mm zu positionieren. Somit wird ein größtmöglicher Zwischenbereich realisiert. Aufgrund der Lage des Nahbezugspunkts und der damit verbundenen relativ langen Progressionszonenlänge bietet das Brillenglas einstärkenähnliches Sehen und ist nahezu verzeichnungsfrei. Dies kommt dem Brillenglasträger bei der Arbeit mit zeichnerischen Skizzen zugute.

### Beispiel 7

Auf die Frage, für welche Tätigkeiten bzw. Sehanforderungen die Brillenglasträgerin ihre Brille benutzt, ergibt sich folgendes Profil:
- Sie benutzt die Brille kaum für Tätigkeiten in der Ferne, daher spielt diese eine untergeordnete Rolle;
- Für ihren Beruf ist das Lesen von Schriftstücken besonders wichtig;
- Sie legt großen Wert auf eine angenehme Blicksenkung für Nahsehaufgaben;
- Aufgrund der eher statischen Haltung am Arbeitsplatz spielen Schaukelbewegungen eine untergeordnete Rolle;
- In die mittlere Entfernung schaut die Kundin vor allem bei gelegentlicher Bildschirmarbeit.

Die wichtigste Entfernung für diese Brillenglasträgerin ist also die Nähe. Auch die mittlere Entfernung ist wichtig, die Ferne sowie auch das Aktivverhalten spielen eine untergeordnete Rolle. Daher werden in dem in **Fig. 7** gezeigten "Designprofiler" für die Nähe 4 Punkte, für die mittlere Entfernung 2 Punkte und jeweils 1 Punkt für die Ferne und das Aktivverhalten vergeben. In der graphischen Benutzerschnittstelle "Designempfehlung" wird das Ergebnis der Berechnungen unter Berücksichtigung der Eingaben in die vorherigen Masken dargestellt. Das Programm berechnet automatisch und empfiehlt, den Fernbezugspunkt auf +0,8 mm und den Nahbezugspunkt auf -17,0 mm zu positionieren. Somit wird ein für die individuellen Anforderungen der Brillenglasträgerin größtmöglicher Zwischen- und Nahbereich realisiert. Aufgrund der Lage des Nahbezugspunkts wird der Wunsch der Brillenglasträgerin nach einer angenehmen Blicksenkung für Nahsehaufgaben in ihrem individuellen Gleitsichtglas umgesetzt.

Mit einem Button "Aktive Auswahl übernehmen" kann festgelegt werden, welche Daten für die Bestellung übernommen werden sollen. Es werden z.B. stets die Daten des Bereichs übernommen der gerade aktiv (nicht im Hintergrund) ist. Nach Betätigen des Buttons "Aktive Auswahl übernehmen" kann ferner ein mit dem Ergebnis gefülltes Bestellformular ausgedruckt werden. Das Bestellformular kann ferner z.B. mit weiteren Details, wie z.B. Farbe, Beschichtung, ColorMatic Farbe, Messbrille usw. ergänzt werden. Die individuellen Daten können ebenfalls gespeichert werden und/oder online an einem Brillenglashersteller übermittelt werden.

Die individuellen Daten des Brillenträgers können ebenfalls mittels geeigneter Bestellformulare erfaßt und dem Brillenglashersteller weiter übermittelt werden. **Fig. 13** zeigt ein beispielhaftes Bestellformular. In dem Bestellformular werden die erfaßten individuellen Refraktionsdaten (Sphäre, Zylinder, Achse, Prisma, Basis), Fassungsund Zentrierdaten, individuelle Parameter der Augen des Brillenträgers und der individuellen Gebrauchsstellung (Pupillendistanz, Fassungsscheibenwinkel, Vorneigung, Hornhautscheitelabstand, etc.) und gegebenenfalls weitere individuelle Daten angegeben. Mit dem Bestellformular ist es möglich, die Positionen der Fern- und/oder Nahbezugspunkte so zu wählen, daß diese den Positionen eines universellen Gleitsichtglasdesigns entsprechen (z.B. Impression^{®} oder Impression XS^{®} der Firma Rodenstock GmbH). Ebenfalls ist es möglich eine mittlere Progressionszonenlänge von 16 mm festzulegen. Alternativ können die Positionen der Fern- und/oder Nahbezugspunkte in Abhängigkeit von den individuellen Fassungsdaten festgelegt werden (fassungsoptimiertes Design). So kann z.B. der Fernbezugspunkt auf dem Zentrierpunkt (d.h. bei 0 mm) und der Nahbezugspunkt auf 2 mm oberhalb vom unteren Fassungsrand festgelegt werden. Die Position der Fern- und Nahbezugspunkte kann ferner unter Berücksichtigung von weiteren individuellen Daten (z.B. Tätigkeitsschwerpunkte und Präferenzen bezüglich der Sehbereiche), wie oben in Detail beschrieben, individuell festgelegt werden.

Anschließend wird ein individuelles Brillenglas optimiert bzw. berechnet, wobei die Optimierung unter Berücksichtigung zumindest eines Teils der ermittelten individuellen Daten, insbesondere Daten bezüglich der individuellen Parameter des Brillenträgers und der individuellen Gebrauchsstellung (Fassungsscheibenwinkel, Vorneigung, Pupillendistanz, Hornhautscheitelabstand, etc,) erfolgt.

Um die Abbildungseigenschaften von Brillengläsern in der Tragesituation zu beschreiben und/oder zu berechnen, sind in der geometrischen Optik zwei Berechnungsverfahren bekannt:
- Berechnung mit Lichtstrahlen (Ray Tracing); und
- Berechnung mit Wellenfronten (Wave Tracing).

Der Begriff "Ray Tracing" setzt sich aus Ray = Strahl und Tracing = Verfolgung zusammen. In der geometrischen Optik wird das Ray Tracing Verfahren eingesetzt, um optische Abbildungen zu beschreiben. Die Berechnung eines Brillenglases mit Ray Tracing ist jedoch sehr zeitaufwändig, da für jeden Punkt im Brillenglas außer dem eigentlichen Lichtstrahl bzw. Hauptstrahl auch noch ein "begleitendes" Bündel an Nachbarstrahlen durch das Brillenglas simuliert werden muss.

Vorzugsweise wird das individuelle Brillenglas mittels eines Wavefront-Tracing-Verfahrens, insbesondere mittels einer lokalen Wellenfrontoptimierung berechnet. Der Begriff "Wave Tracing" setzt sich aus Wave = Welle und Tracing = Verfolgung zusammen. Wellenfronten können genauso wie Lichtstrahlen eingesetzt werden, um optische Abbildungen zu beschreiben oder zu berechnen. Eine Wellenfront ist die Fläche gleicher Phase einer sich ausbreitenden Welle. Eine jede solche Wellenfront fasst sämtliche Eigenschaften eines Bündels aus Nachbarstrahlen in einem einzigen Objekt zusammen. Dadurch kann die Berechnungszeit deutlich reduziert werden, so daß eine individuelle Optimierung jedes einzelnen Brillenglases ermöglicht wird. Insbesondere ist es durch die freie Wahl der Designpunkte Ferne und/oder Nähe möglich, die Verteilung der Abbildungseigenschaften auf dem Brillenglas wunschgerecht an die individuellen Sehgewohnheiten des Brillenträgers anzupassen.

**Fig. 14** zeigt eine schematische Darstellung des physiologischen und physikalischen Modells eines Brillenglases in einer vorgegebenen Gebrauchsstellung, welches der individuellen Brillenglasberechnung bzw. Optimierung zugrunde liegt. In **Fig. 14** ist zu erkennen, daß die Strahlen von einem unendlich entfernten Objekt **184** alle parallel verlaufen, was sich in einer ebenen Wellenfront **186** widerspiegelt. Im Gegensatz dazu divergieren die Strahlen, die von einem nahen Objekt **188** ausgehen. Entsprechend ist die Wellenfront **190** gewölbt. Das Brillenglas, welches eine vorzugsweise sphärische Vorderfläche **192** und eine individuelle berechnete progressive-atorische Rückfläche **194** aufweist, muss nun dafür sorgen, daß jede Wellenfront **196, 198** auf der Augenseite vorzugsweise so gekrümmt ist, daß das entsprechende Objekt **184, 188** scharf auf der Netzhaut des Auges **200** abgebildet wird. Idealerweise müssen diese Wellenfronten augenseitig für alle Blickrichtungen gleich stark gekrümmt sein.

Zur Berechnung des Brillenglases wird vorzugsweise eine flexible Flächengestaltung der individuell zu berechnenden progressiven Fläche mit einer Mehrzahl an Bewertungsstellen (vorzugsweise über 7000 Bewertungsstellen) eingesetzt, wobei jeder dieser Bewertungsstellen eine eigene lokale Wellenfrontdurchrechnung zugeordnet wird. Die individuelle progressive Fläche wird vorzugsweise mittels Minimierung einer Zielfunktion, welche an den Bewertungsstellen ausgewertet wird, und unter Berücksichtigung des physiologischen Sehmodells optimiert. Auf diese Weise ist es möglich, durch individuelle Wellenfrontdurchrechnungen sehr schnell und damit online nach dem Bestelleingang die Optimierung eines Brillenglases nach der variablen Zielfunktion durchzuführen.

Die Berechnung des Brillenglases umfaßt vorzugsweise eine Optimierung mit mehr als 2000 Optimierungsparametern in einem hochdimensionalen Raum. Für die so vorgenommene Echtzeit-Online-Optimierung können Multiprozessor-Großrechner eingesetzt werden.

Vorzugsweise werden bei der individuellen Optimierung des Brillenglases nicht nur Abbildungsfehler niedriger Ordnung (Sphäre, Zylinder, Prisma), sondern auch Abbildungsfehler höherer Ordnung (z.B. Koma und sphärische Abberation) minimiert. Diesbezüglich wird z.B. auf US 7,063,421 B1 verwiesen. Die Fertigung des individuell berechneten Brillenglases erfolgt z.B. mittels Präzisionsmaschinen, vorzugsweise CNC-Schleif- und Poliermaschinen, welche die berechneten Flächendaten mit einer Genauigkeit in µm-Bereich umsetzten können.

Vorzugsweise wird bei der Optimierung der individuellen Brillengläser insbesondere die Listing'sche Regel berücksichtigt.

**Figuren 15** **a und b** stellen jeweils schematische Darstellungen der Achslagen in einem Brillenglas ohne Berücksichtigung der Listing'schen Regel (Fig. 15a) und mit Berücksichtigung der Listing'schen Regel (Fig. 15b).

Da das Auge bei peripheren Blickauslenkungen eine leichte Rollbewegung ausführt, darf es keine feste Zylinderachse über das gesamte Brillenglas geben, sondern diese muss sich im Übergang von der Horizontalen zur Vertikalen leicht ändern (Fig. 15b). Wenn der im Auge vorhandene (durch die Refraktion bekannte) Zylinder durch das Brillenglas gut korrigiert werden soll, muss die Achslage des Zylinders im Brillenglas gut zu der Achslage passen, die das Auge aufgrund seiner Rollbewegung tatsächlich einnimmt. Passen die Achslagen von Auge und Brillenglas nicht zusammen, so ergeben sich optisch zwei schief gekreuzte Zylinder. Der Brillenglasträger hätte bei schiefen, seitlichen Blickbewegungen somit einen Astigmatismus, welcher nicht ausgeglichen wird. Das hat einen Visusabfall in diesem Bereich zur Folge. Vorzugsweise wird daher die Torsionseinstellung bei der Berechnung des individuellen Brillenglases berücksichtigt. Die Berücksichtigung der Listing'schen Regel wird umso relevanter:
- je größer der Refraktionszylinder des Kunden ist, und/oder
- je stärker die Blickauslenkung von der horizontalen und vertikalen Auslenkung abweicht, und/oder
- je stärker bzw. größer die Blickauslenkung absolut ist.

In einem konventionellen progressiven Brillenglas mit einer progressiven Vorderfläche und einer sphärische/torischen Rezeptfläche kann die Listing'sche Regel - im Gegensatz zu Brillengläsern mit einer progressiven, individuellen, augenseitigen Freiformfläche - nicht umgesetzt werden.

Ferner bevorzugt wird bei der Optimierung und Berechnung des individuellen progressiven Brillenglases eine individuelle Vordezentration berücksichtig. Somit werden die ausnutzbaren Durchmesser vergrößert. Die optimale Vordezentration kann anhand von Daten bezüglich der Fassungs- bzw. Brillenglasform sowie Daten bezüglich der Zentrierung automatisch berechnet werden. Alternativ kann eine individuelle Vordezentration vom Augenoptiker/Optometrist selbst festgelegt werden. In diesem Fall kann der anhand einer speziellen Zentrierkarte ermittelte gewünschte Durchmesser ebenfalls berücksichtigt werden. Insbesondere kann eine Vordezentration von bis zu 5 mm berücksichtigt werden.

Das individuell berechnete Brillenglas weist vorzugsweise eine sphärische oder rotationssymmetrische asphärische objektseitige Vorderfläche und eine individuelle in Abhängigkeit von den individuell festgelegten Bezugs- bzw. Designpunkte Ferne und Nähe, den individuellen Refraktionsdaten, den individuellen Parameter des Brillenträgers und der Gebrauchssituation (z.B. Pupillendistanz, Vorneigung, Fassungsscheibenwinkel, Hornhautscheitelabstand, etc.) optimierte progressive, augenseitige Freiformfläche auf.

Die Position der individuellen Fern- und Nahbezugspunkte wird vorzugsweise mittels einer individuellen Stempelung mittels nicht permanenten Markierungen aufgezeichnet. Vorzugsweise kann die Position der individuellen Fern- und Nahbezugspunkte ferner anhand von permanenten Markierungen bzw. Mikrogravuren des Brillenglases und einer Rekonstruktionsvorschrift (Schablone, Zentrierkarte) eindeutig rekonstruiert werden.

**Fig. 16** **a** und **b** zeigen Beispiele von nicht permanenter Stempelung zweier individuellen progressiven Brillengläsern.

Die nicht permanente Markierung bzw. der Stempel eines individuell nach einem bevorzugten erfindungsgemäßen Verfahren optimierten Brillenglases besteht aus "beweglichen" und "fixen" Bestandteilen. Zu den beweglichen Teilen gehören zwei runde Klammern **202,** welche die Lage des Fernbezugspunkts bzw. des Designpunkts Ferne kennzeichnen, sowie der Nahmeßkreis **204,** welcher die Lage des Nahbezugspunkts bzw. des Designpunkts "Nähe" markiert. Der Fernbezugspunkt befindet sich in der Mitte der runden Klammern **202** und der Nahbezugspunkt in der Mitte des Nahmeßkreises **204.** Abhängig von der Lage der Fern- und Nahbezugspunkte kann der Stempel eines individuellen Brillenglases daher unterschiedlich aussehen. Mittels eines Kreuzes **206** (Zentrierkreuz) wird die Position des Zentrier- bzw. Anpaßpunkts gekennzeichnet.

Der Prismenbezugspunkt **208** befindet sich im Normalfall 4 mm unter dem Zentrierpunkt. Bei höherer Anisometropie und Kundenwunsch bezüglich einer bestimmten Gewichtung (z. B. wenn im Nahbereich die prismatischen Vertikaldifferenzen angeglichen werden sollen) kann ein Prismenangleichspunkt in die gewünschte Richtung verschoben werden.

Bei dem in **Fig. 16a** gezeigten Beispiel befindet sich der Fernbezugspunkt auf der Höhe des Zentrierpunkts. Der Nahbezugspunkt befindet sich auf einer vertikalen Höhe von -18 mm unterhalb des Zentrierpunkts. In **Fig. 16b** ist ein weiteres Beispiel einer individuellen Stempelung bzw. eines individuellen Stempelbildes eines individuellen Brillenglases dargestellt. Das Brillenglas- wird für einen Brillenträger, welcher viel Wert auf einen großen Fernbereich legt, individuell berechnet bzw. optimiert. Der Fernbezugspunkt liegt auf einer vertikalen Höhe von -4 mm unterhalb des Zentrier- bzw. Anpaßpunkts und der Nahbezugspunkt liegt auf einer vertikalen Höhe von -18 mm unterhalb des Zentrier- bzw. Anpaßpunkts.

Vorzugsweise sind die Werte für die Lage der Fern- und Nahbezugspunkte (insbesondere für die vertikale Höhe im Bezug auf den Zentrier- bzw. Anpaßpunkt) ebenfalls permanent im Brillenglas eingraviert.

In Ausnahmefällen kann die Stempelung von der oben beschriebenen abweichen. Ferner kann eine explizite nicht permanente Kennzeichnung der Positionen der Fern- und Nahbezugspunkte und/oder des Zentrier- bzw. Anpaßpunkts fehlen. Die Bezugspunkte können jedoch mit Hilfe einer Rekonstruktionsvorschrift, umfassend eine Zentrierkarte, aufgestempelte Skalen in 1 mm-Schritten und eine Glastüte eindeutig, ermittelt werden. Zur Rekonstruktion der Bezugspunkte wird die Brillenfassung mit gekennzeichnetem Zentrierpunkt auf das Zentrierkreuz der Zentrierkarte gelegt und die Position der Fern- und Nahbezugspunkte auf das Brillenglas gezeichnet. Die Position der Fern- und Nahbezugspunkte kann ebenfalls mit Hilfe der permanent eingravierten Werte unterhalb der nasalen Basiskurven- und Indexgravur ermittelt werden.

Neben einer Rekonstruktion der Position der Bezugspunkte ist es mit einer entsprechenden Zentrierkarte möglich einen optimalen Durchmesser des rohrunden Brillenglases zu ermitteln.

Die Ermittlung eines optimalen Durchmessers mittels Zentrierkarte kann folgendermaßen erfolgen:
1) Bestimmen des für die ausgesuchte Fassung entsprechenden Mindestdurchmessers, der - unabhängig von der seitlichen Zentrierung - dem kleinsten umschreibenden Durchmesserkreis der Zentrierkarte entspricht. Dieser Wert entspricht dem ersten Wert in einer Durchmesserbestellung, z. B. 50/60.
2) Positionieren des in der Anpassung ermittelten Durchblickspunkts so auf der Zentrierkarte, daß er mit dem Zentrierkreuz der Zentrierkarte zur Deckung kommt.
3) Ablesen des größten notwendigen Durchmessers. Dies ist bei einer Dezentration Richtung nasal, wie sie in den meisten Fällen auftritt (Pupillendistanz PD kleiner als der Mittenabstand der Fassung), der Durchmesserkreis, welcher die Fassung temporal umschreibt. Dieser Wert entspricht dem zweiten Wert der Durchmesserbestellung, z.B. 50/60. Vorzugsweise ist die Differenz zwischen nutzbarem und Mindestdurchmesser nicht größer als 10 mm.
4) Sind die Durchmesser nasal und temporal gleich, so empfiehlt sich die Bestellung in zentrischer Ausführung.

Zusätzlich zu der nicht permanenten Markierungen bzw. Stempelungen weist das individuelle Brillenglas ebenfalls permanente (Mikro-)Gravuren auf.

Fig. 17 zeigt die permanente Gravur eines individuell optimierten linken Brillenglases, das von hinten betrachtet wird (d.h. von der augenseitigen Seite). Die Funktionsgravur bzw. die permanente Markierung zur Ausrichtung des Brillenglases ist das Unendlichzeichen. Die beiden Funktionsgravuren **210, 212** befinden sich in einem Abstand von 34 mm zueinander auf Höhe des Zentrierpunkts bzw. Zentrierkreuzes. Unterhalb des nasalen Unendlichzeichens **212** befinden sich die jeweils zweistellige Basiskurvengravur **214** und Indexgravur **216.** Darunter befindet sich die Gravur **218** für die Lage von Fern- und Nahbezugspunkt. Die erste Zahl gibt dabei den vertikalen Abstand des Fernbezugspunkts relativ zum Zentrier- bzw. Anpaßpunkt an. Die zweite Zahl gibt den vertikalen Abstand des Nahbezugspunkts relativ zum Zentrier- bzw. Anpaßpunkt an.

Der Fernbezugspunkt kann vorzugsweise in einem Bereich zwischen -4 und +4 mm unterhalb bzw. oberhalb des Zentrierpunkts befinden. Der Nahbezugspunkt kann sich vorzugsweise in einem Bereich zwischen -13 und -20 mm unter dem Zentrier- bzw. Anpaßpunkt befinden.

Temporal unter der Funktionsgravur **210** befindet sich die zweistellige Additionsgravur **220.**

Zusammengefaßt bezeichnen in **Fig. 17**:
- ∞: die Funktionsgravur;
- 25: die Addition;
- 65: die Basiskurve;
- 60: die Brechzahl;
- -4: den individuellen vertikalen Abstand des Fernbezugspunkts vom Zentrier- bzw. Anpaßpunkt;
- 18: den individuellen vertikalen Abstand des Nahzbezugspunkts vom Zentrier- bzw. Anpaßpunkt.

Das fertigbearbeitete und gestempelte Brillenglas wird in einer Glastüte verpackt und dem Augenoptiker/Kunden geliefert. Ein Beispiel einer Glastüte ist in **Fig. 18** gezeigt. **Fig. 19** zeigt eine Liste der auf der Glastüte verwendeten Piktogramme bzw. Symbole.

Die individuellen Daten des Brillenträgers sind auf jeder Glastüte aufgedruckt. Insbesondere sind auf jeder Glastüte folgende Daten aufgedruckt:
- Glastyp, Material, Farbe, Beschichtung, Durchmesser
- Bestellwert: Sphäre, Zylinder, Achse, Prisma (resultierend), Basis (resultierend), Addition;
- Soll-Messwerte für das Scheitelbrechwertmessgerät im Messpunkt für Ferne und Addition in konkaver Scheitelmessstellung inkl. des messbaren Prismas im Prismenbezugspunkt (zusammengesetzt aus DRP und bestellten Prismen);

- Bei prismatischen Refraktionsdaten: Angaben zur Art der Refraktion: Pupillenmittenzentrierung (PMZ) oder Formelfall (FF) und Größe und Richtung der notwendigen Zentrierkorrektur;
- Allgemeine Auftragsdaten, ergänzende Informationen und Kommission auf der Rückseite der Glastüte;
- Angaben zu den individuellen Parametern: monokulare PD, HSA, VN, FSW;
- Angaben zu den Designpunkten: Lage von Fern- und Nahbezugspunkt bezogen auf den Zentrier- bzw. Anpaßpunkt;
- Basiskurve, Vordezentration und Inset des Glases; Angabe der korrigierten PD zum Einschleifen (COR PD), wenn die Scheibenform und die Zentrierdaten bekannt sind.

Auf der Glastüte finden sich insbesondere die relevanten Daten für ein korrektes Einschleifen in der Brillenfassung, insbesondere Daten bezüglich der Fassungs- bzw. Scheibenform.

Insbesondere wird bei einer Bestellung mit Angabe der Scheibenform und der Zentrierdaten (wie bei den Sportgläsern), die korrigierte Pupillendistanz PD zum Einschleifen (COR PD) berechnet. Diese ist notwendig, um die richtige Kunden-PD in der fertig verglasten Brille zu erhalten. Auch bei Brillengläsern mit Korrektionsprisma ist die COR PD anstatt der Kunden PD zum Einschleifen zu verwenden, wenn die Form angegeben wurde. Die notwendige Zentrierkorrektur für Prismen mit horizontaler und vertikaler Basislage ist bereits bei der Berechnung der Brillengläser berücksichtigt worden. Der Wert für die Zentrierkorrektur auf der Glastüte ist deshalb stets null.

Bei einer Bestellung ohne Formangabe kann die COR PD nicht berechnet werden, da die zu deren Berechnung notwendigen Parameter (Fassungs- und Zentrierdaten) nicht übermittelt werden. Bei einem individuell nach einem bevorzugten Optimierungsverfahren optimierten progressiven Brillenglas mit Korrektionsprismen wird die Zentrierkorrektur für Prismen mit horizontaler und vertikaler Basislage vorzugsweise bereits bei der Berechnung der Gläser berücksichtigt. Der Wert für die Zentrierkorrektur auf der Glastüte bleibt null. Dieser Wert bezieht sich bei einer Bestellung ohne Formangabe auf die PD.

**Figuren 20** **a** und **b** illustrieren die Zentrierung eines progressiven Brillenglases vor den Augen des Brillenträgers sowie die entsprechende Lage der Bezugspunkte. Das in **Fig. 20a** gezeigte Brillenglas ist ein individuelles Brillenglas mit individuell nach einem bevorzugten erfindungsgemäßen Verfahren ermittelten Positionen der Fernund Nahbezugspunkte. Insbesondere sind die Positionen des in **Fig. 20a** gezeigten Brillenglases individuell in Abhängigkeit von den Fassungsdaten festgelegt. Das in **Fig. 20b** gezeigte Brillenglas ist ein Standardbrillenglas.

Die individuell berechneten progressiven Brillengläser werden nach Bezugspunktforderung angepasst. Das bedeutet, daß der Zentrier- bzw. Anpaßpunkt (bzw. das Zentrierkreuz) bei Nullblickrichtung in habitueller Kopf- und Körperhaltung auf Pupillenmitte liegen soll. Die Mindesteinschleifhöhe hängt von der Lage des Nahbezugspunkts ab. Vorzugsweise bleiben mindestens noch 2 mm unterhalb des Nahbezugspunkts in der Fassung erhalten. Die minimale Einschleifhöhe liegt somit vorzugsweise bei 15 mm unter dem Zentrierpunkt. Werden Gleitsichtgläser abweichend von der Zentrierempfehlung angepasst, kann es zu Einschränkungen in den Abbildungseigenschaften kommen.

Bei einer fehlerhaften Zentrierung des Brillenglases, insbesondere bei einer zu tiefen Zentrierung, ergeben sich bereits im Fernbereich geringfügige Einschränkungen durch die tiefe Zentrierung. Die Unterschiede kommen insbesondere dadurch zustande, daß das Brillenglas nicht in der bei der Optimierung zugrunde gelegten Gebrauchssituation getragen wird.

Im Nahbereich hingegen zeigen sich, im Gegensatz zum Fernbereich, deutliche Einschränkungen in einem tiefer zentrierten Brillenglas. Zum einen resultieren diese Einschränkungen aus der einfachen Tatsache, daß der Nahbereich je nach Fassungsgröße nicht mehr in der Fassung vorhanden ist und der Brillenträger beim Blick in die Nähe durch den Progressionsbereich sieht, der deutlich schmäler ist als der Nahbereich. Zum anderen entstehen zusätzliche Fehler dadurch, daß das Brillenglas nicht in der bei der Optimierung zugrunde gelegten Gebrauchssituation getragen wird. Zusätzlich wird bei gleicher Blicksenkung die Nahwirkung nicht erreicht und der Kunde hat einen zusätzlichen Akkommodationsaufwand.

Eine Betonung der Sehbereiche kann daher richtigerweise wie oben beschrieben durch eine Verschiebung des Fern- und/oder Nahbezugspunkts erzeugt werden. Zusätzlich können bei einer abweichenden Hauptblickrichtung, z.B. bei besonders großen oder kleinen Menschen, die Hauptsehbereiche so individuell angeordnet werden, daß diese mit der jeweiligen Hauptblickrichtung zusammenfallen.

In den Bezugspunkten werden auch die so genannten Soll-Messwerte gemessen, wobei die Soll-Messwerte neben den Bestellwerten auf der Glastüte des individuellen Brillenglases angegeben werden. Die Soll-Messwerte beziehen sich vorzugsweise auf die konkave Scheitelmessstellung. Toleranzbetrachtungen beziehen sich dabei auf die Soll-Messwerte, nicht auf die Bestellwerte.

### Fernwirkung

Die Sollmesswerte für Sphäre, Zylinder und Achse werden im Fernbezugspunkt überprüft. Dieser Fernbezugspunkt befindet sich individuell unterschiedlich, vorzugsweise innerhalb eines Bereichs von +4 bis -4 mm, um den Zentrierpunkt. Die genaue Lage des Fernbezugspunkts kann der Zusatzgravur unterhalb der Basiskurven- und Indexgravur entnommen werden. Die Messung der Fernteilwirkung ist in **Fig. 21a** schematisch dargestellt.

### Prismatische Wirkung

Im Prismenbezugspunkt wird eine kombinierte Wirkung aus Dickenreduktionsprisma (Basislage immer 270°) und Korrektionsprismen gemessen. Die Messung der prismatischen Wirkung ist in **Fig. 21b** schematisch dargestellt.

### Nahwirkung

Der Nahbezugspunkt befindet sich individuell unterschiedlich innerhalb eines Bereichs von -13 bis -20 mm unterhalb des Zentrierpunkts. Die genaue Lage des Nahbezugspunkts kann der Zusatzgravur unterhalb der Basiskurven- und Indexgravur entnommen werden. Die Messung der Nahwirkung ist in **Fig. 21c** gezeigt.

### Addition

Der Sollmesswert der Addition entspricht der Differenz der mittleren Wirkung (sphärisches Äquivalent) zwischen Fern- und Nahbezugspunkt. In vielen Fällen ist es jedoch einfacher und im Allgemeinen ausreichend die Übereinstimmung von bestellter und gravierter Addition zu überprüfen.

Das nach dem oben beschriebenen Verfahren bzw. flexible Brillenglasdesign zeichnet sich insbesondere durch folgende vorteilhafte Eigenschaften aus:
- optimale Korrektur der Fehlsichtigkeit durch Berücksichtigung aller Refraktionsdaten (Wirkungsoptimierung), der Fassungs- und Zentrierdaten sowie von PD, HSA, VN und FSW;
- Sehbereiche sind immer optimal groß und ideal überlappend angeordnet, da alle individuellen Parameter und Refraktionsdaten bei der Optimierung berücksichtigt werden;
- Optimierung
   - in Gebrauchsstellung;
   - für alle Refraktionsdaten;
   - Wellenfrontenoptimierung unter Berücksichtigung der Abbildungsfehler höherer Ordnung, wie Koma und sphärische Aberration;
   - Berücksichtigung der Listing'schen Regel;
   - in Freiformtechnologie;
- Höchste Spontanverträglichkeit;
- Punktgenauer Inset, auch abweichend von 100% Konvergenzvermögen bestellbar (z. B. für Einäugige);
- Identische Sehbereiche rechts/links, auch bei Anisometropien;
- Bestellung der Refraktionsdaten für die Ferne auch in 0,12-dpt-Schritten;
- Bestellung von Prismen/MDM inklusive;
- Perfekte Ästhetik.

Vorzugsweise weist das individuell in Abhängigkeit von den Kundenbedürfnissen und Parametern ermittelte bzw. berechnete Brillenglasdesign charakteristische Eigenschaften eines ausgewogenen, universellen Brillenglasdesigns, d.h. maximal große Sehbereiche für alle Entfernungen bei harmonischen Übergängen zwischen den zentralen und peripheren Sehbereichen auf. Ein solches Design bzw. ein solches Brillenglas bietet somit einen optimalen Sehkomfort für ein breites Spektrum von Situationen im täglichen Leben (Autofahren, Freizeitaktivitäten, Lesen, etc.).

## Patentansprüche

1. Computerimplementiertes Verfahren zum Festlegen bzw. Bestimmen der räumlichen Position eines Fern- und eines Nahbezugspunkts eines progressiven Brillenglases zur Korrektur einer Fehlsichtigkeit eines Brillenträgers umfassend folgende Schritte:
- Erfassen von individuellen Daten des Brillenträgers;
- Ermitteln bzw. Berechnen der individuellen vertikalen und/oder horizontalen Position des Fern- und des Nahbezugspunkts in Abhängigkeit von der erfaßten individuellen Daten des Brillenträgers,
wobei der vertikale Abstand des Fern- und des Nahbezugspunkts von einem Zentrier- bzw. Anpaßpunkt des Brillenglases in Abhängigkeit von den individuellen Daten des Brillenträgers festgelegt wird; wobei die individuellen Daten des Brillenträgers Daten bezüglich der Präferenzen bzw. der Gewichtungen des Fern- und des Nahbereichs und optional des Progressionsbereichs umfassen, und wobei
- für den vertikalen Abstand *y_{BF}* des Fernbezugspunkts von dem Zentrier- bzw. Anpaßpunkt des Brillenglases gilt ${y}_{BF} = 4 - 4 * {G}_{F} / 33 , 33 \left[mm\right] f ⁢ {u}^{¨} ⁢ r 0 \leq {G}_{F} < 33$
und ${y}_{BF} = 0 - 4 * \left({G}_{F}-33,33\right) / 66 , 66 \left[mm\right] f ⁢ {u}^{¨} ⁢ r 33 \leq {G}_{F} \leq 100 ;$
und
- für den vertikalen Abstand *y_{BN}* des Nahbezugspunkts von dem Zentrierbzw. Anpaßpunkt gilt ${y}_{BN} = - 20 + 2 * {G}_{N} / 33 \left[mm\right] f ⁢ {u}^{¨} ⁢ r 0 \leq {G}_{N} < 33$
und ${y}_{BN} = - 18 + 5 * \left({G}_{N}-33,33\right) / 66 , 66 \left[mm\right] f ⁢ {u}^{¨} ⁢ r 33 \leq {G}_{N} \leq 100 ;$
wobei *G_{F}* die Gewichtung des Fernbereichs und *G_{N}* die Gewichtung des Nahbereichs bezeichnen.

2. Verfahren gemäß Anspruch 1, wobei:
- die vertikale Höhe des Fernbezugspunkts gemessen von dem Zentrier- bzw. Anpaßpunkt des Brillenglases auf einen Wert von -4 mm unterhalb bis 4 mm oberhalb des Zentrier- bzw. Anpaßpunkts, vorzugsweise in 0,1 mm Stufen, in Abhängigkeit von den individuellen Daten des Brillenträgers festgelegt werden kann; und/oder
- die vertikale Höhe des Nahbezugspunkts gemessen von dem Zentrier- bzw. Anpaßpunkt des Brillenglases auf einen Wert von -13 mm bis -20 mm unterhalb des Zentrier- bzw. Anpaßpunkts, vorzugsweise in 0,1 mm Stufen, in Abhängigkeit von den individuellen Daten des Brillenträgers festgelegt werden kann.

3. Verfahren gemäß einem der vorangegangenen Ansprüche, wobei für den vertikalen Abstand *y_{BF}* des Fernbezugspunkts von dem Zentrier- bzw. Anpaßpunkt und für den vertikalen Abstand *y_{NF}* des Nahbezugspunkts von dem Zentrier- bzw. Anpaßpunkt gelten: ${y}_{BF} = \left(GW⁢1*{y}_{BF ⁢ 1}+{y}_{BF ⁢ 2}\right) / \left({g}_{1}\right)$ ${y}_{BN} = \left(GW⁢2*{y}_{BN ⁢ 1}+{y}_{BN ⁢ 2}\right) / \left({g}_{2}\right)$
wobei ${y}_{BF ⁢ 1} = 4 - 4 * {G}_{F} / 33 , 33 \left[mm\right] f ⁢ {u}^{¨} ⁢ r 0 \leq {G}_{F} < 33$
und ${y}_{BF ⁢ 1} = 0 - \left(4*{G}_{F}-33,33\right) / 66 , 66 \left[mm\right] f ⁢ {u}^{¨} ⁢ r 33 \leq {G}_{F} \leq 100 ;$ ${y}_{BN ⁢ 1} = - 20 + 2 * {G}_{N} / 33 \left[mm\right] f ⁢ {u}^{¨} ⁢ r 0 \leq {G}_{N} < 33$
und ${y}_{BN ⁢ 1} = - 18 + 5 * \left({G}_{N}-33,33\right) / 66 , 66 \left[mm\right] f ⁢ {u}^{¨} ⁢ r 33 \leq {G}_{N} \leq 100 ;$ ${y}_{BF ⁢ 2} = {y}_{BN ⁢ 1} + {y}_{P}$ ${y}_{BN ⁢ 2} = {y}_{BF ⁢ 1} - {y}_{P}$ ${y}_{P} = 13 + 5 * {G}_{P} / 33 , 33 \left[mm\right] f ⁢ {u}^{¨} ⁢ r 0 \leq {G}_{P} < 33$
und ${y}_{P} = 18 + 2 * \left({G}_{P}-33,33\right) / 66 , 66 \left[mm\right] f ⁢ {u}^{¨} ⁢ r 33 \leq {G}_{P} \leq 100 ;$
wobei:
die Koeffizienten *GW*1 und GW 2 Werte zwischen 1 und 2 annehmen und wobei
*G_{F}* die Gewichtung des Fernbereichs;
*G_{N}* die Gewichtung des Nahbereichs; und
*G_{P}* die Gewichtung des Zwischen- bzw. Progressionsbereichs ${g}_{1} = 1 + GW ⁢ 1$ ${g}_{2} = 1 + GW ⁢ 2$
bezeichnen.

4. Verfahren gemäß einem der vorangegangenen Ansprüche, wobei die individuellen Daten des Brillenträgers individuelle Parameter der Augen des Brillenträgers und/oder der Anordnung der Brille vor den Augen des Brillenträgers umfassen.

5. Verfahren gemäß einem der vorangegangenen Ansprüche, wobei die individuellen Daten des Brillenträgers Daten bezüglich
- des individuellen Objektabstands für die Ferne und/oder des individuellen Objektabstands für die Nähe; und/oder
- des individuellen Objektabstands für die Ferne bei der Refraktionsbestimmung und/oder des individuellen Objektabstands für die Nähe bei der Refraktionsbestimmung; und/oder
- Daten bezüglich einer bisher getragenen Brille; und/oder
- Daten bezüglich zu Verbesserungswünschen zu der bisherigen getragenen Brille; und/oder
- Daten bezüglich der individuellen Hauptblickrichtung für die Ferne und die Nähe; und/oder
- Daten bezüglich der individuellen Kopf- und Körperhaltung; und/oder
- physiologische Parameter, insbesondere des Auges des Brillenträgers; und/oder
- Präferenzen bzw. eine Gewichtung der Wichtigkeit der Abbildungseigenschaften gegenüber den ästhetischen Eigenschaften des Brillenglases umfassen.

6. Verfahren gemäß einem der vorangegangenen Ansprüche, wobei die individuellen Daten des Brillenträgers Daten in zumindest zwei unterschiedlichen Kategorien der individuellen Daten umfassen und
wobei das Ermitteln der Position des Fern- und/oder des Nahbezugspunkts folgende Schritte umfaßt
- Ermitteln einer idealen Position des Fern- und/oder einer idealen Position des Nahbezugspunkts für jede der Kategorien anhand von individuellen Daten des Brillenträgers in der jeweiligen Kategorie;
- Berechnen der Position des Fern- und/oder der Position des Nahbezugspunkts anhand der ermittelten idealen Positionen des Fern- und/oder Nahbezugspunkts in den jeweiligen Kategorien.

7. Verfahren gemäß Anspruch 6, wobei die Position des Fern- und/oder des Nahbezugspunkts nach der Formel: ${y}_{DF , DN} = {\sum }_{i = 1}^{N} {g}_{i} ⁢ {y}_{DF , DN}^{i}$ ${\sum }_{i = 1}^{N} {g}_{i} = 1$
berechnet wird, wobei:
*gᵢ* die Gewichtung der *i* -ten Kategorie;
${y}_{DF , DN}^{i}$ die ideale Position des Fernbezugspunkts DF bzw. des Nahbezugspunkts DN für die *i* -te Kategorie ; und
N die Anzahl der unterschiedlichen Kategorien
bezeichnen.

8. Verfahren gemäß einem der vorangegangenen Ansprüche, ferner umfassend die Schritte
- Berechnen eines individuellen Brillenglasdesigns, welches den individuell festgelegten Fern- und/oder Nahbezugspunkt aufweist;
- Visualisierung des berechneten individuellen Brillenglasdesigns und der räumlichen Position des individuellen Fern- und/oder Nahbezugspunkts.

9. Computerprogrammerzeugnis, welches ausgelegt ist, wenn geladen und ausgeführt auf einem Computer, das Verfahren zum Festlegen bzw. Bestimmen der individuellen räumlichen Position eines Fern- und eines Nahbezugspunkts eines progressiven Brillenglases gemäß einem der Ansprüche 1 bis 9 durchzuführen.

10. Vorrichtung zum Bestimmen der individuellen räumlichen Position eines Fernund eines Nahbezugspunkts eines progressiven Brillenglases umfassend:
- Erfassungsmittel zum Erfassen von individuellen Daten des Brillenträgers;
- Berechnungs- bzw.- Optimierungsmittel, welche derart ausgelegt sind, ein Verfahren zum Bestimmen der räumlichen Position des Fern- und des Nahbezugspunkts in Abhängigkeit von den erfaßten individuellen Daten des Brillenträgers gemäß einem der Ansprüche 1 bis 8 durchzuführen.

11. Grafische Benutzerschnittstelle zum Festlegen bzw. Bestimmen und Darstellen der räumlichen Position eines Fern- und eines Nahbezugspunkts eines progressiven Brillenglases, umfassend
- zumindest einen Individualdaten-Eingabeabschnitt, welcher ausgelegt ist, individuelle Daten des Brillenträgers einzugeben; und
- zumindest einen Anzeigeabschnitt, welcher ausgelegt ist, die räumlichen Position des Fern- und Nahbezugspunkts darzustellen, wobei die räumliche Position des Fern- und Nahbezugspunkts nach dem Verfahren gemäß einem der Ansprüche 1 bis 8 in Abhängigkeit von den individuellen Daten des Brillenträgers ermittelt wird.

12. Computerimplementiertes Verfahren zum Bestimmen bzw. Berechnen eines individuellen Brillenglasdesigns für ein progressives Brillenglases zur Korrektur einer Fehlsichtigkeit eines bestimmten Brillenträgers, umfassend folgende Schritte:
- Erfassen von individuellen Daten des Brillenträgers;
- Ermitteln bzw. Berechnen der räumlichen Position eines Fern- und eines Nahbezugspunkts in Abhängigkeit von den erfaßten individuellen Daten des Brillenträgers nach dem Verfahren gemäß einem der Ansprüche 1 bis 8;
- Berechnen der räumlichen Lage und/oder der Größe eines Fern-, Nah- und Progressionsbereichs des Brillenglasdesigns in Abhängigkeit von der ermittelten individuellen räumlichen Position des Fern- und des Nahbezugspunkts.

13. Computerprogrammerzeugnis, welches ausgelegt ist, wenn geladen und ausgeführt auf einem Computer, ein Verfahren zum Bestimmen bzw. Berechnen eines individuellen Brillenglasdesigns für ein progressives Brillenglases gemäß Anspruch 12 durchzuführen.

14. Vorrichtung zum Bestimmen bzw. Berechnen eines individuellen Brillenglasdesigns für ein progressives Brillenglas zur Korrektur einer Fehlsichtigkeit eines bestimmten Brillenträgers, umfassend
- Erfassungsmittel zum Erfassen von individuellen Daten des Brillenträgers;
- Berechnungs- bzw. Optimierungsmittel zum Berechnen der individuellen räumlichen Position eines Fern- und eines Nahbezugspunkts in Abhängigkeit von den individuellen Daten des Brillenträgers nach dem Verfahren gemäß einem der Ansprüche 1 bis 8;
- Berechnungs- bzw. Optimierungsmittel zum Berechnen der räumlichen Lage und/oder der Größe eines Fern-, Nah- und Progressionsbereichs des Brillenglasdesigns in Abhängigkeit von der berechneten individuellen räumlichen Position des Fern- und des Nahbezugspunkts.

15. Grafische Benutzerschnittstelle zum Festlegen bzw. Bestimmen und Darstellen eines individuellen Brillenglasdesigns für ein progressives Brillenglas, umfassend:
- zumindest einen Individualdaten-Eingabeabschnitt, welcher ausgelegt ist, individuelle Daten des Brillenträgers einzugeben; und
- zumindest einen Anzeigeabschnitt, welcher ausgelegt ist, das individuelle Brillenglasdesign darzustellen, wobei das individuelle Brillenglasdesign nach einem Verfahren gemäß Anspruch 12 berechnet bzw. bestimmt wird.

## Claims

1. Computer-implemented method for defining or determining the spatial position of a far and a near reference point of a progressive spectacle lens to correct a defective vision of a spectacle wearer comprising the following steps:
• acquiring individual data of the spectacle wearer;
• determining or calculating the individual vertical and/or horizontal position of the far and the near reference point as a function of the acquired individual data of the spectacle wearer,
wherein the vertical distance of the far and the near reference point from a centring or adjusting point of the spectacle lens is defined as a function of the individual data of the spectacle wearer; wherein the individual data of the spectacle wearer comprise data relating to the preferences or weightings of the far and the near zone and optionally of the progression zone, and wherein
• the following applies for the vertical distance y_{BF} of the far reference point from the centring or adjusting point of the spectacle lens ${y}_{BF} = 4 - 4 * {G}_{F} / 33.33 \left[mm\right] for 0 \leq {G}_{F} < 33$
and ${y}_{BF} = 0 - 4 * \left({G}_{F}-33.33\right) / 66.66 \left[mm\right] for 33 \leq {G}_{F} \leq 100 ;$
and
• the following applies for the vertical distance y_{BN} of the near reference point from the centring or adjusting point ${y}_{BN} = - 20 + 2 * {G}_{N} / 33 \left[mm\right] for 0 \leq {G}_{N} < 33$
and ${y}_{BN -} = - 18 + 5 * \left({G}_{N}-33.33\right) / 66.66 \left[mm\right] for 33 \leq {G}_{N} \leq 100 ;$
wherein G_{F} represents the weighting of the far zone and G_{N} represents the weighting of the near zone.

2. Method according to claim 1, wherein:
- the vertical height of the far reference point measured from the centring or adjusting point of the spectacle lens can be determined to a value of -4 mm below to 4 mm above the centring or adjusting point, preferably in 0.1 mm steps, as a function of the individual data of the spectacle wearer; and/or
- the vertical height of the near reference point measured from the centring or adjusting point of the spectacle lens can be determined to a value of -13 mm to -20 mm below the centring or adjusting point, preferably in 0.1 mm steps, as a function of the individual data of the spectacle wearer.

3. Method according to one of the preceding claims, wherein the following applies for the vertical distance y_{BF} of the far reference point from the centring or adjusting point and for the vertical distance y_{NF} of the near reference point from the centring or adjusting point: ${y}_{BF} = \left(GW⁢1*{y}_{BF ⁢ 1}+{y}_{BF ⁢ 2}\right) / \left({g}_{1}\right)$ ${y}_{BN} = \left(GW⁢2*{y}_{BN ⁢ 1}+{y}_{BN ⁢ 2}\right) / \left({g}_{2}\right)$
wherein ${y}_{BF ⁢ 1} = 4 - 4 * {G}_{F} / 33.33 \left[mm\right] for 0 \leq {G}_{F} < 33$
and ${y}_{BF ⁢ 1} = 0 - \left(4*{G}_{F}/33.33\right) / 66.66 \left[mm\right] for 33 \leq {G}_{F} \leq 100 ;$ ${y}_{BN ⁢ 1} = - 20 + 2 * {G}_{N} / 33 \left[mm\right] for 0 \leq {G}_{N} < 33$
and ${y}_{BN ⁢ 1} = - 18 + 5 * \left({G}_{N}-33.33\right) / 66.66 \left[mm\right] for 33 \leq {G}_{N} \leq 100 ;$ ${y}_{BF ⁢ 2} = {y}_{BN ⁢ 1} + {y}_{p}$ ${y}_{BN ⁢ 2} = {y}_{BF ⁢ 1} - {y}_{p}$ ${y}_{p} = 13 + 5 * {G}_{p} / 33.33 \left[mm\right] for 0 \leq {G}_{p} < 33$
and ${y}_{p} = - 18 + 2 * \left({G}_{p}-33.33\right) / 66.66 \left[mm\right] for 33 \leq {G}_{p} \leq 100 ;$
wherein:
the coefficients GW1 and GW2 assume values between 1 and 2 and wherein
G_{F} represents the weighting of the far zone;
G_{N} represents the weighting of the near zone;
G_{P} represents the weighting of the intermediate or progression zone; ${g}_{1} = 1 + GW ⁢ 1$ ${g}_{2} = 1 + GW ⁢ 2.$

4. Method according to one of the preceding claims, wherein the individual data of the spectacle wearer comprise individual parameters of the eyes of the spectacle wearer and/or the arrangement of the spectacles in front of the eyes of the spectacle wearer.

5. Method according to one of the preceding claims, wherein the individual data of the spectacle wearer comprise data relating to
• the individual object distance for long distance and/or the individual object distance for near distance; and/or
• the individual object distance for long distance on determination of the refraction and/or the individual object distance for near distance on determination of the refraction; and/or
• data relating to spectacles worn hitherto; and/or
• data relating to desired improvements to the spectacles worn hitherto; and/or
• data relating to the individual main viewing direction for long distance and near distance; and/or
• data relating to the individual positioning of the head and body; and/or
• physiological parameters, in particular of the eye of the spectacle wearer; and/or
• preferences or weighting of the importance of the image-forming properties in relation to the aesthetic qualities of the spectacle lens.

6. Method according to one of the preceding claims, wherein the individual data of the spectacle wearer comprise data in at least two different categories of the individual data and
wherein the determination of the position of the far and/or the near reference point comprises the following steps
• determining an ideal position of the far reference point and/or an ideal position of the near reference point for each of the categories on the basis of individual data of the spectacle wearer in the respective category;
• calculating the position of the far reference point and/or the position of the near reference point on the basis of the determined ideal positions of the far and/or near reference point in the respective categories.

7. Method according to claim 6, wherein the position of the far and/or near reference point is calculated according to the formula: ${y}_{DF , DN} = {\sum }_{i = 1}^{N} {g}_{i} ⁢ {y}_{DF , DN}^{i}$ ${\sum }_{i = 1}^{N} {g}_{i} = 1$
wherein:
*gᵢ* represents the weighting of the i-th category;
${y}_{DF , DN}^{i}$ represents the ideal position of the far reference point DF or the near reference point DN for the i-th category; and
N represents the number of different categories.

8. Method according to one of the preceding claims, additionally comprising the steps
• calculating an individual spectacle lens design, which has the individually defined far and/or near reference point;
• visualising the calculated individual spectacle lens design and the spatial position of the individual far and/or near reference point.

9. Computer program product, which when loaded and implemented on a computer is designed to conduct the method for defining or determining the individual spatial position of a far and a near reference point of a progressive spectacle lens according to one of claims 1 to 9?.

10. Device for determining the individual spatial position of a far and a near reference point of a progressive spectacle lens comprising:
• acquisition means for acquiring individual data of the spectacle wearer;
• calculation or optimisation means that are designed so as to conduct a method for determining the spatial position of the far and the near reference point as a function of the acquired individual data of the spectacle wearer according to one of claims 1 to 8.

11. Graphical user interface for defining or determining and displaying the spatial position of a far and a near reference point of a progressive spectacle lens comprising
• at least one individual data input section that is designed to input individual data of the spectacle wearer; and
• at least one display section that is designed to display the spatial position of the far and near reference point, wherein the spatial position of the far and near reference point is determined as a function of the individual data of the spectacle wearer using the method according to one of claims 1 to 8.

12. Computer-implemented method for determining or calculating an individual spectacle lens design for a progressive spectacle lens to correct a defective vision of a specific spectacle wearer comprising the following steps:
• acquiring individual data of the spectacle wearer;
• determining or calculating the spatial position of a far and a near reference point as a function of the acquired individual data of the spectacle wearer using the method according to one of claims 1 to 8;
• calculating the spatial position and/or the size of a far, near and progression zone of the spectacle lens design as a function of the determined individual spatial position of the far and the near reference point.

13. Computer program product, which when loaded and implemented on a computer is designed to conduct a method for determining or calculating an individual spectacle lens design for a progressive spectacle lens according to claim 12.

14. Device for determining or calculating an individual spectacle lens design for a progressive spectacle lens to correct a defective vision of a specific spectacle wearer comprising:
• acquisition means for acquiring individual data of the spectacle wearer;
• calculation or optimisation means for calculating the individual spatial position of a far and a near reference point as a function of the individual data of the spectacle wearer using the method according to one of claims 1 to 8;
• calculation or optimisation means for calculating the spatial position and/or the size of a far, near and progression zone of the spectacle lens design as a function of the calculated individual spatial position of the far and the near reference point.

15. Graphical user interface for defining or determining and displaying an individual spectacle lens design for a progressive spectacle lens comprising:
• at least one individual data input section that is designed to input individual data of the spectacle wearer; and
• at least one display section that is designed to display the individual spectacle lens design, wherein the individual spectacle lens design is calculated or determined using a method according to claim 12.

## Revendications

1. Procédé mis en oeuvre par ordinateur pour fixer, respectivement, déterminer, la position dans l'espace d'un point de référence de loin et d'un point de référence de près d'un verre de lunettes progressif pour la correction d'un défaut de vision d'un porteur de lunettes, comprenant les étapes suivantes :
- saisie des données individuelles du porteur de lunettes ;
- détermination, respectivement, calcul, des positions individuelles verticales et/ou horizontales du point de référence de loin et du point de référence de près en fonction des données individuelles saisies pour le porteur de lunettes,
la distance verticale du point de référence de loin et du point de référence de près par rapport à un point central, respectivement à un point d'accommodation, du verre de lunettes étant déterminée en fonction des données individuelles du porteur de lunettes ; les données individuelles du porteur de lunettes comprenant les préférences, respectivement les pondérations, des domaines de loin et de près, et éventuellement du domaine de progression, et où
- l'on a ${y}_{BF} = 4 - 4 * {G}_{F} / 33 , 33 \left[mm\right] pour 0 \leq {G}_{F} \leq 33$
et ${y}_{BF} = 0 - 4 * \left({G}_{F}-33,33\right) / 66 , 66 \left[mm\right] pour 33 \leq {G}_{F} \leq 100$
pour la distance verticale y_{BF} du point de référence de loin par rapport au point central, respectivement au point d'accommodation, du verre de lunettes ;
et
- l'on a ${y}_{BN} = - 20 + 2 * {G}_{N} / 33 \left[mm\right] pour 0 \leq {G}_{N} \leq 33$ ${y}_{BF} = - 18 + 5 * \left({G}_{N}-33,33\right) / 66 , 66 \left[mm\right] pour 33 \leq {G}_{N} \leq 100$
pour la distance verticale *y_{BN}* du point de référence de près par rapport au point central, respectivement au point d'accommodation ;
*G_{F}* représentant la pondération du domaine lointain et *G_{N}* représentant la pondération du domaine proche.

2. Procédé selon la revendication 1, où
- la hauteur verticale du point de référence de loin mesurée à partir du point central, respectivement du point d'accommodation, du verre de lunettes, peut être fixée à une valeur entre - 4 mm en dessous et 4 mm au-dessus du point central, respectivement du point d'accommodation, de préférence par des incrémentations de 0,1 mm, en fonction des données individuelles du porteur de lunettes ; et/ou
- la hauteur verticale du point de référence de près mesurée à partir du point central, respectivement du point d'accommodation du verre de lunettes, peut être fixée à une valeur entre - 13 mm à - 20 mm en dessous du point central, respectivement du point d'accommodation, de préférence par des incrémentations de 0,1 mm, en fonction des données individuelles du porteur de lunettes.

3. Procédé selon l'une des revendications précédentes, où l'on a, pour la distance verticale *y_{BF}* du point de référence de loin par rapport au point central, respectivement du point d'accommodation, et pour la distance verticale *y_{NF}* du point de référence de près par rapport au point central, respectivement du point d'accommodation : ${Y}_{BF} = \left(GW⁢1*{y}_{BF ⁢ 1}+{y}_{BF ⁢ 2}\right) / \left({g}_{1}\right)$ ${Y}_{BN} = \left(GW⁢2*{y}_{BN ⁢ 1}+{y}_{BN ⁢ 2}\right) / \left({g}_{2}\right)$
où ${y}_{BF ⁢ 1} = 4 - 4 * {G}_{F} / 33 , 33 \left[mm\right] pour 0 \leq {G}_{F} \leq 33$
et ${y}_{BF ⁢ 1} = 0 - \left(4*{G}_{F}-33,33\right) / 66 , 66 \left[mm\right] pour 33 \leq {G}_{F} \leq 100$ ${y}_{BN ⁢ 1} = - 20 + 2 * {G}_{N} / 33 \left[mm\right] pour 0 \leq {G}_{N} \leq 33$
et ${y}_{BN ⁢ 1} = - 18 + 5 * \left({G}_{N}-33,33\right) / 66 , 66 \left[mm\right] pour 33 \leq {G}_{N} \leq 100$ ${y}_{BF ⁢ 2} = {y}_{BN ⁢ 1} + {y}_{P}$ ${y}_{BN ⁢ 2} = {y}_{BF ⁢ 1} - {y}_{P}$ ${y}_{P} = 13 + 5 * {G}_{P} / 33 , 33 \left[mm\right] pour 0 \leq {G}_{P} \leq 33$
et ${y}_{P} = 18 + 2 * \left({G}_{P}-33,33\right) / 66 , 66 \left[mm\right] pour 33 \leq {G}_{P} \leq 100 ;$
où
les coefficients *GW1* et *GW2* adoptent des valeurs entre 1 et 2 et où
*G_{F}* désigne la pondération du domaine lointain ;
*G_{N}* désigne la pondération du domaine proche ; et
*G_{P}* désigne la pondération du domaine intermédiaire, respectivement, de progression ${g}_{1} = 1 + GW ⁢ 1$ ${g}_{2} = 1 + GW ⁢ 2.$

4. Procédé selon l'une des revendications précédentes, les données individuelles du porteur de lunettes comprenant les paramètres individuels des yeux du porteur de lunettes et/ou de la disposition des lunettes devant les yeux du porteur de lunettes.

5. Procédé selon l'une des revendications précédentes, où les données individuelles du porteur de lunettes comprennent des données concernant
- la distance à l'objet individuelle pour le lointain et/ou la distance à l'objet individuelle pour le proche ; et/ou
- la distance à l'objet individuelle pour le lointain lors de la détermination de la réfraction et/ou la distance à l'objet individuelle pour le proche lors de la détermination de la réfraction ; et/ou
- des données concernant les lunettes portées jusqu'à présent ; et/ou
- des données concernant les souhaits d'amélioration par rapport aux lunettes portée jusqu'à présent ; et/ou
- des données concernant la direction individuelle du regard principal pour le lointain et le proche ; et/ou
- des données concernant la tête et le port de tête individuels ; et/ou
- des paramètres physiologiques, concernant notamment l'oeil du porteur de lunettes ; et/ou
- des préférences, respectivement une pondération de l'importance des caractéristiques d'adaptation par rapport aux caractéristiques esthétiques du verre de lunettes.

6. Procédé selon l'une des revendications précédentes, les données individuelles du porteur de lunettes comprenant des données dans au moins deux catégories différentes des données individuelles et
la détermination de la position du point de référence de loin et/ou de près comprenant les étapes suivantes
- détermination d'une position idéale du point de référence de loin et/ou de près pour chaque catégorie à l'aide de données individuelles dans la catégorie correspondante ;
- calcul de la position du point de référence de loin et/ou de la position du point de référence de près à l'aide des positions idéales déterminées pour les points de référence de loin et/ou de près dans les catégories correspondantes.

7. Procédé selon la revendication 6 où la position du point de référence de loin, et/ou la position du point de référence de près, est calculée selon la formule : ${y}_{DF , DN} = {\sum }_{i = 1}^{N} {g}_{i} {{y}^{i}}_{DF , DN}$ ${\sum }_{i = 1}^{N} {g}_{i} = 1$
où :
*gᵢ* représente la pondération de la i^{ème} catégorie ;
*yⁱ_{DF,DN}* représente la position idéale du point de référence de loin DF, respectivement du point de référence de près DN, pour la i^{ème} catégorie ; et
N représente le nombre de catégories différentes.

8. Procédé selon l'une des revendications précédentes, comprenant en outre les étapes de
- calcul d'un design de verre de lunettes individuel, qui présente le point de référence de loin et/ou le point de référence de près individuel(s) défini(s) ;
- visualisation du design du verre de lunettes individuel calculé et de la position dans l'espace du point de référence de loin et/ou du point de référence de près individuel(s).

9. Résultat d'un programme d'ordinateur qui est conçu, lorsqu'il est enregistré et exécuté sur un ordinateur, pour exécuter le procédé de définition, respectivement de détermination, de la position individuelle dans l'espace d'un point de référence de loin et d'un point de référence de près d'un verre de lunettes progressif selon l'une des revendications 1 à 9.

10. Dispositif destiné à déterminer la position individuelle dans l'espace d'un point de référence de loin et d'un point de référence de près d'un verre de lunettes progressif, comprenant :
- des moyens de saisie pour la saisie des données individuelles du porteur de lunettes ;
- des moyens de calcul, respectivement, d'optimisation, qui sont conçus de telle façon pour exécuter un procédé de détermination de la position dans l'espace du point de référence de loin et du point de référence de près en fonction des données individuelles du porteur de lunettes saisies, selon l'une des revendications 1 à 8.

11. Interface utilisateur graphique pour définir, respectivement, déterminer et représenter, la position dans l'espace d'un point de référence de loin et d'un point de référence de près d'un verre de lunettes progressif, comprenant
- au moins une séquence d'introduction de données individuelles, qui est destinée à introduire les données individuelles du porteur de lunettes ; et
- au moins une séquence d'affichage qui est destinée à représenter la position dans l'espace du point de référence de loin et du point de référence de près, la position dans l'espace du point de référence de loin et du point de référence de près étant déterminée d'après le procédé selon l'une des revendications 1 à 8 en fonction des données individuelles du porteur de lunettes.

12. Procédé mis en oeuvre par ordinateur pour déterminer, respectivement, calculer, un design de verre de lunettes individuel pour un verre de lunettes progressif destiné à la correction d'un défaut de vision d'un porteur de lunettes défini, comprenant les étapes suivantes :
- saisie des données individuelles du porteur de lunettes ;
- détermination, respectivement calcul, de la position dans l'espace d'un point de référence de loin et d'un point de référence de près en fonction des données individuelles saisies pour le porteur de lunettes, d'après le procédé selon l'une des revendications 1 à 8 ;
- calcul de la localisation dans l'espace et/ou de la grandeur d'un domaine lointain, proche et de progression du design d'un verre de lunettes en fonction de la position dans l'espace individuelle déterminée du point de référence de loin et du point de référence de près.

13. Résultat d'un programme d'ordinateur qui est conçu, lorsqu'il est enregistré et exécuté sur un ordinateur, pour exécuter un procédé de détermination, respectivement de calcul, d'un design d'un verre de lunettes individuel pour un verre de lunettes progressif selon la revendication 12.

14. Dispositif destiné à déterminer, respectivement, à calculer, un design de verre de lunettes individuel pour un verre de lunettes progressif destiné à la correction d'un défaut de vision d'un porteur de lunettes défini, comprenant
- des moyens de saisie pour la saisie des données individuelles du porteur de lunettes ;
- des moyens de calcul, respectivement, d'optimisation, pour le calcul de la position dans l'espace d'un point de référence de loin et d'un point de référence de près en fonction des données individuelles du porteur de lunettes d'après le procédé selon l'une des revendications 1 à 8 ;
- des moyens de calcul, respectivement, d'optimisation, pour le calcul de la localisation dans l'espace d'un domaine lointain, d'un domaine proche, et d'un domaine de progression du design du verre de lunettes en fonction de la position dans l'espace individuelle calculée du point de référence de loin et du point de référence de près.

15. Interface utilisateur graphique pour la définition, respectivement, la détermination et la représentation d'un design de verre de lunettes individuel pour un verre de lunettes progressif, comprenant :
- au moins une séquence d'introduction de données individuelles, qui est destinée à introduire les données individuelles du porteur de lunettes ; et
- au moins une séquence d'affichage qui est destinée à représenter le design du verre de lunettes individuel, le design du verre de lunettes individuel étant calculé, respectivement, déterminé, d'après un procédé selon la revendication 12.
